(19) Europäisches Patentamt
European Patent Office
Office européen des brevets

(11) EP 1 693 452 B1

(12) **EUROPEAN PATENT SPECIFICATION**

(45) Date of publication and mention of the grant of the patent:
**25.12.2013 Bulletin 2013/52**

(21) Application number: **04819489.8**

(22) Date of filing: **26.11.2004**

(51) Int Cl.:
***C12N 15/09*** *(2006.01)* ***C12Q 1/68*** *(2006.01)*
***C12Q 1/02*** *(2006.01)* ***G01N 33/53*** *(2006.01)*
***G01N 33/50*** *(2006.01)* ***G01N 33/15*** *(2006.01)*

(86) International application number:
**PCT/JP2004/017995**

(87) International publication number:
**WO 2005/052154 (09.06.2005 Gazette 2005/23)**

(54) **METHOD OF ESTIMATING TOXICITY OF DRUG**

VERFAHREN ZUR ABSCHÄTZUNG DER TOXIZITÄT EINES ARZNEISTOFFS

PROCEDE POUR ESTIMER LA TOXICITE DE MEDICAMENTS

(84) Designated Contracting States:
**AT BE BG CH CY CZ DE DK EE ES FI FR GB GR HU IE IS IT LI LU MC NL PL PT RO SE SI SK TR**

(30) Priority: **27.11.2003 JP 2003397551**

(43) Date of publication of application:
**23.08.2006 Bulletin 2006/34**

(73) Proprietor: **Takeda Pharmaceutical Company Limited**
**Osaka-shi,**
**Osaka 541-0045 (JP)**

(72) Inventors:
- **TAKAMI, Kenji**
  **Osaka-shi,**
  **Osaka 532-8686 (JP)**
- **SAWADA, Hiroshi**
  **Osaka-shi,**
  **Osaka 532-8686 (JP)**
- **ASAHI, Satoru**
  **Osaka-shi,**
  **Osaka 532-8686 (JP)**

(74) Representative: **Tilmann, Max Wilhelm et al**
**König-Szynka-Tilmann-von Renesse**
**Patentanwälte Partnerschaft mbB**
**Postfach 11 09 46**
**40509 Düsseldorf (DE)**

(56) References cited:
**WO-A-02/068579 WO-A-03/065993**

- **"AFFYMETRIX CATALOG; PRODUKT: HUMAN GENOME U133A ARRAY; ARRAY FINDER" AFFYMETRIX PRODUCT CATALOG, XX, XX, 1 July 2002 (2002-07-01), page 1, XP002267612**
- **NUWAYSIR E F ET AL: "MICROARRAYS AND TOXICOLOGY: THE ADVENT OF TOXICOGENOMICS" MOLECULAR CARCINOGENESIS, ALAN LISS, NEW YORK, NY, US, vol. 24, no. 3, 1 March 1999 (1999-03-01), pages 153-159, XP001064021 ISSN: 0899-1987**
- **AARDEMA MARILYN J ET AL: "Toxicology and genetic toxicology in the new era of toxicogenomics: Impact of -omics technologies" MUTATION RESEARCH, AMSTERDAM, NL, vol. 499, no. 1, 29 January 2002 (2002-01-29), pages 13-25, XP002471693 ISSN: 0027-5107**
- **DE LONGUEVILLE FRANCOISE ET AL: "Use of a low-density microarray for studying gene expression patterns induced by hepatotoxicants on primary cultures of rat hepatocytes." TOXICOLOGICAL SCIENCES : AN OFFICIAL JOURNAL OF THE SOCIETY OF TOXICOLOGY OCT 2003, vol. 75, no. 2, October 2003 (2003-10), pages 378-392, XP002489829 ISSN: 1096-6080**
- **ARNOLD H ET AL: "Simultaneous exposure of fish to endosulfan and disulfoton in vivo: Ultrastructural, stereological and biochemical: Reactions in hepatocytes of mala rainbow trout (Oncorhynchus mykiss)" AQUATIC TOXICOLOGY 1995 NL, vol. 33, no. 1, 1995, pages 17-43, XP002489830 ISSN: 0166-445X**
- **BRIND ET AL: "Drugs that Damage the Liver" 20021101, vol. 30, no. 11, 1 November 2002 (2002-11-01), pages 21-23, XP005769439**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- REASOR M J ET AL: "Drug-induced phospholipidosis: are there functional consequences?" EXPERIMENTAL BIOLOGY AND MEDICINE, KARGER, BASEL, CH, vol. 226, no. 9, 1 October 2001 (2001-10-01), pages 825-830, XP002999628 ISSN: 1535-3699
- HALLIWELL W H: "CATIONIC AMPHIPHILIC DRUG-INDUCED PHOSPHOLIPIDOSIS" TOXICOLOGIC PATHOLOGY, SOCIETY OF TOXICOLOGIC PATHOLOGISTS, LAWRENCE, KS, US, vol. 25, no. 1, 1 January 1997 (1997-01-01), pages 53-60, XP000881113 ISSN: 0192-6233
- CASARTELLI A ET AL: "A cell-based approach for the early assessment of the phospholipidogenic potential in pharmaceutical research and drug development" CELL BIOLOGY AND TOXICOLOGY, XX, XX, vol. 19, no. 3, 1 June 2003 (2003-06-01), pages 161-176, XP002435160
- NICHOLLS ANDREW W ET AL: "A metabonomic approach to the investigation of drug-induced phospholipidosis: An NMR spectroscopy and pattern recognition study" BIOMARKERS, TAYLOR AND FRANCIS, LONDON, GB, vol. 5, no. 6, 1 November 2000 (2000-11-01), pages 410-423, XP009094851 ISSN: 1354-750X
- SAWADA H ET AL: "A toxicogenomic approach to drug-induced phospholipidosis: analysis of its induction mechanism and establishment of a novel in vitro screening system" TOXICOLOGICAL SCIENCES, ACADEMIC PRESS, SAN DIEGO, FL, US, vol. 83, no. 2, 1 January 2005 (2005-01-01), pages 282-292, XP002986941 ISSN: 1096-6080
- GERBAUX C. ET AL: 'Hyperactivity of cathepsin B and other lysosomal enzymes in fibroblasts exposed to azithromycin, a dicationic macrolide antibiotic with exceptional tissue accumulation' FEBS LETTERS vol. 394, 1996, pages 307 - 310, XP002986940
- SAWADA H. ET AL: 'A toxicogenomic approach to drug-induced phospholipidosis: analysis of its induction mechanism and establishment of a novel in vitro screening system' TOXICOL. SCI. vol. 83, no. 2, 2005, pages 282 - 292, XP002986941
- CLARK H.F. ET AL: 'The secreted protein discovery initiative (SPDI), a large effort to identify novel human secreted and transmembrane proteins: a bioinformatics assessment, 2003, ACCESSION: NM_014960, NM_022823,' GENOME RES. vol. 13, no. 10, 2003, pages 2265 - 2270, XP001189293
- CHURCHILL J.R. ET AL: 'A new gene family predicted by a novel human heart cDNA, 1995, ACCESSION: U47674' MOL. BIOL. CELL vol. 6, 1995, page 418A, XP002986942
- STRAUSBERG R.L. ET AL: 'Generation and initial analysis of more than 15,000 full-length human and mouse cDNA sequences, 2002, ACCESSION: NM_024307' PROC. NATL. ACAD. SCI. USA vol. 99, no. 26, 2002, pages 16899 - 16903, XP002298284
- SUNG C.K. ET AL: 'Molecular cloning of cDNA encoding human lanosterol synthase, 1995, ACCESSION: D63807' BIOL. PHAR. BULL. vol. 18, 1995, pages 1459 - 1461, XP002986943
- LAI K. ET AL: 'Estrogen Receptor Regulates Expression of the Orphan Receptor Small Heterodimer Partner, Sep 2003, ACCESSION: NM_021969' J. BIOL. CHEM. vol. 278, no. 38, September 2003, pages 36418 - 36429, XP002970741
- PELSERS M.M. ET AL: 'Intestinal-type and liver-type fatty acid-binding protein in the intestine. Tissue distribution and clinical utility, Oct. 2003, ACCESSION: NM_001443' CLIN. BIOCHEM. vol. 36, no. 7, October 2003, pages 529 - 535, XP002986944
- TSUJI A. ET AL: 'Hepsin, a cell membrane-associated protease. Characterization, tissue distribution, and gene localization, 1991, ACCESSION: NM_002151' J. BIOL. CHEM. vol. 266, no. 25, 1991, pages 16948 - 16953, XP002251018
- HUTCHINSON S. ET AL: 'Purification of human kallikrein 6 from biological fluids and identification of its complex with alpha ()-antichymotrypsin, may 2003, ACCESSION: NM_001085' CLIN. CHEM. vol. 49, no. 5, May 2003, pages 746 - 751, XP002969807
- WIEMANN S. ET AL: 'Toward a catalog of human genes and proteins: sequencing and analysis of 500 novel complete protein coding human cDNAs, 2001, ACCESSION : AL136653' GENOME RES. vol. 11, no. 3, 2001, pages 422 - 435, XP002221682
- KAYANO T. ET AL: 'Human facilitative glucose transporters. Isolation, functional characterization, and gene localization of cDNAs encoding an isoform (GLUT) expressed in small intestine, kidney, muscle, and adipose tissue and an unusual glucose transporter pseudogene-like sequence (GLUT6), 1990, ACCESSION : NM_ 006931' J. BIOL. CHEM. vol. 265, no. 22, 1990, pages 13276 - 13282, XP000978912
- SHIELDS J.M. ET AL: 'Loss of transgelin in breast and colon tumors and in RIE-1 cells by Ras deregulation of gene expression through Raf-independent pathways, 2002, ACCESSION: NM_ 003186' J. BIOL. CHEM. vol. 277, no. 12, 2002, pages 9790 - 9795, XP002986945

## Description

### Technical Field

The present invention relates to a method for predicting the toxicity of drugs and tools therefor. More particularly, the present invention relates to a method for predicting the toxicity of pharmaceutical candidate compounds (e.g., phospholipidosis induction potential etc.) with expression variations of marker genes as indices, and reagents, kits and the like for the detection of the toxicity marker genes.

### Background Art

In recent years, the bottleneck up to the optimization of a lead compound in drug discovery research has been shifting from the efficacy screening to the toxicity screening as a result of the introduction of combinatorial chemistry and high-throughput screening. Therefore, establishment of highly efficient toxicity evaluation or toxicity prediction system capable of contributing to the obliteration of such bottleneck is strongly demanded.
At present, toxicity evaluation of pharmaceutical candidate compounds is generally conducted by in vivo toxicity tests on administration of compounds to laboratory animals such as rat and the like. Such tests are defective in that they require (1) several days or months for the expression of toxicity, (2) compounds in large amounts and the like. Particularly, for rapid prediction of the presence or absence of toxicity and efficient optimization of structure in the initial stage of development when the synthesis amount of compound is limited, construction of an in vitro screening system capable of evaluating many samples in smaller amounts in a short time is essential.
Drug induced phospholipidosis (Phospholipidosis; hereinafter sometimes to be abbreviated as "PLsis") is defined to be a phenomenon where phospholipid accumulates in excess in the cell, and induced by many pharmaceutical agents such as antidepressants, antianginal drugs, antimalarial drugs, anti-anorectic drugs, hypolipidemic drugs and the like or metabolites thereof. In PLsis, phospholipid is mainly accumulated in lysosome and a cyclic or elliptic myelin-like structure (lamellar body) is observed electron microscopically. While the expression mechanism of toxicity has not been completely elucidated, it is considered to be caused by 1) inhibition of lysosomal enzyme (mainly phospholipid degradation enzyme (phospholipase)) activity by compound, 2) inhibition of transport pathway involved in phospholipid metabolism by compound, 3) inhibition of degradation of complex by the formation of a complex of compound and phospholipid, 4) promotion of synthesis of phospholipid by compound and the like.
Many of the PLsis-inducing compounds have a structure comprising, in a molecule, a hydrophobic domain and a positively electrically charged hydrophilic domain in combination (cationic amphiphilic drug; CAD). In recent years, along with the progress of genome analysis, the value of orphan receptors as drug discovery targets has been recognized and the development of agonists or antagonists against the receptors is being undertaken. However, because such compounds act on receptors, they mostly have CAD structures and an increasing number of incidents have been experienced where expression of PLsis prevents development of pharmaceutical products. Therefore, the development of an efficient, evaluation or prediction system of PLsis inducibility has been urgently desired.
In response, an evaluation method using phospholipase activity inhibition as an index (Matsuzawa, Y. and Hostetler, K.Y., J. Biol. Chem. (US), 1980, vol. 255 (No. 2), pp. 646-652), a detection method of phospholipid accumulation in hepatocyte or cultured lymphocyte using a fluorescence dye (Gum, R.J. et al., Biochem. Pharmacol. (UK), 2001, Vol. 62, pp. 1661-1673 and Xia, Z. et al., Biochem. Pharmacol. (UK), 1997, Vol. 53, pp. 1521-1532) and the like have been proposed. However, all of them are insufficient in terms of reliability and/or rapid performance and the like, and a practical in vitro screening system has not been established yet.
Incidentally, microarray technology for simultaneously monitoring expression of several thousand to several tens of thousand kinds of mRNAs (comprehensive gene expression analysis, transcriptomics) has been actively used in various kinds of fields of medicine and biology. In the field toxicology, too, this technology has been utilized for the elucidation of the mechanism of toxicity expression and the study of toxicity prediction, and is expected to be a new study field called toxicogenomics (Aardema, M.J. and MacGregor, J.T. et al., Mutat. Res. (Netherlands), 2002, Vol. 499, pp. 13-25). Toxicity phenomenon is considered to accompany not only independent changes in one or several genes, but also integral variations where many genes are correlated, such as interaction of genes, cascade and the like. Based thereon, it is expected that the behavior of molecules involved in toxicity expression can be comprehensively perceived by the use of a technique of microarray capable of analysis at transcriptome level. For example, WO02/10453 and WO02/095000 disclose methods to predict hepatotoxicity or kidney toxicity of a test compound, which comprises examining an expression amount of not less than 2 to 100 genes selected from an enormous group of genes in the presence of a test compound and comparing the results thereof with positive average and/or negative average expression amount (s) of respective genes previously calculated using known positive and negative compounds.

## Disclosure of the Invention

It is an object of the present invention to provide a high throughput method for predicting a PLsis induction potential.

As a result of a comprehensive analysis using a microarray of gene expression in human cultured cells exposed to various known PLsis-inducing compounds, the present inventors identified genes that showed remarkably varying expression for most of these compounds. From these, they extracted 12 genes having different functions and high expression variation rates and fully examined them by real-time quantitative PCR. As a result, these genes were confirmed to show varying expression in correlation with the level of emergence of a myelin-like structure or a structure having a high electron density, which is an early stage image thereof, by electromicroscopic observation. Furthermore, the present inventors perceived PLsis expression in correlation with comprehensive behavior of these marker genes, introduced the conception of average variation rate of expression, and succeeded in constructing a highly reliable in vitro evaluation system of a PLsis induction potential, which is associated with an extremely low false-positive and false-negative probability, which resulted in the completion of the present invention.

Accordingly, the present invention provides kits and methods as defined in the claims. The present disclosure further provides

[1] a reagent for predicting a phospholipidosis induction potential of a compound, which comprises a nucleic acid capable of hybridizing to a nucleic acid having a base sequence shown by any of SEQ ID NOs:1, 3, 5, 7, 9, 11, 13, 15, 17, 19, 21 and 23 under high stringent conditions and/or a nucleic acid capable of hybridizing to a nucleic acid having a base sequence complementary to the base sequence under high stringent conditions;

[2] a kit for predicting a phospholipidosis induction potential of a compound, which comprises one or more reagents containing a nucleic acid capable of hybridizing to a transcription product of a gene showing varying expression in correlation with expression of phospholipidosis under high stringent conditions and/or a nucleic acid capable of hybridizing to a nucleic acid having a base sequence complementary to the transcription product under high stringent conditions, wherein, when two or more reagents are contained, each reagent can detect expression of different genes;

[3] the kit of the above-mentioned [2], wherein at least one reagent comprises a nucleic acid capable of hybridizing to a nucleic acid having a base sequence shown by any of SEQ ID NOs:1, 3, 5, 7, 9, 11, 13, 15, 17, 19, 21 and 23 under high stringent conditions and/or a nucleic acid capable of hybridizing to a nucleic acid having a base sequence complementary to the base sequence under high stringent conditions;

[4] the kit of the above-mentioned [2], wherein a prediction hitting ratio of the phospholipidosis induction potential is not less than about 70% when a mammalian cell is exposed to a test compound, using an average variation rate of expression of a nucleic acid, to which the nucleic acid contained in each reagent is capable of hybridizing, in said cell as an index;

[5] a method for predicting a phospholipidosis induction potential of a compound, which comprises detecting expression variation of one or more genes showing expression variation in correlation with phospholipidosis expression, in a sample containing a mammalian cell exposed to the compound or a sample taken from a mammal administered with the compound;

[6] the method of the above-mentioned [5], wherein at least one gene has the same or substantially the same base sequence as the base sequence shown by any of SEQ ID NOs:1, 3, 5, 7, 9, 11, 13, 15, 17, 19, 21 and 23;

[7] a method for determining the standard for the judgment of the presence or absence of a phospholipidosis induction potential of a compound, which comprises

(1) detecting expression variation of one or more genes showing expression variation in correlation with phospholipidosis expression, in samples containing a mammalian cell exposed to each of two or more known phospholipidosis-inducing compounds and two or more known phospholipidosis non-inducing compounds or samples taken from mammals administered with each of said compounds, and

(2) using, as a standard value, an average variation rate capable of correctly judging the presence or absence of a phospholipidosis induction potential of the above-mentioned compounds by not less than about 70% based on the relationship between an average expression variation rate of the genes and the phospholipidosis induction potential;

[8] the method of the above-mentioned [7], wherein at least one gene has the same or substantially the same base sequence as the base sequence shown by any of SEQ ID NOs:1, 3, 5, 7, 9, 11, 13, 15, 17, 19, 21 and 23;

[9] the method of the above-mentioned [7], further comprising examining validity of the standard value using other known phospholipidosis inducing compound and known phospholipidosis non-inducing compound;

[10] the method of the above-mentioned [5], comprising comparing the average variation rate of gene expression with the standard value obtained by the method of the above-mentioned [7] or [9] ;

[11] a method for predicting the toxicity of a compound, which comprises,

(1) detecting expression variation of one or more genes showing expression variation in correlation with toxicity expression, in a sample containing a mammalian cell exposed to the compound or a sample taken from a mammal administered with the compound, and
(2) judging the presence or absence of toxicity of the compound with an average variation rate of the gene expression as an index;

and the like.

## Brief Description of the Drawings

Fig. 1 shows structural formulas, molecular weights, efficacy and addition concentrations of PLsis-inducing compounds (amiodarone, amitriptyline, AY-9944, chlorcyclizine, chlorpromazine and clomipramine).
Fig. 2 shows structural formulas, molecular weights, efficacy and addition concentrations of PLsis-inducing compounds (fluoxetine, imipramine, perhexiline, tamoxifen, thioridazine and zimelidine).
Fig. 3 shows structural formulas, molecular weights, efficacy and addition concentrations of PLsis-inducing compounds (clozapine, ketoconazole, loratadine, pentamidine and sertraline).
Fig. 4 shows structural formulas, molecular weights, efficacy and addition concentrations of PLsis non-inducing compounds (acetaminophen, clarithromycin, disopyramide, erythromycin, flecainide and haloperidol).
Fig. 5 shows structural formulas, molecular weights, efficacy and addition concentrations of PLsis non-inducing compounds (levofloxacin, ofloxacin, procainamide, quinidine, sotalol, sulfamethoxazole and sumatryptan).
Fig. 6 shows a correlation between an average variation rate of PLsis marker gene expression in HepG2 cells at 24 hr after addition of compound (vertical axis) and the frequency of emergence of a myelin-like structure in HepG2 cells at 72 hr after addition of compound (axis of abscissas), wherein +++: large myelin-like structure is found in plurality; ++: medium myelin-like structure is found in a small number; +: minor myelin-like structure is found in a small number; -: myelin-like structure is not found.
Fig. 7 shows reproducibility of the average variation rates of PLsis marker gene expression in HepG2 cells at 24 hr after addition of various compounds, wherein the axis of abscissas shows an average variation rate obtained by the first experiment, and the vertical axis shows an average variation rate obtained by the second experiment.

## Best Mode for Embodying the invention

The present disclosure provides a reagent for predicting a PLsis induction potential containing a nucleic acid capable of detecting the expression of a gene showing varying expression in correlation with PLsis expression (i.e., PLsis marker gene). As used herein, the "PLsis induction potential" means an ability to produce a myelin-like structure or a structure having a high electron density, which is an early image thereof, in a target mammalian cell upon contact with the compound. Therefore, even in the case of a compound inducing PLsis by in vivo administration, when in vivo metabolite alone induces PLsis, it is PLsis induction potential negative. Even in the case of a compound to be rapidly metabolized and neutralized in the body, when the compound itself induces PLsis, it is PLsis induction potential positive.
By "varying expression in correlation with PLsis expression" is meant a statistically significant tendency toward, upon exposure of mammalian cells to various compounds, substantial increase or decrease of expression when the compound produces a myelin-like structure or a structure having a high electron density, which is an early image thereof, in the cells, and substantially no variation of expression when the compound does not produce a myelin-like structure or a structure having a high electron density, which is an early image thereof, in the cells. By the "substantial increase or decrease" is meant an increase to not less than 1.5-fold of that by non-exposure or a decrease to not more than 2/3 of that by non-exposure, and by the "substantially no variation" is meant an expression level of 2/3 to 1.5-fold of that by non-exposure.
Specifically, as the PLsis marker gene, a gene encoding a lysosomal enzyme, a gene encoding a lipid metabolism (e.g., cholesterol synthesis, fatty acid elongation, unsaturated fatty acid synthesis etc.)-related protein, a gene encoding a transport (e.g., fatty acid transport, protein transport, amino acid transport etc.)-related protein, a gene encoding a cell growth-related protein, a gene encoding a protease or protease inhibitor, a gene encoding an amino acid metabolism-related protein, and the like can be mentioned. More specifically, as a gene showing an increased expression in correlation with PLsis extracted by the present invention, human genes containing base sequences registered with IDs of NM_014960, NM_000859, AL518627, NM_002130, AA639705, BC005807, AF116616, NM_025225, U47674, D80010, NM_001731, AW134535, NM_004354, AF135266, AC007182, NM_003832, NM_019058, AB040875, AA488687, NM_018687, NM_021158, BG231932, NM_024307, NM_000235, AA873600, D63807, AF096304, AW150953, NM_001360, NM_021969, AC001305, NM_024090, NM_001443, NM_006214, NM_024108, NM_021980, NM_002151, AF003934, NM_000596, U15979, M92934, NM_002087, AK023348, NM_002773, NM_000131, BC003169, NM_002217, NM_003122, NM_001673, NM_000050, NM_001085, U08024, NM_003167, BC005161, AF162690, AW517464, AF116616, UM_017983, AL136653, NM_016061, BE966922, BE552428, NM_022823, NM_012445, NM_000792, NM_015930, NM_021800, NM_005980, NM_000565 and AB033025 in GenBank database, and homologues thereof in other mammals and the like can be mentioned. On the other hand, as a gene showing a decreased expression in correlation with PLsis, human genes containing base sequences registered with IDs of NM_006931, AL110298, NM_006931, NM_001955, NM_003897, NM_003186, AA778684, NM_001283, NM_012242, AI934469, NM_003186 and NM_002450 in GenBank database, and homologues thereof in other mammals and the like can be mentioned.

Preferably, as a PLsis marker gene in the present invention, 12 kinds of genes having the same base sequences as shown in SEQ ID NOs:1, 3, 5, 7, 9, 11, 13, 15, 17, 19, 21 and 23, or substantially the same base sequences, can be mentioned. As used herein, by the "substantially the same base sequences" is meant base sequences capable of hybridizing to nucleic acids having complementary strand sequences of base sequences shown in SEQ ID NOs:1, 3, 5, 7, 9, 11, 13, 15, 17, 19, 21 and 23 under high stringent conditions, wherein the proteins encoded thereby are the same or substantially the same proteins encoded by the base sequences shown in said SEQ ID NOs. The "high stringent conditions" refers to the conditions under which nucleic acids having complementary strand sequences of base sequences shown in SEQ ID NOs:1, 3, 5, 7, 9, 11, 13, 15, 17, 19, 21 and 23 can hybridize with nucleic acids having base sequences showing complementarity of not less than about 70%, preferably not less than about 80%, more preferably not less than about 90%, particularly preferably not less than about 95%, in the overlapping regions, and, for example, a sodium concentration of about 19-40 mM, preferably about 19-20 mM, a temperature of about 50-70°C, preferably about 60-65°C, and particularly preferably, a sodium concentration of about 19 mM and a temperature of about 65°C can be mentioned. Those skilled in this field can easily obtain desired stringency by suitably changing a salt concentration of the hybridization solution, a temperature of hybridization reaction, a probe concentration, a probe length, a mismatch number, a hybridization reaction time, a salt concentration of the washing solution, a washing temperature, and the like.

The "substantially the same protein" refers to a protein having an amino acid sequence showing not less than about 70%, preferably not less than about 80%, more preferably not less than about 90%, particularly preferably not less than about 95%, most preferably not less than about 98%, homology to the amino acid sequence shown in SEQ ID NO:2, 4, 6, 8, 10, 12, 14, 16, 18, 20, 22 or 24 and the same level of activity as the protein having the amino acid sequence shown in the above-mentioned SEQ ID NO:2. The "same level of activity" means that the activity is qualitatively the same (e.g., physiologically or pharmacologically), and preferably, quantitatively equivalent (e.g., 0.5- to 2-fold), but may be different. As long as the homology conditions of amino acid sequence are satisfied, moreover, other quantitative elements such as molecular weight and the like may be different.

With regard to the amino acid sequence, a "homology" means a proportion (%) of the same amino acid residue and analogous amino acid residue to the whole amino acid residues overlapped in the optimal alignment (preferably, the algorithm is such that a gap can be introduced into one or both of the sequences for an optimal alignment) where two amino acid sequences are aligned using a mathematic algorithm known in the technical field. The "analogous amino acid" means amino acids having similar physiochemical properties, and for example, the amino acids are classified into groups such as an aromatic amino acid (Phe, Trp, Tyr), an aliphatic amino acid (Ala, Leu, Ile, Val), a polar amino acid (Gln, Asn), a basic amino acid (Lys, Arg, His), an acidic amino acid (Glu, Asp), an amino acid having a hydroxy group (Ser, Thr) and an amino acid having a small side-chain (Gly, Ala, Ser, Thr, Met). Substitution by such analogous amino acids is expected not to change the phenotype of proteins (i.e., conservative amino acid substitution). Specific examples of the conservative amino acid substitution are known in this technical field and described in various literatures (e.g., see Bowie et al., Science, 247: 1306-1310 (1990)).

Algorithms to determine a homology of amino acid sequence include, for example, but are not limited to, the algorithm as described in Karlin et al., Proc. Natl. Acad. Sci. USA, 90: 5873-5877 (1993) [the algorithm is incorporated into NBLAST and XBLAST programs (version 2.0) (Altschul et al., Nucleic Acids Res., 25: 3389-3402 (1997))], the algorithm as described in Needleman et al., J. Mol. Biol., 48: 444-453 (1970) [the algorithm is incorporated into a GAP program in a GCG software package], the algorithm as described in Myers and Miller, CABIOS, 4: 11-17 (1988) [the algorithm is incorporated into an ALIGN program (version 2.0) which is a part of a CGC sequence alignment software package], the algorithm as described in Pearson et al., Proc. Natl. Acad. Sci. USA, 85: 2444-2448 (1988) [the algorithm is incorporated into a FASTA program in a GCG software package], and the like.

More preferably, an amino acid sequence substantially the same as the amino acid sequence shown in SEQ ID NO:2, 4, 6, 8, 10, 12, 14, 16, 18, 20, 22 or 24 is an amino acid sequence having not less than about 70%, preferably not less than about 80%, more preferably not less than about 90%, homology to the amino acid sequence shown in any of said SEQ ID NOs.

**[0020]** The protein having such homology includes, for example, 1) an amino acid sequence shown in SEQ ID NO:2, 4, 6, 8, 10, 12, 14, 16, 18, 20, 22 or 24 wherein one or more (preferably about 1-30, more preferably about 1-10, particularly preferably several (1-5)) amino acids have been deleted, 2) an amino acid sequence shown in SEQ ID NO:2, 4, 6, 8, 10, 12, 14, 16, 18, 20, 22 or 24 wherein one or more (preferably about 1-30, more preferably about 1-10, particularly preferably several (1-5)) amino acids have been added, 3) an amino acid sequence shown in SEQ ID NO:2, 4, 6, 8, 10, 12, 14, 16, 18, 20, 22 or 24 wherein one or more (preferably about 1-30, more preferably about 1-10, particularly preferably several (1-5)) amino acids have been inserted, 4) an amino acid sequence shown in SEQ ID NO:2, 4, 6, 8, 10, 12, 14, 16, 18, 20, 22 or 24 wherein one or more (preferably about 1-30, more preferably about 1-10, particularly preferably several (1-5)) amino acids have been substituted by other amino acids, 5) a protein containing an amino acid sequence wherein the above-mentioned sequences have been combined, and the like.

When the amino acid sequence is inserted, deleted or substituted as mentioned above, the position of the insertion, deletion or substitution is not particularly limited.

More specifically, as a gene having a substantially the same base sequence as the base sequence shown in SEQ ID NOs:1, 3, 5, 7, 9, 11, 13, 15, 17, 19, 21 or 23, allele variants of a gene having the base sequence shown in any of these SEQ ID NOs, orthologs of the gene in non-human mammals (e.g., monkey , bovine, horse, swine, sheep, goat, dog, cat, rabbit, hamster, guinea pig, mouse, rat etc.) and the like can be mentioned.

The base sequences of human-derived PLsis marker gene of the present invention (i.e., base sequences shown in SEQ ID NOs:1, 3, 5, 7, 9, 11, 13, 15, 17, 19, 21 and 23) are all known, and published in the GenBank database under the accession Nos. of NM_014960, U47674, NM_024307, D63807, NM_021969, NM_001443, NM_002151, NM_001085, AL136653, NM_022823, NM_006931 and NM_003186, respectively.

A gene having the same or substantially the same base sequence as the base sequence shown in SEQ ID NO:1 (hereinafter sometimes to be abbreviated as "kiaa1001") encodes KIAA1001 protein, which is a lysosomal enzyme belonging to the sulfatase family.

A gene having the same or substantially the same base sequence as the base sequence shown in SEQ ID NO:3 (hereinafter sometimes to be abbreviated as "asah1") encodes N-acylsphingosine amidehydrolase (acidic ceramidase) 1, which is a lysosomal enzyme involved in ceramide metabolism and the lack of which causes Farber's disease (ceramide accumulation) in human.

A gene having the same or substantially the same base sequence as the base sequence shown in SEQ ID NO:5 (hereinafter sometimes to be abbreviated as "mgc4171") encodes MGC4171 protein belonging to the glycerophosphoryl diester phosphodiesterase family involved in phospholipid degradation.

A gene having the same or substantially the same base sequence as the base sequence shown in SEQ ID NO:7 (hereinafter sometimes to be abbreviated as "lss") encodes lanosterol synthase involved in cholesterol synthesis.

A gene having the same or substantially the same base sequence as the base sequence shown in SEQ ID NO:9 (hereinafter sometimes to be abbreviated as "nr0b2") encodes an intranuclear receptor protein (subfamily 0, group b, member 2) involved in the regulation of expression of cholesterol 7a-hydroxylase (CYP7A1).

A gene having the same or substantially the same base sequence as the base sequence shown in SEQ ID NO:11 (hereinafter sometimes to be abbreviated as "fabp1") encodes liver fatty acid binding protein-1 involved in lipid transport.

While the above-mentioned six genes encode lysosomal enzyme and lipid metabolism-related protein generally suggested to be related to PLsis induction, there is no report on the actual correlation between the expression of these respective genes and PLsis induction potential of a drug.

A gene having the same or substantially the same base sequence as the base sequence shown in SEQ ID NO:13 (hereinafter sometimes to be abbreviated as "hpn") encodes hepsin, which is a transmembrane serineprotease.

A gene having the same or substantially the same base sequence as the base sequence shown in SEQ ID NO:15 (hereinafter sometimes to be abbreviated as "serpina3") encodes serine (cysteine) protease inhibitor (grade A, member 3).

A gene having the same or substantially the same base sequence as the base sequence shown in SEQ ID NO:17 (hereinafter sometimes to be abbreviated as "depp") encodes a protein derived from a decidual membrane induced by progesterone.

A gene having the same or substantially the same base sequence as the base sequence shown in SEQ ID NO:19 (hereinafter sometimes to be abbreviated as "flj22362") encodes a protein (FLJ22362) having high homology to fibronectin type III.

A gene having the same or substantially the same base sequence as the base sequence shown in SEQ ID NO:21 (hereinafter sometimes to be abbreviated as "slc2a3") encodes a protein (family 2, member 3) belonging to a solute carrier family, which is a glucose transport carrier.

A gene having the same or substantially the same base sequence as the base sequence shown in SEQ ID NO:23 (hereinafter sometimes to be abbreviated as "tagln") encodes transgelin, which is a cytoskeletal protein.

The proteins encoded by these six genes are generally not known to have any correlation with PLsis induction at all.

The genes having the same or substantially the same base sequence as the base sequences shown in SEQ ID NOs:1, 3, 5, 7, 9, 11, 13, 15, 17 and 19 show increased expression in correlation with PLsis expression, and the genes having the same or substantially the same base sequence as the base sequences shown in SEQ ID NO:21 and 23 show decreased expression in correlation with PLsis expression.

As a nucleic acid capable of detecting the expression of a PLsis marker gene contained in the reagent for predicting a PLsis induction potential of the present disclosure (hereinafter sometimes to be abbreviated as "the reagent of the present disclosure"), for example, a nucleic acid (probe) capable of hybridizing to a transcription product of a PLsis marker gene, an oligonucleotide set capable of functioning as a primer amplifying a part or whole of the transcription product and the like can be mentioned. That is, as the nucleic acid, for example, a nucleic acid capable of hybridizing to a nucleic acid having a base sequence shown by any of SEQ ID NOs:1, 3, 5, 7, 9, 11, 13, 15, 17, 19, 21 and 23 (sense strand = coding strand) under high stringent conditions, and/or a nucleic acid capable of hybridizing to a nucleic acid having a base sequence complementary to the base sequence (antisense strand = noncoding strand) under high stringent conditions can be preferably mentioned. Being "capable of hybridizing under high stringent conditions" means as defined above. The nucleic acid may be a DNA or RNA, or a DNA/RNA chimera. Preferably, DNA can be mentioned.

The nucleic acid to be used as a probe may be double strand or single strand. In the case of a double-strand polynucleotide, it may be a double-strand DNA, a double-strand RNA or a DNA:RNA hybrid. In the case of a single strand, a sense strand (e.g., in case of cDNA, cRNA) or an antisense strand (e.g., in case of mRNA, cDNA) can be selected for use according to the sample to be used. The length of the nucleic acid is not particularly limited as long as it can specifically hybridize to a target nucleic acid and, for example, it is not less than about 15 bases, preferably not less than about 30 bases. The nucleic acid is preferably labeled with a label to enable detection or quantitation of a target nucleic acid.

As examples of the labeling agent, a radioisotope, an enzyme, a fluorescent substance, a luminescent substance and the like can be used. As examples of the radioisotope, [$^{32}P$], [$^{3}H$], [$^{14}C$] and the like can be used. As the enzyme, those that are stable and high in specific activity are preferred; for example, β-galactosidase, p-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of a probe and a labeling agent. For immobilization of a nucleic acid to be a probe on a solid phase, a nucleic acid in a sample can be labeled using a label similar to those mentioned above.

An oligonucleotide set to be used as a primer is not particularly limited as long as it can specifically hybridize to each of a base sequence shown in each SEQ ID NO (sense strand) and a base sequence complementary thereto (antisense strand), and amplify a DNA fragment sandwiched therebetween and, for example, a set of oligo DNAs each designed to have a length of about 15 to about 100 bases, preferably about 15 to about 50 bases, and amplify about 100 bp to several kbp DNA fragments can be mentioned.

For quantitative analysis of PLsis marker gene expression using a trace amount of an RNA sample, competitive RT-PCR or real-time RT-PCR is preferably used. Competitive RT-PCR is a method comprising calculating the amount of an object DNA by carrying out a competitive amplification reaction in a reaction mixture in the co-presence of a known amount of other template nucleic acid as a competitor, which can be amplified by a primer set capable of amplifying the object DNA, and comparing the amounts of the amplification products. When using competitive RT-PCR, therefore, the reagent of the present disclosure can further contain, besides the above-mentioned primer set, a nucleic acid which is amplified by the primer set to produce an amplification product distinguishable from the object DNA (e.g., amplification product different from the object DNA in size, amplification product showing different migration pattern by a restriction enzyme treatment and the like). This competitor nucleic acid may be a DNA or an RNA. In the case of a DNA, cDNA is synthesized from an RNA sample by a reverse transcription reaction and a competitor is added to perform PCR, and in the case of an RNA, it may be added to an RNA sample from the start to perform RT-PCR. In the latter case, an absolute amount of the original mRNA can also be assumed since the efficiency of the reverse transcription reaction is taken into consideration.

On the other hand, real-time RT-PCR does not require electrophoresis, since the amplification amount by PCR can be monitored real-time, and the expression of PLsis marker gene can be analyzed more rapidly. Generally, monitoring is performed using various fluorescent reagents. These include reagents (intercalator) emitting fluorescence by binding to double stranded DNA such as SYBR Green I, ethidium bromide and the like, nucleic acids usable as the above-mentioned probes (the nucleic acid hybridizes to the target nucleic acid within amplification region), wherein the both ends are respectively modified with a fluorescent substance (e.g., FAM, HEX, TET, FITC etc.) and a quenching substance (e.g., TAMRA, DABCYL etc.) and the like.

The nucleic acid functionable as a probe capable of detecting the expression of a PLsis marker gene can be obtained by amplifying a nucleic acid having a desired length by PCR using the above-mentioned primer set capable of amplifying a part or whole of a transcription product of the gene and using cDNA or genomic DNA of any cell (for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like) of a mammal (for example, human, monkey, bovine, horse, swine, sheep, goat, dog, cat, rabbit, hamster, guinea pig, mouse, rat and the like), or any tissue where such cells are present (for example, brain, any portion of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue, skeletal muscle, and the like) as a template, or cloning the above-mentioned PLsis marker gene or cDNA from cDNA or genomic DNA library derived from the aforementioned cell or tissue by colony or plaque hybridization and the like and, where necessary, treating same to give a fragment having a suitable length with a restriction enzyme and the like. The hybridization can be carried out according to the method described in, for example, Molecular Cloning, 2nd. ed. (mentioned above) and the like. When a commercially available library is used, hybridization can be carried out according to the method described in the instruction manual attached to the library. Alternatively, the nucleic acid can also be obtained by chemically synthesizing a part or whole of the base sequence and/or its complementary strand sequence based on the information of the base sequences shown in SEQ ID NOs:1, 3, 5, 7, 9, 11, 13, 15, 17, 19, 21 and 23 using a commercially available DNA/RNA automatic synthesizer and the like. In addition, a chip with a solid phased nucleic acid can also be prepared by direct *in situ* (on chip) synthesis of the nucleic acid on a solid phase such as silicone, glass and the like.

The nucleic acid functionable as a primer capable of amplifying a part or whole of a transcription product of a PLsis marker gene can also be obtained by chemically synthesizing a part of a base sequence and its complementary strand sequence, based on the information of the base sequences shown in SEQ ID NOs:1, 3, 5, 7, 9, 11, 13, 15, 17, 19, 21 and 23, using a commercially available DNA/RNA automatic synthesizer and the like.

The nucleic acid capable of detecting the expression of a PLsis marker gene can be provided as a solid in a dry state or in the form of an alcohol precipitate, or in a dissolution state in water or a suitable buffer (e.g., TE buffer etc.). When used as a labeled probe, the nucleic acid can be provided after labeling with any of the above-mentioned labeling substances, or provided independently of a labeling substance and labeled when in use.

Alternatively, the nucleic acid can be immobilized on a suitable solid phase. The solid phase is exemplified by, but not limited to, glass, silicone, plastic, nitrocellulose, nylon, polyvinylidene difluoride and the like. The solidifying means is exemplified by, but not limited to, a method comprising previously introducing a functional group such as amino group, aldehyde group, SH group, biotin and the like into a nucleic acid, introducing, into a solid phase, a functional group (e.g., aldehyde group, amino group, SH group, streptavidin and the like) reactive with the nucleic acid, and crosslinking the solid phase and the nucleic acid with covalent bond between these functional groups, or immobilizing a polyanionic nucleic acid with electrostatic bond using a polycation coating solid phase and the like.

One preferable embodiment of a nucleic acid probe immobilized on a solid phase is ArrayPlate™ etc. provided by High Throughput Genomics, Inc. ArrayPlate™ is a 96 well plate immobilized with various nucleic acid probes regularly disposed in the bottom of each well (e.g., 4x4 array). By the presence of a nucleic acid having one end hybridizable to a probe and the other end hybridizable to a target nucleic acid as a mediating spacer, a hybridization reaction of the probe with a target nucleic acid can be carried out in a liquid phase rather than on the solid phase surface, which enables a quantitative measurement of the target nucleic acid. Accordingly, expression variation of various PLsis marker genes can be simultaneously detected at once in a single well, and once sufficient quantitation performance is achieved, an advantage of higher efficiency than real-time PCR wherein expression variation in each marker gene is individually detected can be afforded.

While the reagent of the present disclosure has been explained with the emphasis on a nucleic acid capable of detecting a gene having the same or substantially the same base sequence as a base sequence shown by any of SEQ ID NOs:1, 3, 5, 7, 9, 11, 13, 15, 17, 19, 21 and 23, the reagent of the present disclosure may contain a nucleic acid capable of detecting a PLsis marker gene other than the above-mentioned 12 PLsis marker genes, such as a gene encoding a lysosomal enzyme, a gene encoding a lipid metabolism (e.g., cholesterol synthesis, fatty acid elongation, unsaturated fatty acid synthesis etc.)-related protein, a gene encoding a transport (e.g., fatty acid transport, protein transport, amino acid transport etc.)-related protein, a gene encoding a cell growth-related protein, a gene encoding a protease or protease inhibitor, a gene encoding an amino acid metabolism-related protein and the like, more specifically, human genes having base sequences registered in the GenBank database under the ID Nos. of NM_000859, AL518627, NM_002130, AA639705, BC005807, AF116616, NM_025225, D80010, NM_001731, AW134535, NM_004354, AF135266, AC007182, NM_003832, NM_019058, AB040875, AA488687, NM_018687, NM_021158, BG231932, NM_000235, AA873600, AF096304, AW150953, NM_001360, AC001305, NM_024090, NM_006214, NM_024108, NM_021980, AF003934, NM_000596, U15979, M92934, NM_002087, AK023348, NM_002773, NM_000131, BC003169, NM_002217, NM_003122, NM_001673, NM_000050, U08024, NM_003167, BC005161, AF162690, AW517464, AF116616, NM_017983, NM_016061, BE966922, BE552428, NM_012445, NM_000792, NM_015930, NM_021800, NM_005980, NM_000565, AB033025, AL110298, NM_006931, NM_001955, NM_003897, AA778684, NM_001283, NM_012242, AI934469, NM_003186 and NM_002450, homologues thereof in other mammals and the like.

The reagent of the present disclosure can further contain, in addition to a nucleic acid capable of detecting the expression of a PLsis marker gene, other substance necessary for detecting the expression of the gene, which does not adversely affect the reaction when preserved in coexistence. Alternatively, the reagent of the present disclosure can also be provided as a kit together with a separate reagent containing other substance necessary for the reaction for detecting the expression of a PLsis marker gene. For example, when the reaction for detecting the expression of a PLsis marker gene is PCR, as said other substance, for example, a reaction buffer, dNTPs, a heat resistance DNA polymerase and the like can be mentioned. When competitive PCR or real-time PCR is used, a competitor nucleic acid, a fluorescent reagent (the above-mentioned intercalator, fluorescence probe etc.) and the like can be further contained.

Individual PLsis marker genes are not such that they show expression variation for any PLsis-inducing compound and substantially no expression variation for any PLsis non-inducing compound. Therefore, when expression of each marker gene is used as a single index, emergence of a certain extent of false-positive and false-negative compounds is inevitable. However, by examining expression variation of plural PLsis marker genes, the prediction hitting ratio can be further improved.

Accordingly, the present invention also provides a kit for prediction of PLsis induction potential of a drug, which contains 12 reagents containing a nucleic acid capable of detecting a PLsis marker gene in combination, as defined in claim 1. The nucleic acid to be contained in each reagent can detect PLsis marker genes different from each other. At least one of the reagents contains a nucleic acid capable of detecting a gene having a base sequence the same or substantially the same as the base sequence shown by any of SEQ ID NOs:1, 3, 5, 7, 9, 11, 13, 15, 17, 19, 21 and 23.

The nucleic acid contained in each reagent constituting the kit is particularly preferably so constructed as to detect expression of a PLsis marker gene by the same method (e.g., northern blot, dot blot, DNA array technique, quantitative RT-PCR etc.).

Alternatively, as a combination of preferable marker genes, a combination affording a prediction hitting ratio of PLsis induction potential of not less than about 70%, more preferably not less than about 80%, further preferably not less than about 90%, and particularly preferably not less than about 95%, using, as an index, an average variation rate (explained in detail in the explanation of the PLsis induction potential prediction method below) of expression upon exposure of mammalian cells to a test compound can be mentioned. As used herein, by the hitting of prediction is meant that, when a compound predicted to be PLsis positive is exposed to a mammalian cell, a myelin-like structure or a structure having a high electron density, which is an early image thereof, is observed in the cell, and when a compound predicted to be PLsis negative is exposed to a mammalian cell, a myelin-like structure or a structure having a high electron density, which is an early image thereof, is not observed in the cell. In this specification, the prediction hitting ratio is calculated with the PLsis positive compounds described in Figs. 1-3 and the PLsis negative compounds described in Figs. 4-5 as standard compounds.

The constitution of the kit of the present invention is exemplified by, but not limited to, one wherein the above-mentioned reagents of the present disclosure are separately provided [e.g., when nucleic acid functions as a labeled probe (particularly dot blot analysis), a primer for PCR (particularly real-time quantitative PCR) etc.], one wherein nucleic acids capable of detecting the expression of different PLsis marker genes are contained in a single reagent [e.g., when nucleic acid functions as PCR (particularly, each marker gene can be distinguished by the size of an amplification product and the like), a labeled probe (particularly, each marker gene can be distinguished by the size of a transcription product by northern blot analysis) and the like], one wherein nucleic acids capable of detecting the expression of different PLsis marker genes are immobilized in separate regions of a single solid phase [e.g., when functions as a probe for hybridization to label cRNA etc., and the like] and the like.

The present invention also provides a method for predicting a PLsis induction potential of a compound, which comprises detecting expression variation of the 12 PLsis marker genes comprising the base sequences shown by SEQ ID NO: 1, 3, 5, 7, 9, 11, 13, 15, 17, 19, 21 and 23 when a test compound is exposed to a mammalian cell-containing sample or a human or non-human mammal.

As the compound to be tested by the method of the present invention, for example, candidate compounds of pharmaceutical agent or animal drug and the like can be mentioned. Particularly, application to many candidate compound groups synthesized in the initial stage of drug discovery is preferable, since many samples can be treated rapidly. In this case, cell-containing samples or non-human mammals are used as test subject. On the other hand, expression variation of a PLsis marker gene can be preferably used in the final phase of development of pharmaceutical products (clinical trial), since it can be determined using a cell-containing sample that can be easily obtained, such as blood and the like.

As a mammalian cell-containing sample, any cell of a mammal (for example, human, monkey, bovine, horse, swine, sheep, goat, dog, cat, rabbit, hamster, guinea pig, mouse, rat and the like), desirably a mammal to be the administration subject of a test compound (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or corresponding precursor cell, stem cell or cancer cell thereof, and the like), or any tissue where such cells are present (for example, brain, any portion of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue, skeletal muscle, and the like), a cell line established from the above-mentioned cell or tissue and the like can be mentioned. Preferably, hepatocyte, renal cells, monocyte, peripheral blood lymphocyte, fibroblast, adrenal gland steroid-producing cells, testis cell, ovary cell, abdominal cavity macrophage, alveolar epithelial cell, bronchial epithelial cell, alveolar macrophage and the like can be mentioned. In addition, use of a cell line is preferable in view of superior reproducibility (particularly in the case of human cell), easy availability and the like. As a human cell line, for example, liver cancer-derived HepG2 cell line, lymphoma-derived U-937 cell line, monocyte-derived THP-1 cell line, colorectal cancer-derived Caco-2 cell line, cervical cancer-derived HeLa cell line and the like can be mentioned.

The non-human mammal is exemplified by, but not limited to, rat, hamster, guinea pig, rabbit, mouse, monkey, dog, swine, cat, sheep, goat, horse, bovine and the like. Preferred are rat, hamster, guinea pig, rabbit, mouse, monkey, dog and the like.

While a method for exposing a mammalian cell-containing sample to a test compound is not particularly limited, specifically when, for example, a cell line is used as a sample, cells in a cell growth phase, which were cultured in a suitable medium under preferable conditions are detached using trypsin-EDTA and the like, recovered by centrifugation, and a suitable medium [e.g., MEM medium (Science, 122: 501 (1952)), DMEM medium (Virology, 8: 396 (1959)), RPMI 1640 medium (The Journal of the American Medical Association, 199:519 (1967)), 199 medium (Proceeding of the Society for the Biological Medicine, 73: 1 (1950)) containing about 5 to about 20% fetal bovine serum (FBS) and the like (where necessary, antibiotics such as penicillin, streptomycin, hygromycin and the like may be further added)] is added to give a suspension at a desired cell density. While the cell density is not particularly limited as long as the gene expression and its variation can be detected, it is preferable to so control the cells as to keep the cell growth phase. Therefore, preferable initial cell density varies depending on the growth rate of the cells to be used and the like, which can be easily determined by those of ordinary skill in the art according to the cells to be used. It is generally about $5\times10^4$ - about $1\times10^7$ cells/mL. A test compound dissolved in a suitable solvent is further diluted with the medium, added to the above-mentioned cell suspension to a final concentration of, for example, the highest concentration at which the cells can survive (the concentration can be determined separately by histological observation), and cultivated under conventional conditions, for example, in a $CO_2$ incubator under an atmosphere of 5% $CO_2$/95% air, 5% $CO_2$/5% $O_2$/90% air and the like, at about 30°C - about 40°C for about 0.5 - about 168 hr, preferably about 3 - about 48 hr, more preferably about 23 - about 25 hr.

A method for exposing a mammal to a test compound is not particularly limited as long as it includes administration of the test compound to the animal such that a sufficient amount of the test compound reaches the target cell (cell contained in a sample to be recovered later from the animal to examine expression variation of a PLsis marker gene) and, for example, the test compound can be administered in the form of a solid, a semi-solid, a liquid, an aerosol and the like orally or parenterally (e.g., intravenously, intramuscularly, intraperitoneally, intraarterially, subcutaneously, intradermally, airway etc.). The dose of the test compound varies depending on the kind of the compound, animal species, body weight, administration form and the like and, for example, an amount within the range where the animal can survive and necessary for the target cell to be exposed to the highest concentration of the test compound, at which the cell can survive, for more than a given time and the like can be mentioned. The dose can be administered in one to several portions. While the time from the administration to the sample collection varies depending on the in vivo kinetics of the test compound and the like, it is generally about 3 hr to about 3 days from the initial administration.

As a sample to be taken from a mammal administered with the test compound, those containing various cells exemplified as the mammalian cell-containing sample can be preferably mentioned. Blood (e.g., peripheral blood) and the like are particularly preferable since they can be recovered rapidly and conveniently, invasion into an animal is few and the like.

The PLsis marker gene whose expression variation is examined in the prediction method of the present invention is not particularly limited and, for example, a gene encoding a lysosomal enzyme, a gene encoding a lipid metabolism (e.g., cholesterol synthesis, fatty acid elongation, unsaturated fatty acid synthesis etc.)-related protein, a gene encoding a transport (e.g., fatty acid transport, protein transport, amino acid transport etc.)-related protein, a gene encoding a cell growth-related protein, a gene encoding a protease or protease inhibitor, a gene encoding an amino acid metabolism-related protein and the like can be mentioned. More specifically, as a gene that shows an increased expression in correlation with PLsis, human genes having base sequences registered in the GenBank database under the ID Nos. of NM_014960, NM_000859, AL518627, NM_002130, AA639705, BC005807, AF116616, NM_025225, U47674, D80010, NM_001731, AW134535, NM_004354, AF135266, AC007182, NM_003832, NM_019058, AB040875, AA488687, NM_018687, NM_021158, BG231932, NM_024307, NM_000235, AA873600, D63807, AF096304, AW150953, NM_001360, NM_021969, AC001305, NM_024090, NM_001443, NM_006214, NM_024108, NM_021980, NM_002151, AF003934, NM_000596, U15979, M92934, NM_002087, AK023348, NM_002773, NM_000131, BC003169, NM_002217, NM_003122, NM_001673, NM_000050, NM_001085, U08024, NM_003167, BC005161, AF162690, AW517464, AF116616, NM_017983, AL136653, NM_016061, BE966922, BE552428, NM_022823, NM_012445, NM_000792, NM_015930, NM_021800, NM_005980, NM_000565 and AB033025, homologues thereof in other mammals and the like can be mentioned. On the other hand, as a gene that shows a decreased expression in correlation with PLsis, human genes having base sequences registered in the GenBank database under the ID Nos. of NM_006931, AL110298, NM_006931, NM_001955, NM_003897, NM_003186, AA778684, NM_001283, NM_012242, AI934469, NM_003186 and NM_002450, homologues thereof in other mammals and the like can be mentioned.

Preferably, at least one of the PLsis marker genes capable of examining the expression variation of the prediction method of the present invention contains a base sequence the same or substantially the same as the base sequence shown by any of SEQ ID NOs:1, 3, 5, 7, 9, 11, 13, 15, 17, 19, 21 and 23.

Alternatively, as a combination of preferable marker genes, a combination affording a prediction hitting ratio of PLsis induction potential of not less than about 70%, more preferably not less than about 80%, further preferably not less than about 90%, and particularly preferably not less than about 95%, using, as an index, an average variation rate of expression of all marker genes by the present prediction method can be mentioned. In the prediction method of the present invention, the "average variation rate" is defined as follows. That is, an expression amount is measured for each marker gene when mammal (cells) is and is not exposed to a test compound and when the expression amount increased upon exposure, its magnification (e.g., 2 when increased two-fold) is taken as an expression variation rate (X) of each gene, and when the expression amount decreased, an inverse number of its magnification (e.g., 2 when decreased to 1/2) is taken as an expression variation rate (X) of each gene, and an average value of the expression variation rate of the total marker genes (n genes) is defined to be an average variation rate (following formula).

$$\text{Average variation rate} = m_1 X_1 + m_2 X_2 + \cdots + m_n X_n$$

$$(m_1 + m_2 + \cdots + m_n = 1)$$

wherein $m_i$(i=1-n) shows the weight of each gene. While the weight is not particularly limited, it is preferably $m_i \times n$=0.2-5, for example, it is the same weight for all ($m_i$=1/n).

In the method of the present invention, when the average variation rate is not less than the standard value determined by the below-mentioned method, PLsis positive is predicted and when it is less than the standard value, PLsis negative is predicted.

The expression of a PLsis marker gene in a sample containing a mammalian cell exposed to the test compound and a sample taken from a mammal administered with the test compound can be examined by preparing an RNA (e.g., total RNA, mRNA) fraction from the sample, and detecting a transcription product of the marker gene contained in the fraction. An RNA fraction can be prepared by a known means such as guanidine-CsCl ultracentrifugation method, AGPC method and the like. A high purity total RNA can be prepared rapidly and conveniently from a trace sample using a commercially available RNA extraction kit (e.g., RNeasy Mini Kit; manufactured by QIAGEN etc.). As a means for detecting a transcription product of a PLsis marker gene in an RNA fraction, for example, a method using hybridization (northern blot, dot blot, DNA chip analysis etc.), a method using PCR (RT-PCR, competitive PCR, real-time PCR etc.) and the like can be mentioned. Since expression variation of a PLsis marker gene can be detected rapidly and conveniently with high quantitation performance from a trace sample, quantitative PCR such as competitive PCR, real-time PCR and the like, and since expression variation of multiple marker genes can be detected at once and quantitation performance can be improved by the selection of a detection method and the like, DNA chip analysis is preferable.

In the case of northern blot or dot blot hybridization, a PLsis marker gene can be detected using the above-mentioned reagent or kit of the present invention containing a nucleic acid to be used as a labeled probe. In the case of Northern hybridization, therefore, an RNA fraction prepared as mentioned above is separated by gel electrophoresis, transferred to a membrane such as nitrocellulose, nylon, polyvinylidene difluoride and the like, hybridized under the above-mentioned "high stringent conditions" in a hybridization buffer containing the reagent of the present disclosure or each reagent contained in the kit of the present invention, the amount of a label bonded to the membrane by a suitable method is measured for each band, whereby the expression amount of each PLsis marker gene can be measured. In the case of dot blot, too, a membrane spotted with an RNA fraction is subjected to a hybridization reaction in the same manner (conducted for each PLsis marker gene), and the label amount of the spot is measured, whereby the expression amount of each marker gene can be measured.

In the case of DNA chip analysis (solid phased probe described for the above-mentioned reagent of the present disclosure), for example, cDNA incorporating a suitable promoter such as T7 promoter and the like is synthesized by reverse transcription reaction from the RNA fraction prepared as mentioned above, and cRNA is synthesized using an RNA polymerase (where labeled cRNA is obtained by using, as a substrate, a mononucleotide labeled with biotin and the like). The labeled cRNA is contacted with the above-mentioned solid phased probe to allow hybridization reaction, and a label amount bonded to each probe on the solid phase is measured, whereby the expression amount of each PLsis marker can be measured. This method is advantageous in terms of rapidness and convenience as the number of PLsis marker genes (i.e., probes to be solid phased) to be detected increases.

In a preferable embodiment of the prediction method of the present invention, as a method for detecting the expression of a PLsis marker gene, quantitative PCR is used. As quantitative PCR, for example, competitive PCR, real-time PCR and the like can be mentioned. Since electrophoresis after amplification reaction is not necessary, real-time PCR is more superior in rapid performance.

In the case of competitive PCR, a known amount of a competitor nucleic acid is used, which can be amplified by said primer set in addition to the primer set described for the above-mentioned reagent of the present disclosure, and which, after amplification, can be distinguished from an amplification product of a target nucleic acid (i.e., a transcription product of a PLsis marker gene), based on different amplification size, different migration pattern of restriction enzyme-treated fragment and the like. Since amplification occurs competitively where a target nucleic acid and a competitor nucleic acid compete for a primer, the amount ratio of the amplification products reflects the amount ratio of the original templates. The competitor nucleic acid may be DNA or RNA. In the case of DNA, cDNA is synthesized from an RNA fraction prepared as mentioned above by reverse transcription reaction, and then PCR is performed in the coexistence of the reagent of the present disclosure and a competitor, and in the case of RNA, a competitor is added to an RNA fraction to allow reverse transcription reaction, and the reagent of the present disclosure is further added to perform PCR.

In real-time PCR, the amplification amount is monitored in real-time using a fluorescent reagent, and an apparatus integrally comprising a thermal cycler and a spectrofluorophotometer is necessary. Such apparatus is commercially available. There are several methods depending on the fluorescent reagent to be used and, for example, intercalator method, TaqMan™ probe method, Molecular Beacon method and the like can be mentioned. In any case, cDNA is synthesized by reverse transcription reaction from an RNA fraction prepared as mentioned above, and the reagent of the present disclosure and a fluorescent reagent (probe) called intercalator, TaqMan™ probe or Molecular Beacon probe is added to each PCR reaction system. Since intercalator binds to a synthesized double stranded DNA and emits fluorescence upon irradiation of excitation light, the amount of an amplification product can be monitored by measuring the intensity of fluorescence, based on which the amount of original template cDNA can be assumed. The TaqMan™ probe is an oligonucleotide capable of hybridizing to an amplification region of the target nucleic acid, which has both ends modified by a fluorescent substance and a quenching substance, respectively. It hybridizes to a target nucleic acid during annealing but is prohibited from emitting fluorescence by the presence of the quenching substance, and emits fluorescence when decomposed by the exonuclease activity of DNA polymerase during elongation, which liberates the fluorescent substance. Accordingly, by measuring fluorescence intensity, the amount of the amplification product can be monitored, based on which the amount of original template cDNA can be assumed. The Molecular Beacon probe is an oligonucleotide capable of hybridizing to an amplification region of a target nucleic acid and having a hairpin type secondary structure, which has both ends modified by a fluorescent substance and a quenching substance, respectively. When it has a hairpin structure, it does not emit fluorescence due to the presence of a quenching substance, and emits fluorescence when the distance between the fluorescent substance and the quenching substance grows upon hybridization to the target nucleic acid during annealing. Therefore, the amount of the amplification product can be monitored by measuring the fluorescence intensity, based on which the amount of original template cDNA can be assumed.

In the prediction method of the present invention, the standard according to which the presence or absence of a PLsis induction potential of a compound is judged is not particularly limited as long as the prediction results based on the standard have sufficient reliability for use as a compound screening system. For example, (1) a method for judging PLsis positive when expression of all PLsis marker genes to be the detection target substantially increases or decreases (where "expression substantially increases or decreases" means as defined above) due to exposure to the test compound, and judging PLsis negative when expression of any of the PLsis marker genes does not substantially change (where "expression does not substantially change" means as defined above) due to exposure to the test compound, (2) a method for judging PLsis negative when expression of all PLsis marker genes to be the detection target does not substantially change due to exposure to the test compound, and judging PLsis positive when expression of any of PLsis marker genes substantially increases or decreases due to exposure to the test compound, (3) a method for judging PLsis positive when expression of not less than a certain number (e:g., 2-(n-1) genes) out of PLsis marker genes in the number of n to be the detection target substantially increases or decreases due to exposure to the test compound and the like can be mentioned. However, according to the above-mentioned method (1), the frequency of appearance of false-positive compounds can be reduced, but the frequency of appearance of false-negative compounds increases and a considerable number of PLsis-inducing compounds cannot be eliminated. On the other hand, according to the method of (2), the frequency of appearance of false-negative compounds can be reduced, but the frequency of appearance of false-positive compounds increases, which possibly eliminates potential compounds and the room of development of pharmaceutical products and the like may be narrowed.

The present description provides a method for determining the judgment standard for maximally improving the reliability (prediction hitting ratio) system for the combination of selected PLsis marker genes. That is, this method comprises exposing mammalian cell-containing samples or humans or non-human mammals to each of not less than 2 (preferably not less than 5, more preferably not less than 10, further preferably not less than 15) known PLsis-inducing compounds (e.g., compounds described in Figs. 1-3) and not less than 2 (preferably not less than 5, more preferably not less than 10, further preferably not less than 15) known PLsis non-inducing compounds (e.g., compounds described in Figs. 4-5), detecting expression variation of one or more selected PLsis marker genes in the samples or samples taken from the mammals, and comparing the average expression variation rate of the marker genes (here, the "average variation rate" is as defined above) with the presence or absence of actual PLsis induction potential. In the present invention, the presence or absence of actual PLsis induction potential is determined by the appearance of a myelin structure or a structure having a high electron density, which is an early image thereof, in the cell on exposure of mammalian cell to a compound.

As a result of comparison, an average variation rate capable of correctly judging the presence or absence of the PLsis inducing property of the above-mentioned known PLsis inducing and non-inducing compounds with the probability of not less than about 70%, preferably not less than about 80%, more preferably not less than about 90%, particularly preferably not less than about 95%, is determined and used as a standard value. For example, when expression variation of 12 PLsis marker genes having the base sequences shown in SEQ ID NOs:1, 3, 5, 7, 9, 11, 13, 15, 17, 19, 21 and 23, respectively, is examined with regard to the compounds described in Figs. 1-5, an average variation rate (weight of each gene is the same) is as shown in Fig. 6, and an average variation rate of 1.5 that can evaluate all of 17 kinds of PLsis-inducing compounds as positive and 12 kinds out of 13 kinds of PLsis non-inducing compounds as negative (therefore, evaluation with about 97% probability of correctness) can be determined as the standard value.

The standard value determined as mentioned above is more preferably examined for its validity by further comparing an average variation rate of a PLsis marker gene expression and the presence or absence of actual PLsis induction potential in a similar manner using known PLsis inducing and non-inducing compounds. When an average variation rate capable of correctly evaluating the presence or absence of a PLsis induction potential more accurately with higher probability can be obtained from overall evaluation results of newly examined compounds, the standard value only needs to be amended. Moreover, by accumulating the data relating to known compounds by evaluation by the present method and microscopic observation, extremely accurate prediction of compound groups unknown as to the presence or absence of a PLsis induction potential becomes possible.

The concept of the average variation rate to be preferably used as an index of the PLsis prediction method of the present invention can be applied to other toxicity prediction methods of compounds using an exhaustive gene expression analysis. For example, as a prediction method of hepatotoxicity (e.g., inducibility such as hepatitis, liver necrosis, fatty liver and the like), a screening system capable of predicting hepatotoxicity of a test compound with precision can be constructed by exposing hepatocytes to several kinds to several dozen kinds of known hepatotoxic compounds (e.g., acetaminophen, amitriptyline, ANIT, carbon tetrachloride, cyproterone acetate, estradiol, indomethacin etc.), extracting RNA, synthesizing cDNA and then labeled cRNA by a conventional method, fragmenting them, performing an exhaustive gene expression analysis using a commercially available mammal genome DNA microarray (e.g., GeneChip (trademark) manufactured by Affymetrix and the like), identifying, for example, gene groups wherein not less than half of the compounds examined showed common variation of expression as hepatotoxic marker genes, selecting some from these marker genes, and examining the average variation rate of marker gene expression upon exposure of the hepatocytes to the test compound in the same manner as above.

Abbreviations for bases, amino acids and the like used herein are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.

| | |
|---|---|
| DNA: | Deoxyribonucleic acid |
| cDNA: | Complementary deoxyribonucleic acid |
| A: | Adenine |
| T: | Thymine |
| G: | Guanine |
| C: | Cytosine |
| RNA: | Ribonucleic acid |
| mRNA: | Messenger ribonucleic acid |

dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyrroglutamic acid
Sec: Selenocysteine

**Examples**

The present invention is explained in more detail in the following by referring to Examples, which are mere examples and do not limit the scope of the present invention which is defined by the enclosed claims.

[Example 1]

Reference Example: Electromicroscopic examination of PLsis induction potential of compound

Using 30 kinds of the following commercially available drugs as test compounds, the level of PLsis induction potential was examined with the emergence of an intracellular myelin-like structure or a structure having a high electron density, which is an early image thereof, as an index by electronmicroscopic observation. Amiodarone and clozapine were purchased from ICN Biomedicals, imipramine, clarithromycin, disopyramide, erythromycin, haloperidol, ketoconazole, quinidine, sertraline and sulfamethoxazole were purchased from Wako Pure Chemical Industries, Ltd., amitriptyline, AY-9944, chlorcyclizine, chlorpromazine, clomipramine, fluoxetine, perhexiline, tamoxifen, thioridazine, zimelidine, acetaminophen, flecainide, ofloxacin and sotalol were purchased from Sigma, levofloxacin was purchased from Apin Chemicals, loratadine and sumatriptan were purchased from KEMPROTEC, pentamidine was purchased from Tronto Research Chemicals and procainamide was purchased from Aldrich Chemical.
The test compound was dissolved in dimethylsulfoxide (DMSO) to the final concentration (separately, cells were exposed to the compound for 72 hr and the highest concentration at which the cells were viable was employed) of 8.3-25 μmol/L. The structural formulas, molecular weights, efficacy, addition concentrations of the test compounds are shown in Figs. 1-5.
HepG2 cells (purchased from ATCC) were exposed to the test compound by a conventional method. HepG2 cells used were of the cell growth phase. The attached HepG2 cells were washed twice with Dulbecco's phosphate buffered saline (calcium and magnesium salt free; PBS(-)) (Dainippon Pharmaceutical Co., Ltd.) containing 0.05 w/v% EDTA, detached with a cell dissociation solution of 0.25 volt trypsin-1 mmol/L EDTA (Gibco BRL) diluted 2-fold with PBS (-), centrifuged to remove the supernatant, and adjusted to a concentration of $2x10^5$ cells/250 μL with a culture medium [Dulbecco's modified Eagle medium (DMEM) (Gibco BRL) supplemented with 50 U/mL penicillin (Gibco BRL).-50 mmol/L streptomycin (Gibco BRL) and 5 vol% FBS (Bio whittaker)]. DMSO alone or a culture medium containing the above-mentioned test compound in DMSO solution was dispensed to a well by 250 μL, the above-mentioned cell suspension (250 μL) was added, and cultured in a $CO_2$ incubator (7100; Napco) under an atmosphere of 5% $CO_2$-95% air at 37°C.

After culture for 72 hr, the culture medium was removed, and the cells were fixed with 1 w/v% glutaraldehyde solution. After fixing with 2 w/v% osmic acid for 2 hr by a conventional method and dehydrating with an alcohol series, the cells were embedded in a resin (Quetol 812). Ultrathin slices were prepared and, after electron staining, observed with an electron microscope (H-300; Hitachi) and photographed in 3 or more pictures per each sample (magnification ×5000). The processing after post-fixation was performed by Applied Medical Research. The electron micrographs were grossly observed and the level of appearance of a myelin-like structure was divided into 4 classes of heavy, moderate, light, no change by a blind test (n=4). In the classification criteria, "heavy" means multiple large myelin-like structures, "medium" means a small number of moderate level myelin-like structures, "light" means a small number of minor myelin-like structures, and "no change" means absence of a myelin-like structure.

As a result, all of 12 known PLsis-inducing compounds (compounds shown in Figs. 1 and 2), and 5 compounds out of 18 compounds for examination of evaluation system (compounds shown in Fig. 3) showed a myelin-like structure, which is a typical PLsis image, in lysosome. In contrast, 13 compounds (compounds shown in Figs. 4 and 5) out of the compounds for examination of evaluation system showed no change in lysosome. The ranking of myelin-like structure appearance level is shown in Table 1 (addition concentration of compound shows the highest concentration of cells that survived after 72 hr of culture).

Table 1

| frequency of appearance of myelin-like structure | compound | addition concentration (μmol/L) |
|---|---|---|
| severe | amitriptyline | 25 |
| | chlorcyclizine | 25 |
| | fluoxetine | 8.3 |
| moderate | amiodarone | 8.3 |
| | AY-9944 | 8.3 |
| | chlorpromazine | 8.3 |
| | imipramine | 25 |
| | tamoxifen | 8.3 |
| | perhexiline | 8.3 |
| | clozapine | 25 |
| | sertraline | 8.3 |
| mild | clomipramine | 8.3 |
| | thioridazine | 8.3 |
| | zimelidine | 25 |
| | ketoconazole | 8.3 |
| | loratadine | 8.3 |
| | pentamidine | 8.3 |
| no change | solvent | - |
| | acetaminophen | 25 |
| | clarithromycin | 25 |
| | disopyramide | 25 |
| | erythromycin | 25 |
| | flecainide | 25 |
| | haloperidol | 8.3 |
| | levofloxacin | 25 |
| | ofloxacin | 25 |
| | procainamide | 25 |
| | quinidine | 25 |
| | sotalol | 25 |
| | sulfamethoxazole | 25 |
| | sumatriptan | 25 |

[Example 2]

Expression variation of various genes due to exposure to known PLsis inducing and non-inducing compounds

In the same manner as in Example 1 (Reference Example), HepG2 cells were exposed to 17 kinds of PLsis-inducing compounds and 14 kinds of PLsis non-inducing compounds for 24 hr each, the culture medium was removed and the cells were cryopreserved at -80°C. Using RNeasy Mini Kit (QIAGEN), total RNA was purified from the cells, and cDNA was synthesized in a 100 μL system using TaqMan Reverse Transcription Reagents (PE Applied Biosystems).

Based on the base sequences shown in SEQ ID NOs:1, 3, 5, 7, 9, 11, 13, 15, 17, 19, 21 and 23, primer and FAM labeled-probe were designed using Primer Express (PE Applied Biosystems) and synthesized (committed to SIGMA Genosys Japan). The sequences of respective primers and probes are respectively shown in SEQ ID NOs:25-60. As the primer for glyceraldehyde-3-phosphoric acid dehydrogenase (GAPDH) and VIC labeled probe, those attached to TaqMan GAPDH Control Reagents (PE Applied Biosystems) were used.

Using 100 μL of the reaction mixture containing 5 μL of cDNA (1x TaqMan Universal PCR Master Mix (PE Applied Biosystems), 200 nM forward primer, 200 nM reverse primer and 200 nM TaqMan probe), 40 cycles of (1 cycle=95°C, 15 sec; 60°C, 1 min) PCR were performed. PCR and fluorescence detection were performed using ABI PRISM Sequence Detector 7000 (PE Applied Biosystems). As the internal standard, GAPDH was used to amend the measurement values. For the determination of significant difference, t-test was used (n=3).

For each test compound, the expression variation rate of 12 genes each relative to the control group was determined. The results are shown in Table 2. In all the 12 genes examined, there was a tendency toward variation in their expression due to the exposure to a PLsis-inducing compound, and substantially no variation in their expression due to the exposure to a PLsis non-inducing compound. Therefore, it has been clarified that these 12 genes are marker genes useful for the prediction of a PLsis induction potential of a drug.

(Table 2)

| electro-microscopic test | | frequency of appearance of myelin-like structure[a] | | | | | | | | | | |
|---|---|---|---|---|---|---|---|---|---|---|---|---|
| | | +++ | | | ++ | | | | | | | |
| real-time quantitative PCR | gene[b] | expression variation rate[c] | | | | | | | | | | |
| | | amitriptyline | chlorcyclizine | fluoxetine | amiodarone | AY-9944 | chlorpromazine | imipramine | perhexiline | tamoxifen | clozapine | sertraline |
| | kiaa1001 | 2.11 * | 2.47 ** | 1.93 ** | 1.56 | 1.68 * | 1.90 * | 2.11 ** | 1.98 ** | 1.85 ** | 3.34 * | 2.38 * |
| | asah1 | 3.10 * | 3.23 * | 6.55 * | 1.69 | 4.71 * | 1.29 | 4.52 | 3.12 | 4.50 ** | 2.75 | 3.08 |
| | mgc4171 | 2.22 ** | 2.43 ** | 1.34 | 1.32 | 1.63 | 1.74 * | 1.81 * | 1.93 * | 1.60 | 1.88 | 1.94 |
| | lss | 1.79 ** | 2.98 ** | 3.51 | 1.82 | 4.78 * | 1.99 ** | 2.69 ** | 3.10 ** | 3.01 ** | 2.69 ** | 3.91 ** |
| | nr0b2 | 1.64 | 2.46 * | 2.70 | 1.35 | 2.71 ** | 1.20 | 3.41 * | 2.09 * | 2.56 ** | 1.97 | 1.79 |
| | fabp1 | 2.39 ** | 2.62 ** | 2.80 * | 2.01 ** | 3.16 ** | 2.56 ** | 2.45 ** | 2.49 ** | 1.90 ** | 3.89 ** | 4.14 * |
| | hpn | 2.20 * | 2.97 * | 2.39 ** | 2.15 ** | 2.68 ** | 1.70 ** | 2.32 ** | 2.22 * | 2.19 * | 2.35 ** | 3.07 ** |
| | serpina3 | 3.01 ** | 2.23 ** | 1.73 ** | 1.17 | 1.97 * | 1.94 ** | 2.12 ** | 2.50 ** | 2.12 ** | 1.96 * | 2.69 * |
| | depp | 5.08 ** | 3.24 * | 3.08 * | 1.12 | 3.32 ** | 2.95 ** | 3.36 ** | 2.55 ** | 2.02 | 5.15 ** | 4.44 * |
| | flj22362 | 1.80 ** | 2.96 ** | 2.15 ** | 1.07 | 1.45 | 1.70 * | 1.80 * | 3.42 ** | 1.53 | 1.92 ** | 3.49 * |
| | slc2a3 | 0.22 * | 0.20 * | 0.09 * | 0.35 * | 0.22 * | 0.43 | 0.15 * | 0.22 * | 0.24 * | 0.24 ** | 0.14 * |
| | tagln | 0.23 ** | 0.30 ** | 0.20 * | 0.56 * | 0.21 * | 0.65 * | 0.15 * | 0.26 ** | 0.27 * | 0.24 ** | 0.37 ** |

| electro-microscopic test | | frequency of appearance of myelin-like structure[a] | | | | | | | | | | | |
|---|---|---|---|---|---|---|---|---|---|---|---|---|---|
| | | + | | | | | | | | | | | |
| real-time quantitative PCR | gene[b] | expression variation rate[c] | | | | | | | | | | | |
| | | clomipramine | | thioridazine | | zimelidine | | ketoconazole | | loratadine | | pentamidine | |
| | kiaa1001 | 3.83 | | 5.79 | | 2.24 | * | 2.24 | ** | 1.42 | | 1.82 | ** |
| | asah1 | 0.82 | | 1.36 | | 2.91 | | 1.47 | | 1.23 | | 0.93 | |
| | mgc4171 | 1.41 | | 1.32 | | 1.12 | | 1.53 | * | 1.57 | | 0.73 | |
| | lss | 1.52 | * | 2.03 | | 1.05 | | 4.33 | ** | 1.54 | | 0.52 | |
| | nr0b2 | 0.98 | | 0.77 | | 1.33 | | 2.36 | ** | 1.63 | | 1.83 | ** |
| | fabp1 | 2.36 | ** | 5.07 | * | 1.42 | | 5.30 | ** | 1.58 | | 0.15 | * |
| | hpn | 2.39 | ** | 2.85 | | 1.74 | ** | 2.71 | ** | 1.52 | * | 2.50 | |
| | serpina3 | 2.03 | ** | 2.46 | ** | 1.95 | | 2.37 | * | 1.83 | * | 2.12 | * |
| | depp | 2.08 | | 2.02 | ** | 2.67 | ** | 2.29 | ** | 0.79 | | 2.88 | ** |
| | flj22362 | 1.46 | | 1.57 | | 1.71 | | 1.95 | | 1.12 | | 2.49 | ** |
| | slc2a3 | 0.63 | | 0.38 | | 0.70 | | 0.48 | * | 0.46 | * | 0.50 | * |
| | tagln | 0.78 | | 0.46 | ** | 0.59 | * | 0.71 | | 0.44 | ** | 0.68 | |

| electro-microscopic test | frequency of appearance of myelin-like structure | | | | | | | | | | | | |
|---|---|---|---|---|---|---|---|---|---|---|---|---|---|
| | - | | | | | | | | | | | | |
| real-time quantitative PCR | expression variation rate | | | | | | | | | | | | |
| gene | acetaminophen | clarithromycin | disopyramide | erythromycin | flecainide | haloperidol | levofloxacin | ofloxacin | procainamide | quinidine | sotalol | sulfamethoxazole | sumatriptan |
| kiaa1001 | 1.13 | 1.48 | 0.94 | 0.78 | 2.10 * | 1.32 | 0.70 | 0.86 | 1.78 | 1.27 | 0.84 | 0.84 | 1.34 |
| asah1 | 1.18 | 1.55 | 1.85 | 0.90 | 2.42 | 0.77 | 1.10 | 1.17 | 0.86 | 1.43 | 1.39 | 1.26 | 1.35 |
| mgc4171 | 0.75 | 1.18 | 1.21 | 0.86 | 1.57 | 1.24 | 0.79 | 0.77 | 1.08 | 1.20 | 0.72 | 0.78 | 0.99 |
| lss | 0.91 | 0.65 | 1.14 | 0.87 | 1.22 | 1.52 | 0.75 | 0.78 | 0.90 | 1.30 | 0.88 | 0.82 | 1.23 * |
| nr0b2 | 0.69 | 0.78 | 0.63 | 0.90 | 1.02 | 1.49 * | 0.79 | 0.81 | 0.74 | 1.03 | 0.63 | 0.82 | 0.63 |
| fabp1 | 1.11 | 0.91 | 1.02 | 0.73 | 1.50 | 1.50 * | 0.88 | 0.75 | 1.00 | 1.38 | 0.98 | 0.85 | 1.01 |
| hpn | 1.10 | 0.96 | 1.12 | 0.87 | 1.26 | 1.43 | 0.83 | 0.72 | 1.08 | 1.21 | 0.79 | 0.89 | 1.16 |
| serpina3 | 0.99 | 1.39 | 0.99 | 0.85 | 1.60 | 1.31 | 0.92 | 0.82 | 0.96 | 1.22 | 0.78 | 0.98 | 0.95 |
| depp | 0.94 | 0.93 | 0.90 | 0.71 | 1.73 | 1.38 | 0.83 * | 0.79 * | 0.98 | 1.06 | 0.80 | 0.70 * | 1.05 |
| flj22362 | 1.02 | 1.00 | 0.98 | 0.92 | 1.65 | 0.93 | 0.83 | 0.68 * | 0.73 * | 1.26 | 0.85 | 0.87 | 1.26 |
| slc2a3 | 1.50 | 0.86 | 1.03 | 0.73 | 0.46 | 0.44 * | 1.07 | 0.94 | 0.88 | 0.37 ** | 1.53 | 1.11 | 0.99 |
| tagln | 0.93 ** | 0.86 | 0.99 | 0.85 | 0.86 | 0.52 * | 0.90 | 0.74 ** | 0.85 | 0.63 * | 0.80 | 0.99 | 1.06 |

[a] see Example 1 (Reference Example)

[b] abbreviation of PLsis marker gene

[c] relative value (average value) of gene expression amount of compound addition group when gene expression amount (average value) of control group is 1.0

*,** significance from control group (t-test) *, $p<0.05$; **, $p<0.01$

An average variation rate of expression of 12 genes (each gene was considered to have the same weight) by each test compound was calculated. The correlation between the average variation rate and the emergence of a myelin-like structure was examined and the results are shown in Fig. 6. Those having a higher average variation rate tended to show a high frequency of emergence of a myelin-like structure, and a good correlation was found between them. When the judgment standard value for the evaluation of the presence or absence of a PLsis induction potential was an average variation rate of 1.5, 17 PLsis-inducing compounds all showed an average variation rate of not less than 1.5. In contrast, 12 compounds out of 13 PLsis non-inducing compounds showed an average variation rate of less than 1.5. Thus, the presence or absence of a PLsis induction potential could be correctly evaluated with a 30 compounds-29 compounds (about 97%) probability of correctness of evaluation.

[Example 3]

Confirmation of reliability of evaluation system

In the same manner as in Example 1 (Reference Example), HepG2 cells were exposed to 26 kinds of compounds unknown as to the presence or absence of a PLsis induction potential for 24 hr each, and in the same manner as in Example 2, expression variation rate of 12 PLsis marker genes was determined and an average variation rate (each gene was considered to have the same weight) was calculated. The same test was performed twice. The average variation rate of the first test is shown in the x coordinate, the average variation rate of the second test is shown in the y coordinate and the results of each test compound are plotted in Fig. 7. Comparison of the two test results revealed good reproducibility (R=0.907). The presence or absence of emergence of a myelin-like structure upon exposure of HepG2 cells to these 26 compounds for 72 hr was separately detected according to the method of Reference Example, and the relationship with an average variation rate was examined. As a result, PLsis negative compounds were distributed in the area of an average variation rate of less than 1.5 in the graph and PLsis positive compounds were distributed in the area of an average variation rate of not less than 1.5, thus showing an extremely good prediction hitting ratio.

**Industrial Applicability**

The PLsis prediction method of the present invention characteristically detects expression variation of a PLsis marker gene upon exposure of mammalian cells to a compound. Therefore, it affords an advantageous effect of possible rapid and convenient examination of many compounds, as compared to conventional *in vivo* toxicity test and evaluation methods using enzyme activity, intracellular accumulation of phospholipid and the like as indices.

In addition, the toxicity prediction method of the present invention affords an advantageous effect of possible more accurate prediction of the presence or absence of toxicity, as compared to conventional evaluation methods using, as an index, an average expression variation rate of gene groups showing common variation of expression in correlation with the toxicity expression.

The prediction method of a PLsis induction potential of a compound according to the present invention shows low probability of false-positive and false-negative, and is an in vitro evaluation system of a PLsis induction potential, which is superior in reliability. In addition, since the method can treat many samples more rapidly and more conveniently than conventional methods, it is particularly useful for the toxicity evaluation of drug candidate compounds in the initial stages of drug discovery.

Sequence Listing Free Text

SEQ ID NO:25 : oligonucleotide designed to function as a primer for amplifying kiaa1001 gene transcription product.
SEQ ID NO:26 : oligonucleotide designed to function as a primer for amplifying kiaa1001 gene transcription product.
SEQ ID NO:27 : oligonucleotide designed to function as TaqMan probe for detecting kiaa1001 gene transcription product.
SEQ ID NO:28 : oligonucleotide designed to function as a primer for amplifying asah1 gene transcription product.
SEQ ID NO:29 : oligonucleotide designed to function as a primer for amplifying asah1 gene transcription product.
SEQ ID NO:30 : oligonucleotide designed to function as TaqMan probe for detecting asah1 gene transcription product.
SEQ ID NO:31 : oligonucleotide designed to function as a primer for amplifying mgc4171 gene transcription product.
SEQ ID NO:32 : oligonucleotide designed to function as a primer for amplifying mgc4171 gene transcription product.
SEQ ID NO:33 : oligonucleotide designed to function as TaqMan probe for detecting mgc4171 gene transcription product.

SEQ ID NO:34 : oligonucleotide designed to function as a primer for amplifying lss gene transcription product.
SEQ ID NO:35 : oligonucleotide designed to function as a primer for amplifying lss gene transcription product.
SEQ ID NO:36 : oligonucleotide designed to function as TaqMan probe for detecting lss gene transcription product.
SEQ ID NO:37 : oligonucleotide designed to function as a primer for amplifying nr0b2 gene transcription product.
SEQ ID NO:38 : oligonucleotide designed to function as a primer for amplifying nr0b2 gene transcription product.
SEQ ID NO:39 : oligonucleotide designed to function as TaqMan probe for detecting nr0b2 gene transcription product.
SEQ ID NO:40 : oligonucleotide designed to function as a primer for amplifying fabp1 gene transcription product.
SEQ ID NO:41 : oligonucleotide designed to function as a primer for amplifying fabp1 gene transcription product.
SEQ ID NO:42 : oligonucleotide designed to function as TaqMan probe for detecting fabp1 gene transcription product.
SEQ ID NO:43 : oligonucleotide designed to function as a primer for amplifying hpn gene transcription product.
SEQ ID NO:44 : oligonucleotide designed to function as a primer for amplifying hpn gene transcription product.
SEQ ID NO:45 : oligonucleotide designed to function as TaqMan probe for detecting hpn gene transcription product.
SEQ ID NO:46 : oligonucleotide designed to function as a primer for amplifying serpina3 gene transcription product.
SEQ ID NO:47 : oligonucleotide designed to function as a primer for amplifying serpina3 gene transcription product.
SEQ ID NO:48 : oligonucleotide designed to function as TaqMan probe for detecting serpina3 gene transcription product.
SEQ ID NO:49 : oligonucleotide designed to function as a primer for amplifying depp gene transcription product.
SEQ ID NO:50 : oligonucleotide designed to function as a primer for amplifying depp gene transcription product.
SEQ ID NO:51 : oligonucleotide designed to function as TaqMan probe for detecting depp gene transcription product.
SEQ ID NO:52 : oligonucleotide designed to function as a primer for amplifying flj22362 gene transcription product.
SEQ ID NO:53 : oligonucleotide designed to function as a primer for amplifying flj22362 gene transcription product.
SEQ ID NO:54 : oligonucleotide designed to function as TaqMan probe for detecting flj22362 gene transcription product.
SEQ ID NO:55 : oligonucleotide designed to function as a primer for amplifying slc2a3 gene transcription product.
SEQ ID NO:56 : oligonucleotide designed to function as a primer for amplifying slc2a3 gene transcription product.
SEQ ID NO:57 : oligonucleotide designed to function as TaqMan probe for detecting slc2a3 gene transcription product.
SEQ ID NO:58 : oligonucleotide designed to function as a primer for amplifying tagln gene transcription product.
SEQ ID NO:59 : oligonucleotide designed to function as a primer for amplifying tagln gene transcription product.
SEQ ID NO:60 : oligonucleotide designed to function as TaqMan probe for detecting tagln gene transcription product.

This application is based on a patent application No. 2003-397551 filed in Japan (filing date: November 27, 2003).

## Claims

**1.** A kit for predicting a phospholipidopsis induction potential of a compound, which consists essentially of a reagent containing a nucleic acid capable of hybridizing to a nucleic acid having a base sequence shown by SEQ ID NO:1 under high stringent conditions and/or a nucleic acid having a base sequence complementary to the base sequence under high stringent conditions and:

(i) a reagent comprising a nucleic acid capable of hybridizing to a nucleic acid having a base sequence shown by SEQ ID NO:3 under high stringent conditions and/or a nucleic acid having a base sequence complementary to the base sequence under high stringent conditions;
(ii) a reagent comprising a nucleic acid capable of hybridizing to a nucleic acid having a base sequence shown by SEQ ID NO:5 under high stringent conditions and/or a nucleic acid having a base sequence complementary to the base sequence under high stringent conditions;
(iii) a reagent comprising a nucleic acid capable of hybridizing to a nucleic acid having a base sequence shown by SEQ ID NO:7 under high stringent conditions and/or a nucleic acid having a base sequence complementary to the base sequence under high stringent conditions;
(iv) a reagent comprising a nucleic acid capable of hybridizing to a nucleic acid having a base sequence shown by SEQ ID NO:9 under high stringent conditions and/or a nucleic acid having a base sequence complementary to the base sequence under high stringent conditions;
(v) a reagent comprising a nucleic acid capable of hybridizing to a nucleic acid having a base sequence shown by SEQ ID NO:11 under high stringent conditions and/or a nucleic acid having a base sequence complementary to the base sequence under high stringent conditions;

(vi) a reagent comprising a nucleic acid capable of hybridizing to a nucleic acid having a base sequence shown by SEQ ID NO:13 under high stringent conditions and/or a nucleic acid having a base sequence complementary to the base sequence under high stringent conditions;
(vii) a reagent comprising a nucleic acid capable of hybridizing to a nucleic acid having a base sequence shown by SEQ ID NO:15 under high stringent conditions and/or a nucleic acid having a base sequence complementary to the base sequence under high stringent conditions;
(viii) a reagent comprising a nucleic acid capable of hybridizing to a nucleic acid having a base sequence shown by SEQ ID NO:17 under high stringent conditions and/or a nucleic acid having a base sequence complementary to the base sequence under high stringent conditions;
(ix) a reagent comprising a nucleic acid capable of hybridizing to a nucleic acid having a base sequence shown by SEQ ID NO:19 under high stringent conditions and/or a nucleic acid having a base sequence complementary to the base sequence under high stringent conditions;
(x) a reagent comprising a nucleic acid capable of hybridizing to a nucleic acid having a base sequence shown by SEQ ID NO:21 under high stringent conditions and/or a nucleic acid having a base sequence complementary to the base sequence under high stringent conditions; and
(xi) a reagent comprising a nucleic acid capable of hybridizing to a nucleic acid having a base sequence shown by SEQ ID NO:23 under high stringent conditions and/or a nucleic acid having a base sequence complementary to the base sequence under high stringent conditions.

**2.** A method for predicting a phospholipidosis induction potential of a test compound in a mammal, which comprises

(1) detecting expression variation of the 12 human genes comprising the base sequences shown by SEQ ID NOs:1, 3, 5, 7, 9, 11, 13, 15, 17, 19, 21 and 23 or homologues thereof in the non-human mammal in a sample containing a cell derived from the mammal exposed to the test compound or a sample taken from the mammal administered with the compound,
(2) taking a magnification of the expression amount of each gene as an expression variation rate (X) of the gene when the expression amount increased upon exposure and taking an inverse number of magnification of each gene as an expression variation rate (X) of the gene when the expression amount decreased upon exposure,
(3) determining an average value of the expression variation rates of the abovementioned 12 genes according to the following formula as an average variation rate:

$$[\text{average variation rate}] = m_1X_1+m_2X_2+\ \dots\ +m_{12}X_{12}$$

wherein $m_i$ (i=1-12) shows the weight of each gene, which is the same weight for all genes:

$$m_i=1/12$$

and

$$m_1+m_2+\ \dots\ +m_{12}=1,$$

and
(4) predicting that the test compound has a phospholipidosis induction potential when the average variation rate is not less than 1.5, and that the test compound has no phospholipidosis indudion potential when the average variation rate is less than 1.5.

**Patentansprüche**

**1.** Kit zum Vorhersagen eines Phospholipidose-Induktionspotentials einer Verbindung, wobei das Kit im Wesentlichen aus Folgendem besteht: einem Reagens, das eine Nukleinsäure enthält, die mit einer Nukleinsäure einer Basensequenz von SEQ ID Nr: 1 unter hoch-stringenten Bedingungen und/oder mit einer Nukleinsäure einer zur Basensequenz komplementären Basensequenz unter hoch-stringenten Bedingungen hybridisieren kann, und:

(i) einem Reagens, das eine Nukleinsäure umfasst, die mit einer Nukleinsäure einer Basensequenz von SEQ ID Nr: 3 unter hoch-stringenten Bedingungen und/oder mit einer Nukleinsäure einer zur Basensequenz komplementären Basensequenz unter hoch-stringenten Bedingungen hybridisieren kann;
(ii) einem Reagens, das eine Nukleinsäure umfasst, die mit einer Nukleinsäure einer Basensequenz von SEQ ID Nr: 5 unter hoch-stringenten Bedingungen und/oder mit einer Nukleinsäure einer zur Basensequenz komplementären Basensequenz unter hoch-stringenten Bedingungen hybridisieren kann;
(iii) einem Reagens, das eine Nukleinsäure umfasst, die mit einer Nukleinsäure einer Basensequenz von SEQ ID Nr: 7 unter hoch-stringenten Bedingungen und/oder mit einer Nukleinsäure einer zur Basensequenz komplementären Basensequenz unter hoch-stringenten Bedingungen hybridisieren kann;
(iv) einem Reagens, das eine Nukleinsäure umfasst, die mit einer Nukleinsäure einer Basensequenz von SEQ ID Nr: 9 unter hoch-stringenten Bedingungen und/oder mit einer Nukleinsäure einer zur Basensequenz komplementären Basensequenz unter hoch-stringenten Bedingungen hybridisieren kann;
(v) einem Reagens, das eine Nukleinsäure umfasst, die mit einer Nukleinsäure einer Basensequenz von SEQ ID Nr: 11 unter hoch-stringenten Bedingungen und/oder mit einer Nukleinsäure einer zur Basensequenz komplementären Basensequenz unter hoch-stringenten Bedingungen hybridisieren kann;
(vi) einem Reagens, das eine Nukleinsäure umfasst, die mit einer Nukleinsäure einer Basensequenz von SEQ ID Nr: 13 unter hoch-stringenten Bedingungen und/oder mit einer Nukleinsäure einer zur Basensequenz komplementären Basensequenz unter hoch-stringenten Bedingungen hybridisieren kann;
(vii) einem Reagens, das eine Nukleinsäure umfasst, die mit einer Nukleinsäure einer Basensequenz von SEQ ID Nr: 15 unter hoch-stringenten Bedingungen und/oder mit einer Nukleinsäure einer zur Basensequenz komplementären Basensequenz unter hoch-stringenten Bedingungen hybridisieren kann;
(viii) einem Reagens, das eine Nukleinsäure umfasst, die mit einer Nukleinsäure einer Basensequenz von SEQ ID Nr: 17 unter hoch-stringenten Bedingungen und/oder mit einer Nukleinsäure einer zur Basensequenz komplementären Basensequenz unter hoch-stringenten Bedingungen hybridisieren kann;
(ix) einem Reagens, das eine Nukleinsäure umfasst, die mit einer Nukleinsäure einer Basensequenz von SEQ ID Nr: 19 unter hoch-stringenten Bedingungen und/oder mit einer Nukleinsäure einer zur Basensequenz komplementären Basensequenz unter hoch-stringenten Bedingungen hybridisieren kann;
(x) einem Reagens, das eine Nukleinsäure umfasst, die mit einer Nukleinsäure einer Basensequenz von SEQ ID Nr: 21 unter hoch-stringenten Bedingungen und/oder mit einer Nukleinsäure einer zur Basensequenz komplementären Basensequenz unter hoch-stringenten Bedingungen hybridisieren kann;
(xi) einem Reagens, das eine Nukleinsäure umfasst, die mit einer Nukleinsäure einer Basensequenz von SEQ ID Nr: 23 unter hoch-stringenten Bedingungen und/oder mit einer Nukleinsäure einer zur Basensequenz komplementären Basensequenz unter hoch-stringenten Bedingungen hybridisieren kann.

**2.** Verfahren zum Vorhersagen eines Phospholipidose-Induktionspotentials einer Testverbindung in einem Säuger, umfassend:

(1) Detektieren einer Expressionsvariation der 12 menschlichen Gene, die die Basensequenzen von SEQ ID Nr: 1, 3, 5, 7, 9, 11, 13, 15, 17, 19, 21 und 23 umfassen, oder von Homologen davon im nicht-menschlichen Säuger in einer Probe, die eine Zelle enthält, die von dem Säuger abgeleitet ist und der Testverbindung ausgesetzt wird, oder in einer Probe, die dem Säuger, dem die Verbindung verabreicht worden ist, entnommen worden ist,
(2) Festlegen eines Multiplikationsfaktors der Expressionsmenge jedes Gens als Expressionsvariationsrate (X) des Gens, wenn die Expressionsmenge bei dem Aussetzen anstieg, und Festlegen des inversen Multiplikationsfaktors jedes Gens als Expressionsvariationsrate (X) des Gens, wenn die Expressionsmenge bei dem Aussetzen abnahm,
(3) Bestimmen eines Durchschnittswerts der Expressionsvariationsraten der oben erwähnten 12 Gene als durchschnittliche Variationsrate gemäß der folgenden Formel:

$$(\text{durchschnittliche Variationsrate}) = m_1X_1 + m_2X_2 + \dots + m_{12}X_{12},$$

wobei $m_i$ (i = 1 - 12) die Gewichtung jedes Gens zeigt, die für alle Gene dieselbe Gewichtung ist:

$$m_i = 1/12$$

und

$$m_1 + m_2 + \ldots + m_{12} = 1,$$

und

(4) Vorhersagen, dass die Testverbindung ein Phospholipidose-Induktionspotential hat, wenn die durchschnittliche Variationsrate nicht kleiner als 1,5 ist, und dass die Testverbindung kein Phospholipidose-Induktionspotential hat, wenn die durchschnittliche Variationsrate kleiner als 1,5 ist.

**Revendications**

**1.** Trousse servant à prédire la capacité d'un composé à induire une phospholipidose, qui comporte essentiellement un réactif contenant un acide nucléique capable de s'hybrider à un acide nucléique ayant la séquence de bases donnée en tant que Séquence N° 1, dans des conditions hautement stringentes, et/ou à un acide nucléique ayant une séquence de bases complémentaire de cette séquence de bases, dans des conditions hautement stringentes, et :

i) un réactif comprenant un acide nucléique capable de s'hybrider à un acide nucléique ayant la séquence de bases donnée en tant que Séquence N° 3, dans des conditions hautement stringentes, et/ou à un acide nucléique ayant une séquence de bases complémentaire de cette séquence de bases, dans des conditions hautement stringentes ;
ii) un réactif comprenant un acide nucléique capable de s'hybrider à un acide nucléique ayant la séquence de bases donnée en tant que Séquence N° 5, dans des conditions hautement stringentes, et/ou à un acide nucléique ayant une séquence de bases complémentaire de cette séquence de bases, dans des conditions hautement stringentes ;
iii) un réactif comprenant un acide nucléique capable de s'hybrider à un acide nucléique ayant la séquence de bases donnée en tant que Séquence N° 7, dans des conditions hautement stringentes, et/ou à un acide nucléique ayant une séquence de bases complémentaire de cette séquence de bases, dans des conditions hautement stringentes ;
iv) un réactif comprenant un acide nucléique capable de s'hybrider à un acide nucléique ayant la séquence de bases donnée en tant que Séquence N° 9, dans des conditions hautement stringentes, et/ou à un acide nucléique ayant une séquence de bases complémentaire de cette séquence de bases, dans des conditions hautement stringentes ;
v) un réactif comprenant un acide nucléique capable de s'hybrider à un acide nucléique ayant la séquence de bases donnée en tant que Séquence N° 11, dans des conditions hautement stringentes, et/ou à un acide nucléique ayant une séquence de bases complémentaire de cette séquence de bases, dans des conditions hautement stringentes ;
vi) un réactif comprenant un acide nucléique capable de s'hybrider à un acide nucléique ayant la séquence de bases donnée en tant que Séquence N° 13, dans des conditions hautement stringentes, et/ou à un acide nucléique ayant une séquence de bases complémentaire de cette séquence de bases, dans des conditions hautement stringentes ;
vii) un réactif comprenant un acide nucléique capable de s'hybrider à un acide nucléique ayant la séquence de bases donnée en tant que Séquence N° 15, dans des conditions hautement stringentes, et/ou à un acide nucléique ayant une séquence de bases complémentaire de cette séquence de bases, dans des conditions hautement stringentes ;
viii) un réactif comprenant un acide nucléique capable de s'hybrider à un acide nucléique ayant la séquence de bases donnée en tant que Séquence N° 17, dans des conditions hautement stringentes, et/ou à un acide nucléique ayant une séquence de bases complémentaire de cette séquence de bases, dans des conditions hautement stringentes ;
ix) un réactif comprenant un acide nucléique capable de s'hybrider à un acide nucléique ayant la séquence de bases donnée en tant que Séquence N° 19, dans des conditions hautement stringentes, et/ou à un acide nucléique ayant une séquence de bases complémentaire de cette séquence de bases, dans des conditions hautement stringentes ;
x) un réactif comprenant un acide nucléique capable de s'hybrider à un acide nucléique ayant la séquence de bases donnée en tant que Séquence N° 21, dans des conditions hautement stringentes, et/ou à un acide nucléique ayant une séquence de bases complémentaire de cette séquence de bases, dans des conditions hautement stringentes ;
xi) et un réactif comprenant un acide nucléique capable de s'hybrider à un acide nucléique ayant la séquence de bases donnée en tant que Séquence N° 23, dans des conditions hautement stringentes, et/ou à un acide nucléique ayant une séquence de bases complémentaire de cette séquence de bases, dans des conditions hautement stringentes.

**2.** Procédé permettant de prédire la capacité d'un composé testé à induire une phospholipidose chez un mammifère, lequel procédé comporte les étapes suivantes :

1) détecter les variations de l'expression des douze gènes humains comprenant les séquences de bases données en tant que Séquences N° 1, 3, 5, 7, 9, 11, 13, 15, 17, 19, 21 et 23, ou de leurs homologues chez un mammifère non-humain, dans un échantillon contenant une cellule dérivée du mammifère et exposée au composé testé ou dans un échantillon prélevé sur le mammifère après administration à celui-ci du composé testé ;
2) prendre comme facteur de variation de l'expression d'un gène le facteur multiplicatif (X) de la quantité d'expression du gène quand cette quantité d'expression augmente après exposition, et prendre comme facteur de variation de l'expression d'un gène l'inverse du facteur multiplicatif (X) de la quantité d'expression du gène quand cette quantité d'expression diminue après exposition ;
3) déterminer une valeur moyenne des facteurs de variations de l'expression des douze gènes mentionnés ci-dessus, en tant que facteur moyen de variation, d'après la formule suivante :

$$\text{facteur moyen de variation} = m_1X_1 + m_2X_2 + \ldots \ldots + m_{12}X_{12}$$

où les $m_i$ (pour i = 1 à 12) représentent le poids de chaque gène, qui a la même valeur pour tous les gènes, soit :

$$m_i = 1/12 \qquad \text{et} \qquad m_1 + m_2 + \ldots \ldots + m_{12} = 1$$

4) et prédire, si ce facteur moyen de variation vaut au moins 1,5, que le composé testé possède une certaine capacité à induire une phospholipidose, et si ce facteur moyen de variation vaut moins de 1,5, que le composé testé ne possède pas de capacité à induire une phospholipidose.

# FIG. 1

| compound | structural formula | molecular weight | efficacy | addition concentration (μ mol/L) |
|---|---|---|---|---|
| amiodarone | 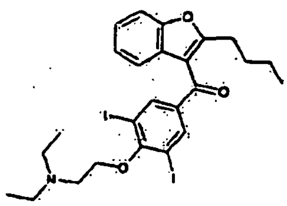 | 681.8 | antiarrhythmic agent | 8.3 |
| amitriptyline | 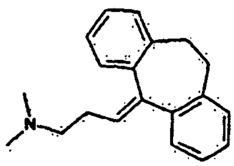 | 313.9 | antidepressant | 25 |
| AY-9944 | 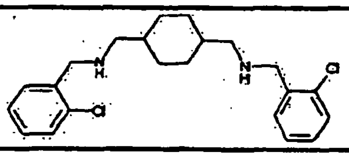 | 464.3 | antilipidemic agent | 8.3 |
| chlorcyclizine | 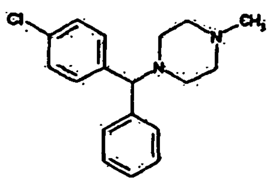 | 337.3 | antihistamine | 25 |
| chlorpromazine | 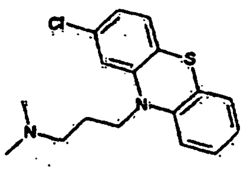 | 355.3 | anxiolytic | 8.3 |
| clomipramine | 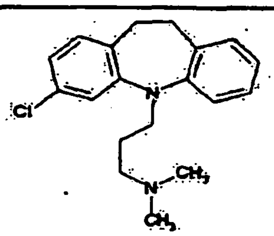 | 351.3 | antidepressant | 8.3 |

# FIG. 2

| compound | structural formula | molecular weight | efficacy | addition concentration (μ mol/L) |
|---|---|---|---|---|
| fluoxetine | 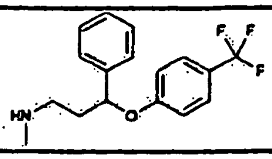 | 345.8 | antidepressant | 8.3 |
| imipramine | 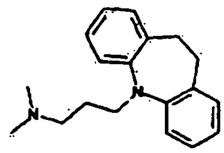 | 316.9 | antidepressant | 25 |
| perhexiline | 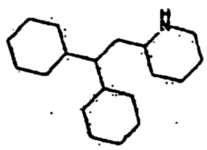 | 393.6 | antianginal agent | 8.3 |
| tamoxifen | 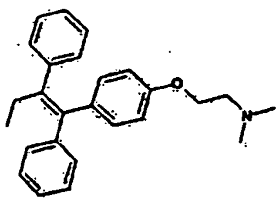 | 563.7 | antiestrogen | 8.3 |
| thioridazine | 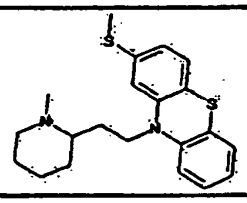 | 407 | anxiolytic | 8.3 |
| zimelidine | 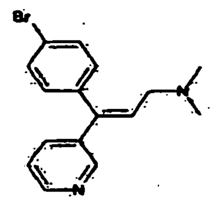 | 390.2 | antidepressant | 25 |

# FIG. 3

| compound | structural formula | molecular weight | efficacy | addition concentration (μ mol/L) |
|---|---|---|---|---|
| clozapine | 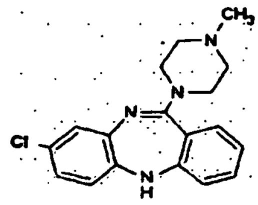 | 326.8 | antipsychotic | 25 |
| ketoconazole | 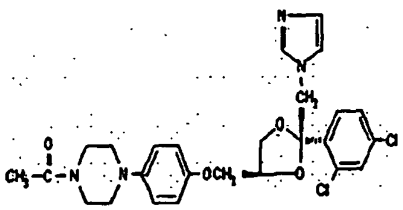 | 531.4 | antifungal | 8.3 |
| loratadine | 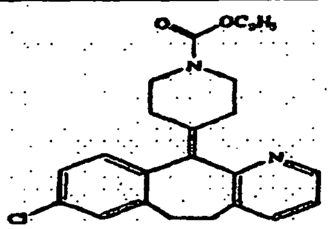 | 382.89 | antihistamine | 8.3 |
| pentamidine | 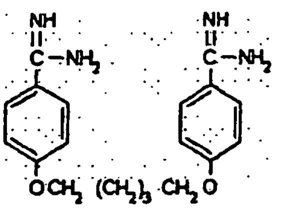 | 340.4 | anti-infective agent | 8.3 |
| sertraline | 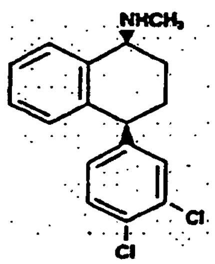 | 342.7 | antidepressant | 8.3 |

# FIG. 4

| compound | structural formula | molecular weight | efficacy | addition concentration (μ mol/L) |
|---|---|---|---|---|
| acetaminophen | 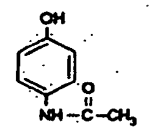 | 515.9 | analgesic antipyretic | 25 |
| clarithromycin | 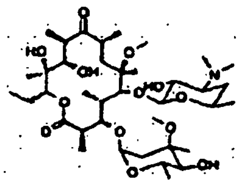 | 748 | antibiotic | 25 |
| disopyramide | 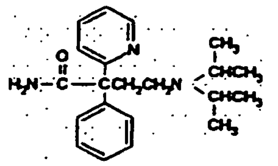 | 339.48 | antiarrhythmic agent | 25 |
| erythromycin | 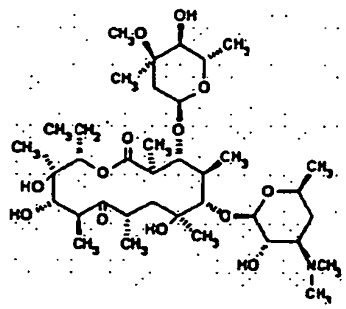 | 733.94 | antibiotic | 25 |
| flecainide | 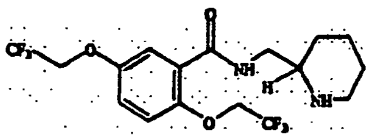 | 474.4 | antiarrhythmic agent | 25 |
| haloperidol | 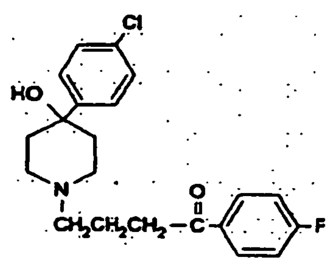 | 375.86 | antipsychotic | 8.3 |

# FIG. 5

| compound | structural formula | molecular weight | efficacy | addition concentration (μ mol/L) |
|---|---|---|---|---|
| levofloxacin | 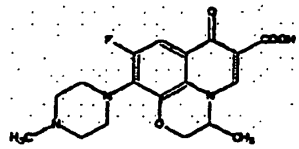 | 370.4 | antibiotic | 25 |
| ofloxacin | 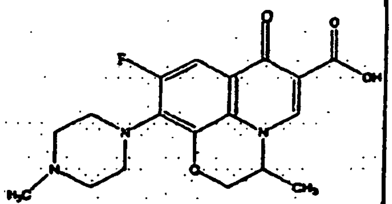 | 361.4 | antibiotic | 25 |
| procainamide | 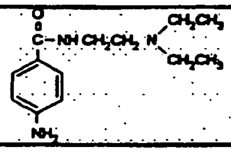 | 271.8 | antiarrhythmic agent | 25 |
| quinidine | 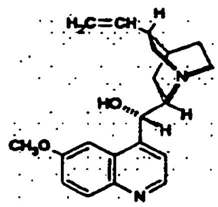 | 324.42 | antiarrhythmic agent | 25 |
| sotalol | 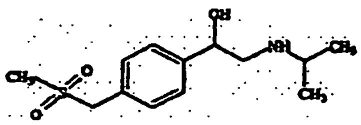 | 308.8 | antiarrhythmic agent | 25 |
| sulfamethoxazole | 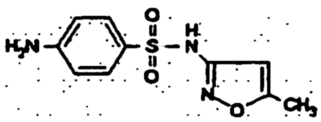 | 253.3 | anti-infective agent | 25 |
| sumatryptan | 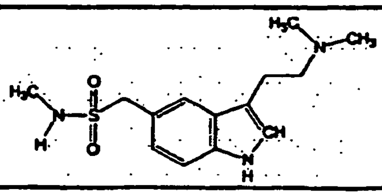 | 413.5 | antimigraine agent | 25 |

FIG. 6

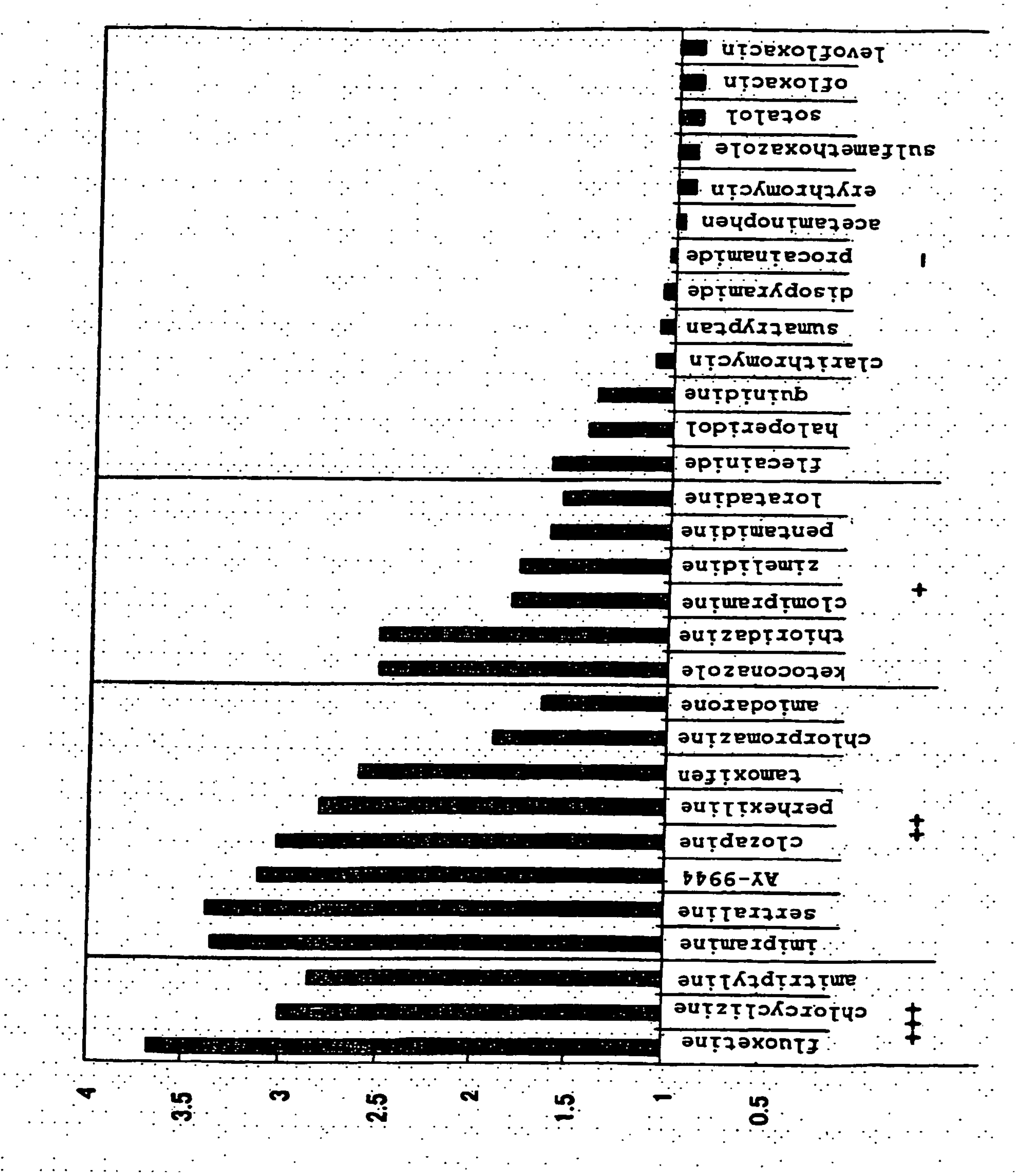
fluoxetine
chlorcyclizine
amitriptyline
imipramine
sertraline
AY-9944
clozapine
perhexiline
tamoxifen
chlorpromazine
amiodarone
ketoconazole
thioridazine
clomipramine
zimelidine
pentamidine
loratadine
flecainide
haloperidol
quinidine
clarithromycin
sumatryptan
disopyramide
procainamide
acetaminophen
erythromycin
sulfamethoxazole
sotalol
ofloxacin
levofloxacin
+++
++
+
-
4
3.5
3
2.5
2
1.5
1
0.5

Fig. 7 (modified)

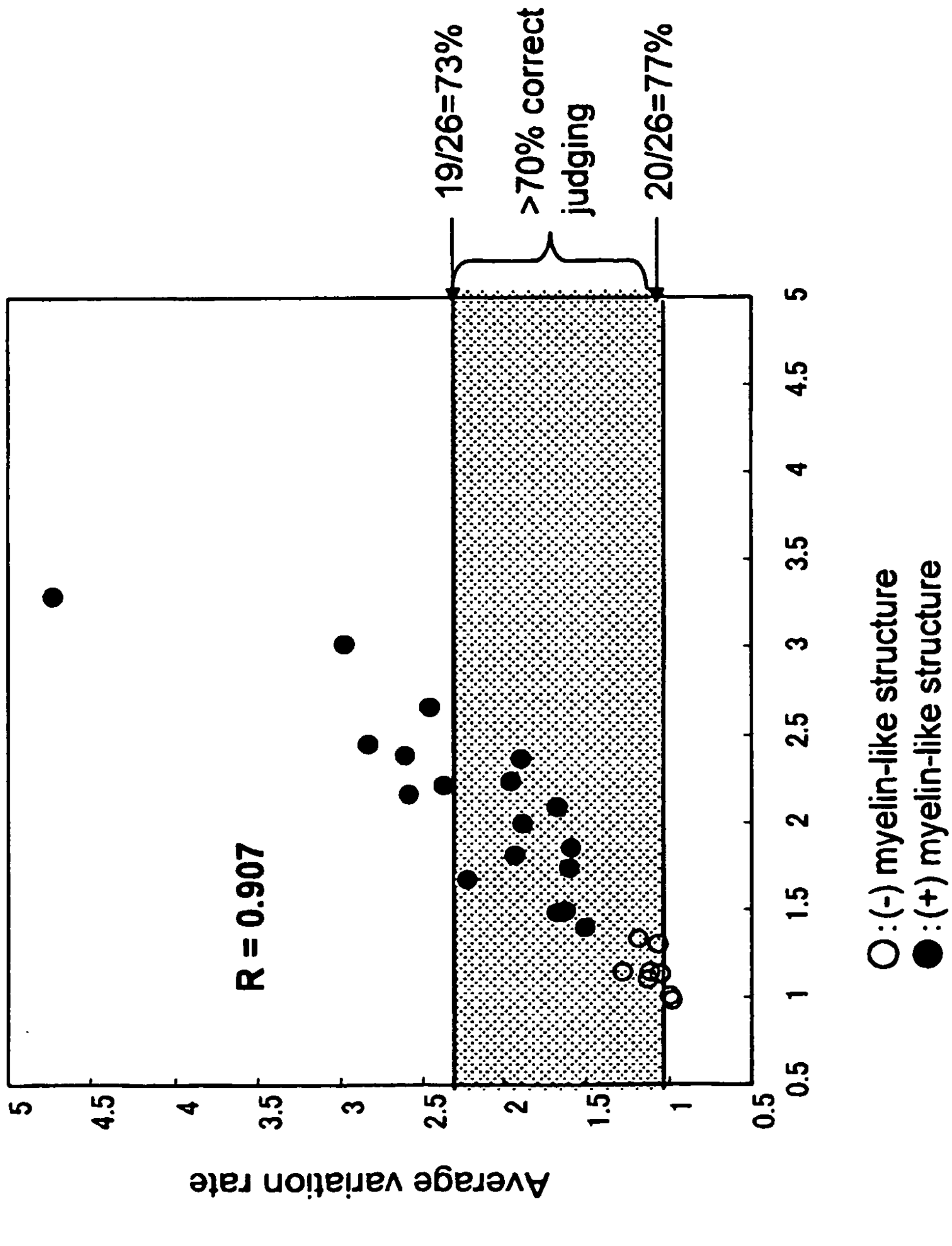

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0210453 A
- WO 02095000 A
- JP 2003397551 A


### Non-patent literature cited in the description


- **MATSUZAWA, Y. ; HOSTETLER, K.Y.** *J. Biol. Chem.,* 1980, vol. 255 (2), 646-652
- **GUM, R.J. et al.** *Biochem. Pharmacol.,* 2001, vol. 62, 1661-1673
- **XIA, Z et al.** *Biochem. Pharmacol.,* 1997, vol. 53, 1521-1532
- **AARDEMA, M.J. ; MACGREGOR, J.T. et al.** *Mutat. Res.,* 2002, vol. 499, 13-25
- **BOWIE et al.** *Science,* 1990, vol. 247, 1306-1310
- **KARLIN et al.** *Proc. Natl. Acad. Sci. USA,* 1993, vol. 90, 5873-5877
- **ALTSCHUL et al.** *Nucleic Acids Res.,* 1997, vol. 25, 3389-3402
- **NEEDLEMAN et al.** *J. Mol. Biol.,* 1970, vol. 48, 444-453
- **MYERS ; MILLER.** *CABIOS,* 1988, vol. 4, 11-17
- **PEARSON et al.** *Proc. Natl. Acad. Sci. USA,* 1988, vol. 85, 2444-2448
- Molecular Cloning
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1

SEQUENCE LISTING

<110> Takeda Pharmaceutical Company Limited

<120> Method for Predicting Drug Toxicity

<130> 09707

<150> JP 2003-397551
<151> 2003-11-27

<160> 60

<170> PatentIn version 3.2

<210> 1
<211> 4304
<212> DNA
<213> Homo sapiens

<220>
<221> CDS
<222> (459) .. (2033)

<400> 1

gcgagaactc atcctgtagt caccagatgg agtcccaaac agccaagcag atgtaaggcc 60

tgtgctgtgg ctctgaggcc ctgaatacag aagggtcact ttcttagtgg ccaaagagca 120

gttgttgaca ttgatgtcta attattgaac acgaccagtc attttactga gctgcggtga 180

ggaaacactg accatagaag atcaagccaa atgagggatt gcaaatttcc tgattctttt 240

gaattaggat tccagatggg ggcctcattt ctacagcccc caacattcct atagccgtta 300

tcactgccat caccactgcc accagcatct tcttgcagat tccacccctg ctccccagag 360

acttcctgct ttgaaagtga gcagaaagga agctctcaga aaaatctcta gtggtggctg 420

ccgtcgctcc agacaatcgg aatcctgcct tcaccacc atg ggc tgg ctt ttt cta 476

Met Gly Trp Leu Phe Leu
1 5

aag gtt ttg ttg gcg gga gtg agt ttc tca gga ttt ctt tat cct ctt 524
Lys Val Leu Leu Ala Gly Val Ser Phe Ser Gly Phe Leu Tyr Pro Leu
10 15 20

gtg gat ttt tgc atc agt ggg aaa aca aga gga cag aag cca aac ttt 572
Val Asp Phe Cys Ile Ser Gly Lys Thr Arg Gly Gln Lys Pro Asn Phe
25 30 35

gtg att att ttg gcc gat gac atg ggg tgg ggt gac ctg gga gca aac 620
Val Ile Ile Leu Ala Asp Asp Met Gly Trp Gly Asp Leu Gly Ala Asn
40 45 50

tgg gca gaa aca aag gac act gcc aac ctt gat aag atg gct tcg gag 668
Trp Ala Glu Thr Lys Asp Thr Ala Asn Leu Asp Lys Met Ala Ser Glu
55 60 65 70

gga atg agg ttt gtg gat ttc cat gca gct gcc tcc acc tgc tca ccc 716
Gly Met Arg Phe Val Asp Phe His Ala Ala Ala Ser Thr Cys Ser Pro

75 80 85

tcc cgg gct tcc ttg ctc acc ggc cgg ctt ggc ctt cgc aat gga gtc 764

Ser Arg Ala Ser Leu Leu Thr Gly Arg Leu Gly Leu Arg Asn Gly Val

90 95 100

aca cgc aac ttt gca gtc act tct gtg gga ggc ctt ccg ctc aac gag 812

Thr Arg Asn Phe Ala Val Thr Ser Val Gly Gly Leu Pro Leu Asn Glu

105 110 115

acc acc ttg gca gag gtg ctg cag cag gcg ggt tac gtc act ggg ata 860

Thr Thr Leu Ala Glu Val Leu Gln Gln Ala Gly Tyr Val Thr Gly Ile

120 125 130

ata ggc aaa tgg cat ctt gga cac cac ggc tct tat cac ccc aac ttc 908

Ile Gly Lys Trp His Leu Gly His His Gly Ser Tyr His Pro Asn Phe

135 140 145 150

cgt ggt ttt gat tac tac ttt gga atc cca tat agc cat gat atg ggc 956

Arg Gly Phe Asp Tyr Tyr Phe Gly Ile Pro Tyr Ser His Asp Met Gly

155 160 165

tgt act gat act cca ggc tac aac cac cct cct tgt cca gcg tgt cca 1004
Cys Thr Asp Thr Pro Gly Tyr Asn His Pro Pro Cys Pro Ala Cys Pro
170 175 180

cag ggt gat gga cca tca agg aac ctt caa aga gac tgt tac act gac 1052
Gln Gly Asp Gly Pro Ser Arg Asn Leu Gln Arg Asp Cys Tyr Thr Asp
185 190 195

gtg gcc ctc cct ctt tat gaa aac ctc aac att gtg gag cag ccg gtg 1100
Val Ala Leu Pro Leu Tyr Glu Asn Leu Asn Ile Val Glu Gln Pro Val
200 205 210

aac ttg agc agc ctt gcc cag aag tat gct gag aaa gca acc cag ttc 1148
Asn Leu Ser Ser Leu Ala Gln Lys Tyr Ala Glu Lys Ala Thr Gln Phe
215 220 225 230

atc cag cgt gca agc acc agc ggg agg ccc ttc ctg ctc tat gtg gct 1196
Ile Gln Arg Ala Ser Thr Ser Gly Arg Pro Phe Leu Leu Tyr Val Ala
235 240 245

ctg gcc cac atg cac gtg ccc tta cct gtg act caa cta cca gca gcg 1244
Leu Ala His Met His Val Pro Leu Pro Val Thr Gln Leu Pro Ala Ala
250 255 260

cca cgg ggc aga agc ctg tat ggt gca ggg ctc tgg gag atg gac agt 1292
Pro Arg Gly Arg Ser Leu Tyr Gly Ala Gly Leu Trp Glu Met Asp Ser
265 270 275

ctg gtg ggc cag atc aag gac aaa gtt gac cac aca gtg aag gaa aac 1340
Leu Val Gly Gln Ile Lys Asp Lys Val Asp His Thr Val Lys Glu Asn
280 285 290

aca ttc ctc tgg ttt aca gga gac aat ggc ccg tgg gct cag aag tgt 1388
Thr Phe Leu Trp Phe Thr Gly Asp Asn Gly Pro Trp Ala Gln Lys Cys
295 300 305 310

gag cta gcg ggc agt gtg ggt ccc ttc act gga ttt tgg caa act cgt 1436
Glu Leu Ala Gly Ser Val Gly Pro Phe Thr Gly Phe Trp Gln Thr Arg
315 320 325

caa ggg gga agt cca gcc aag cag acg acc tgg gaa gga ggg cac cgg 1484

Gln Gly Gly Ser Pro Ala Lys Gln Thr Thr Trp Glu Gly Gly His Arg
330 335 340

gtc cca gca ctg gct tac tgg cct ggc aga gtt cca gtt aat gtc acc 1532
Val Pro Ala Leu Ala Tyr Trp Pro Gly Arg Val Pro Val Asn Val Thr
345 350 355

agc act gcc ttg tta agc gtg ctg gac att ttt cca act gtg gta gcc 1580
Ser Thr Ala Leu Leu Ser Val Leu Asp Ile Phe Pro Thr Val Val Ala
360 365 370

ctg gcc cag gcc agc tta cct caa gga cgg cgc ttt gat ggt gtg gac 1628
Leu Ala Gln Ala Ser Leu Pro Gln Gly Arg Arg Phe Asp Gly Val Asp
375 380 385 390

gtc tcc gag gtg ctc ttt ggc cgg tca cag cct ggg cac agg gtg ctg 1676
Val Ser Glu Val Leu Phe Gly Arg Ser Gln Pro Gly His Arg Val Leu
395 400 405

ttc cac ccc aac agc ggg gca gct gga gag ttt gga gcc ctg cag act 1724
Phe His Pro Asn Ser Gly Ala Ala Gly Glu Phe Gly Ala Leu Gln Thr

410 415 420

gtc cgc ctg gag cgt tac aag gcc ttc tac att acc ggt gga gcc agg 1772
Val Arg Leu Glu Arg Tyr Lys Ala Phe Tyr Ile Thr Gly Gly Ala Arg
425 430 435

gcg tgt gat ggg agc acg ggg cct gag ctg cag cat aag ttt cct ctg 1820
Ala Cys Asp Gly Ser Thr Gly Pro Glu Leu Gln His Lys Phe Pro Leu
440 445 450

att ttc aac ctg gaa gac gat acc gca gaa gct gtg ccc cta gaa aga 1868
Ile Phe Asn Leu Glu Asp Asp Thr Ala Glu Ala Val Pro Leu Glu Arg
455 460 465 470

ggt ggt gcg gag tac cag gct gtg ctg ccc gag gtc aga aag gtt ctt 1916
Gly Gly Ala Glu Tyr Gln Ala Val Leu Pro Glu Val Arg Lys Val Leu
475 480 485

gca gac gtc ctc caa gac att gcc aac gac aac atc tcc agc cca gat 1964
Ala Asp Val Leu Gln Asp Ile Ala Asn Asp Asn Ile Ser Ser Pro Asp
490 495 500

tac act cag gac cct tca gta act ccc tgc tgt aat ccc tac caa att 2012

Tyr Thr Gln Asp Pro Ser Val Thr Pro Cys Cys Asn Pro Tyr Gln Ile

505 510 515

gcc tgc cgc tgt caa gcc gca taacagacca atttttattc cacgaggagg 2063

Ala Cys Arg Cys Gln Ala Ala

520 525

agtacctgga aattaggcaa gtttgcttcc aaatttcatt tttaccctct ttacaaacac 2123

acgctttagt ttagtcttgg agtttagttt tggagttagc cttgcatatc cttctgtat 2183

cctgtccctc ctccacgccg acccgagagc agctgagctg cgctggctct gggcagggag 2243

tgtgccttaa tgggaagcac acgggctttg gagtcaggca caggtgccag ctccagcttt 2303

tgaacttggg caattgttta acctaacctg caagttgatt ttgagggtta aataaaggca 2363

tacatgaaaa tgcctggcaa attacctgac acagagcaga cattcaatac attttagttt 2423

ccttgtttct ctggttccca gtttctctgg tcattttggt gtaaatccat tctaattagt 2483

atttagggca gagcttctct ctcttttctc tttttttcct tccacaaacc agtgtactca 2543

ctggtctcca tctttaatat gcaaacaaat cacctgggat cttgtgagaa tccggattct 2603

gtctcagtag ggctcgagta gatcctgaaa tcctacattt ctatcaaaca atgccttgag 2663

gagcacagat ttagaccaaa gttaggtcgt tttccagatc tcagagcaga cgagtccatg 2723

gataagtctg tggcccaatc cccttcctct ccttttaagg gtgaaatgac tgcatttaaa 2783

agaagttaaa gagttcctcc tgtcccctat aaccacaagg aaacaaaaaa atatataaaa 2843

acctcaaaaa tgcattgcca tgattttatt attagtgtcc aaaatgggac tcccaagtaa 2903

taaatgattt attccagcca cagccaaaaa agactttgcc tggctaaaag agtctctctc 2963

taagtatgta atatacaaga aatacaattc aaagagatgt tcctataagt acatttttta 3023

cacggcatat atttaaaaag gaggcccctt ttaatataaa attccggtta tataccaata 3083

tggttaatta gcatttacac tatagtttga acgtatttta aatagcatga tgtgtataca 3143

atgtctcccg cgcccattgg caaccagggt cgtgggaagc ttggtgagga gttaaccagg 3203

tcctgtggtt taagcagtgg agcacccggg attcctgccc ccctttctgc tcacacaatt 3263

gcactccatt cttccgcctt ccttgttttc tccaaaacca cctgataggg gggatgtcct 3323

gatttctgag gtgtgcttct catcatgact gcttcgtttt gcccttctga tttccacggc 3383

acaagattat ctaccaaaat caaaacagaa tggccttact cttctcagga agaggctggt 3443

aggcaggtgc attatcaaca ggtctgtgcc catgcagagt gagcagggag aggctgggca 3503

ctgtggaatt tttctgtctg aactcgctca tggccacaga atggtcaccc agcttattta 3563

ggtgtagaca agtatgacac agttctagaa aatactgact ataaaaatgt ctctgtgtgt 3623

gtgtgtatgt atttatatgt atatgtatat atttttaaaa ggctcatctt acttgtaaac 3683

atggactgct caatcactat taaaaagtca gtttaggctg ggcgcggtgg ctcacgcctg 3743

tagtcccaga gctttgggag gctgaggtgg gtggatcact gggtcaggag tttgagacca 3803

gcctggccaa catggtgaaa ccccatcgct actaaaaaat acaaaaatta gccgggcatg 3863

gtggcgctca cctgtaatcc cggctactcg ggaggctgag gcaggagaga atcgcttgaa 3923

ccggggaggt ggaggctgca gtgagccgag atcgcaccac tgcactccag cctgggtgat 3983

ggagcaagac tccatctcaa aaaaaaaaaa gtcagtttag gctgggcgca gtggctcaca 4043

cctgtagtcc cagcacttta ggaggctgag gggggtgatc acctgaggtc aggagtttga 4103

gaccagcctg gccaacatgg tgaaatcctg tctctgctaa aaatacaaaa tttagctggg 4163

catggtggcg tgcctgaaac cccagctact tgggaggctg aggcactaga atcgcttgag 4223

cctgggaggt ggaggttgca gtgagtggag atcgcgccaa cacattctag cctgagggac 4283

agagtgagac tctatcatct c 4304

<210> 2
<211> 525
<212> PRT
<213> Homo sapiens

<400> 2

Met Gly Trp Leu Phe Leu Lys Val Leu Leu Ala Gly Val Ser Phe Ser
1 5 10 15

Gly Phe Leu Tyr Pro Leu Val Asp Phe Cys Ile Ser Gly Lys Thr Arg
20 25 30

Gly Gln Lys Pro Asn Phe Val Ile Ile Leu Ala Asp Asp Met Gly Trp
35 40 45

Gly Asp Leu Gly Ala Asn Trp Ala Glu Thr Lys Asp Thr Ala Asn Leu

50 55 60

Asp Lys Met Ala Ser Glu Gly Met Arg Phe Val Asp Phe His Ala Ala

65 70 75 80

Ala Ser Thr Cys Ser Pro Ser Arg Ala Ser Leu Leu Thr Gly Arg Leu

85 90 95

Gly Leu Arg Asn Gly Val Thr Arg Asn Phe Ala Val Thr Ser Val Gly

100 105 110

Gly Leu Pro Leu Asn Glu Thr Thr Leu Ala Glu Val Leu Gln Gln Ala

115 120 125

Gly Tyr Val Thr Gly Ile Ile Gly Lys Trp His Leu Gly His His Gly

130 135 140

Ser Tyr His Pro Asn Phe Arg Gly Phe Asp Tyr Tyr Phe Gly Ile Pro

145 150 155 160

Tyr Ser His Asp Met Gly Cys Thr Asp Thr Pro Gly Tyr Asn His Pro

165 170 175

Pro Cys Pro Ala Cys Pro Gln Gly Asp Gly Pro Ser Arg Asn Leu Gln

180 185 190

Arg Asp Cys Tyr Thr Asp Val Ala Leu Pro Leu Tyr Glu Asn Leu Asn

195 200 205

Ile Val Glu Gln Pro Val Asn Leu Ser Ser Leu Ala Gln Lys Tyr Ala

210 215 220

Glu Lys Ala Thr Gln Phe Ile Gln Arg Ala Ser Thr Ser Gly Arg Pro
225 230 235 240

Phe Leu Leu Tyr Val Ala Leu Ala His Met His Val Pro Leu Pro Val
245 250 255

Thr Gln Leu Pro Ala Ala Pro Arg Gly Arg Ser Leu Tyr Gly Ala Gly
260 265 270

Leu Trp Glu Met Asp Ser Leu Val Gly Gln Ile Lys Asp Lys Val Asp
275 280 285

His Thr Val Lys Glu Asn Thr Phe Leu Trp Phe Thr Gly Asp Asn Gly
290 295 300

Pro Trp Ala Gln Lys Cys Glu Leu Ala Gly Ser Val Gly Pro Phe Thr
305 310 315 320

Gly Phe Trp Gln Thr Arg Gln Gly Gly Ser Pro Ala Lys Gln Thr Thr
325 330 335

Trp Glu Gly Gly His Arg Val Pro Ala Leu Ala Tyr Trp Pro Gly Arg
340 345 350

Val Pro Val Asn Val Thr Ser Thr Ala Leu Leu Ser Val Leu Asp Ile
355 360 365

Phe Pro Thr Val Val Ala Leu Ala Gln Ala Ser Leu Pro Gln Gly Arg
370 375 380

Arg Phe Asp Gly Val Asp Val Ser Glu Val Leu Phe Gly Arg Ser Gln
385 390 395 400

Pro Gly His Arg Val Leu Phe His Pro Asn Ser Gly Ala Ala Gly Glu
405 410 415

Phe Gly Ala Leu Gln Thr Val Arg Leu Glu Arg Tyr Lys Ala Phe Tyr
420 425 430

Ile Thr Gly Gly Ala Arg Ala Cys Asp Gly Ser Thr Gly Pro Glu Leu
435 440 445

Gln His Lys Phe Pro Leu Ile Phe Asn Leu Glu Asp Asp Thr Ala Glu
450 455 460

Ala Val Pro Leu Glu Arg Gly Gly Ala Glu Tyr Gln Ala Val Leu Pro

465 470 475 480

Glu Val Arg Lys Val Leu Ala Asp Val Leu Gln Asp Ile Ala Asn Asp

485 490 495

Asn Ile Ser Ser Pro Asp Tyr Thr Gln Asp Pro Ser Val Thr Pro Cys

500 505 510

Cys Asn Pro Tyr Gln Ile Ala Cys Arg Cys Gln Ala Ala

515 520 525

<210> 3
<211> 2258
<212> DNA
<213> Homo sapiens

<220>
<221> CDS
<222> (20).. (1201)

<400> 3

ggcgttggct gctagagcg atg ccg ggc cgg agt tgc gtc gcc tta gtc ctc 52
Met Pro Gly Arg Ser Cys Val Ala Leu Val Leu
1 5 10

ctg gct gcc gcg tca gct gtg ccg tcg cag cac gcg ccg ccg tgg aca 100
Leu Ala Ala Ala Ser Ala Val Pro Ser Gln His Ala Pro Pro Trp Thr
15 20 25

gag gac tgc aga aaa tca acc tat cct cct tca gga cca acg tac aga 148
Glu Asp Cys Arg Lys Ser Thr Tyr Pro Pro Ser Gly Pro Thr Tyr Arg
30 35 40

ggt gca gtt cca tgg tac acc ata aat ctt gac tta cca ccc tac aaa 196
Gly Ala Val Pro Trp Tyr Thr Ile Asn Leu Asp Leu Pro Pro Tyr Lys
45 50 55

aga tgg cat gaa ttg atg ctt gac aag gca cca atg cta aag gtt ata 244
Arg Trp His Glu Leu Met Leu Asp Lys Ala Pro Met Leu Lys Val Ile
60 65 70 75

gtg aat tct ctg aag aat atg ata aat aca ttc gtg cca agt gga aaa 292
Val Asn Ser Leu Lys Asn Met Ile Asn Thr Phe Val Pro Ser Gly Lys
80 85 90

gtt atg cag gtg gtg gat gaa aaa ttg cct ggc cta ctt ggc aac ttt 340
Val Met Gln Val Val Asp Glu Lys Leu Pro Gly Leu Leu Gly Asn Phe
95 100 105

cct ggc cct ttt gaa gag gaa atg aag ggt att gcc gct gtt act gat 388
Pro Gly Pro Phe Glu Glu Glu Met Lys Gly Ile Ala Ala Val Thr Asp
110 115 120

ata cct tta gga gag att att tca ttc aat att ttt tat gaa tta ttt 436
Ile Pro Leu Gly Glu Ile Ile Ser Phe Asn Ile Phe Tyr Glu Leu Phe
125 130 135

acc att tgt act tca ata gta gca gaa gac aaa aaa ggt cat cta ata 484

Thr Ile Cys Thr Ser Ile Val Ala Glu Asp Lys Lys Gly His Leu Ile
140 145 150 155

cat ggg aga aac atg gat ttt gga gta ttt ctt ggg tgg aac ata aat 532
His Gly Arg Asn Met Asp Phe Gly Val Phe Leu Gly Trp Asn Ile Asn
160 165 170

aat gat acc tgg gtc ata act gag caa cta aaa cct tta aca gtg aat 580
Asn Asp Thr Trp Val Ile Thr Glu Gln Leu Lys Pro Leu Thr Val Asn
175 180 185

ttg gat ttc caa aga aac aac aaa act gtc ttc aag gct tca agc ttt 628
Leu Asp Phe Gln Arg Asn Asn Lys Thr Val Phe Lys Ala Ser Ser Phe
190 195 200

gct ggc tat gtg ggc atg tta aca gga ttc aaa cca gga ctg ttc agt 676
Ala Gly Tyr Val Gly Met Leu Thr Gly Phe Lys Pro Gly Leu Phe Ser
205 210 215

ctt aca ctg aat gaa cgt ttc agt ata aat ggt ggt tat ctg ggt att 724
Leu Thr Leu Asn Glu Arg Phe Ser Ile Asn Gly Gly Tyr Leu Gly Ile

220 225 230 235

cta gaa tgg att ctg gga aag aaa gat gcc atg tgg ata ggg ttc ctc 772

Leu Glu Trp Ile Leu Gly Lys Lys Asp Ala Met Trp Ile Gly Phe Leu

240 245 250

act aga aca gtt ctg gaa aat agc aca agt tat gaa gaa gcc aag aat 820

Thr Arg Thr Val Leu Glu Asn Ser Thr Ser Tyr Glu Glu Ala Lys Asn

255 260 265

tta ttg acc aag acc aag ata ttg gcc cca gcc tac ttt atc ctg gga 868

Leu Leu Thr Lys Thr Lys Ile Leu Ala Pro Ala Tyr Phe Ile Leu Gly

270 275 280

ggc aac cag tct ggg gaa ggt tgt gtg att aca cga gac aga aag gaa 916

Gly Asn Gln Ser Gly Glu Gly Cys Val Ile Thr Arg Asp Arg Lys Glu

285 290 295

tca ttg gat gta tat gaa ctc gat gct aag cag ggt aga tgg tat gtg 964

Ser Leu Asp Val Tyr Glu Leu Asp Ala Lys Gln Gly Arg Trp Tyr Val

300 305 310 315

gta caa aca aat tat gac cgt tgg aaa cat ccc ttc ttc ctt gat gat 1012

Val Gln Thr Asn Tyr Asp Arg Trp Lys His Pro Phe Phe Leu Asp Asp

320 325 330

cgc aga acg cct gca aag atg tgt ctg aac cgc acc agc caa gag aat 1060

Arg Arg Thr Pro Ala Lys Met Cys Leu Asn Arg Thr Ser Gln Glu Asn

335 340 345

atc tca ttt gaa acc atg tat gat gtc ctg tca aca aaa cct gtc ctc 1108

Ile Ser Phe Glu Thr Met Tyr Asp Val Leu Ser Thr Lys Pro Val Leu

350 355 360

aac aag ctg acc gta tac aca acc ttg ata gat gtt acc aaa ggt caa 1156

Asn Lys Leu Thr Val Tyr Thr Thr Leu Ile Asp Val Thr Lys Gly Gln

365 370 375

ttc gaa act tac ctg cgg gac tgc cct gac cct tgt ata ggt tgg 1201

Phe Glu Thr Tyr Leu Arg Asp Cys Pro Asp Pro Cys Ile Gly Trp

380 385 390

tgagcacacg tctggcctac agaatgcggc ctctgagaca tgaagacacc atctccatgt 1261

gaccgaacac tgcagctgtc tgaccttcca aagactaaga ctcgcggcag gttctctttg 1321

agtcaatagc ttgtcttcgt ccatctgttg acaaatgaca gacttttttt ttttccccct 1381

atcagttgat ttttcttatt tatagataac ttctttaggg gaagtaaaac agtcatctag 1441

aattcactga gttttgtttc actttgacat ttggggatct ggtgggcagt cgaaccatgg 1501

tgaactccac ctccgtgaat aaatggagat tcagcgtggg tgttgaatcc agcacgtctg 1561

tgtgagtaac gggacagtaa acactccaca ttcttcagtt tttcacttct acctacatat 1621

ttgtatgttt ttctgtataa cagccttttc cttctggttc taactgctgt taaaattaat 1681

atatcattat ctttgctgtt attgacagcg atataatttt attacatatg attagaggga 1741

tgagacagac attcacctgt atatttcttt taatgggcac aaaattggtg cctttgcctc 1801

taaatagcac tttttcgggg tcaagaagta atcagatgca aagcaatcgt ttatacaata 1861

attgaagcgc acctttcaat accactccag tacctaagga agtgctacta aactgcatcc 1921

acgtctgtat agtaataaca gtcaagctgg aatcgaggac caattaattc caatggcaca 1981

gagtagcatt catgtaataa acaggttttt agtttgttct tcagattgat agggagtttt 2041

aaagaaattt tagtagttac taaaattatg ttactgtatt tttcagaaat ccaactgctt 2101

atgaaaagta ctaatagaac ttgttaacct ttctaacctt cacgattaac tgtgaaatgt 2161

acgtcatttg tgcaagaccg tttgtccact tcattttgta taatcacagt tgtgttcctg 2221

acactcaata aacagtcatt ggaaagagaa aaaaaaa 2258

<210> 4
<211> 394
<212> PRT
<213> Homo sapiens

<400> 4

Met Pro Gly Arg Ser Cys Val Ala Leu Val Leu Leu Ala Ala Ala Ser
1 5 10 15

Ala Val Pro Ser Gln His Ala Pro Pro Trp Thr Glu Asp Cys Arg Lys
20 25 30

Ser Thr Tyr Pro Pro Ser Gly Pro Thr Tyr Arg Gly Ala Val Pro Trp
35 40 45

Tyr Thr Ile Asn Leu Asp Leu Pro Pro Tyr Lys Arg Trp His Glu Leu
50 55 60

Met Leu Asp Lys Ala Pro Met Leu Lys Val Ile Val Asn Ser Leu Lys
65 70 75 80

Asn Met Ile Asn Thr Phe Val Pro Ser Gly Lys Val Met Gln Val Val
85 90 95

Asp Glu Lys Leu Pro Gly Leu Leu Gly Asn Phe Pro Gly Pro Phe Glu
100 105 110

Glu Glu Met Lys Gly Ile Ala Ala Val Thr Asp Ile Pro Leu Gly Glu
115 120 125

Ile Ile Ser Phe Asn Ile Phe Tyr Glu Leu Phe Thr Ile Cys Thr Ser
130 135 140

Ile Val Ala Glu Asp Lys Lys Gly His Leu Ile His Gly Arg Asn Met
145 150 155 160

Asp Phe Gly Val Phe Leu Gly Trp Asn Ile Asn Asn Asp Thr Trp Val
165 170 175

Ile Thr Glu Gln Leu Lys Pro Leu Thr Val Asn Leu Asp Phe Gln Arg
180 185 190

Asn Asn Lys Thr Val Phe Lys Ala Ser Ser Phe Ala Gly Tyr Val Gly
195 200 205

Met Leu Thr Gly Phe Lys Pro Gly Leu Phe Ser Leu Thr Leu Asn Glu
210 215 220

Arg Phe Ser Ile Asn Gly Gly Tyr Leu Gly Ile Leu Glu Trp Ile Leu
225 230 235 240

Gly Lys Lys Asp Ala Met Trp Ile Gly Phe Leu Thr Arg Thr Val Leu

245 250 255

Glu Asn Ser Thr Ser Tyr Glu Glu Ala Lys Asn Leu Leu Thr Lys Thr

260 265 270

Lys Ile Leu Ala Pro Ala Tyr Phe Ile Leu Gly Gly Asn Gln Ser Gly

275 280 285

Glu Gly Cys Val Ile Thr Arg Asp Arg Lys Glu Ser Leu Asp Val Tyr

290 295 300

Glu Leu Asp Ala Lys Gln Gly Arg Trp Tyr Val Val Gln Thr Asn Tyr

305 310 315 320

Asp Arg Trp Lys His Pro Phe Phe Leu Asp Asp Arg Arg Thr Pro Ala

325 330 335

Lys Met Cys Leu Asn Arg Thr Ser Gln Glu Asn Ile Ser Phe Glu Thr

340 345 350

Met Tyr Asp Val Leu Ser Thr Lys Pro Val Leu Asn Lys Leu Thr Val

355 360 365

Tyr Thr Thr Leu Ile Asp Val Thr Lys Gly Gln Phe Glu Thr Tyr Leu

370 375 380

Arg Asp Cys Pro Asp Pro Cys Ile Gly Trp

385 390

<210> 5
<211> 1336
<212> DNA
<213> Homo sapiens

<220>
<221> CDS
<222> (474).. (1241)

<400> 5

agcacagtcc cgcggacggc tgagcgtgtg gctgcaggag cttctgtggg agtacggtca 60

tgagcctttt gctgtactat gccctccctg ccctgggcag ctatgccatg ctctccatct 120

tcttcctgcg ccggcctcat ctgctgcaca cgcccagggc tcccaccttc cgcatccgcc 180

tgggggccca ccgaggagga tctggagagc tgctggagaa caccatggag gccatggaga 240

agtgagtgta tctgcccctg cccaggctgc catgtgaggg tgtgcaggtc gtggactgca 300

tcacagcaag ggacgccatt cacttcctag ctgggtgact ggcgcccccct ggaggtggcc 360

tgcacatccc gccacgaact ccgctctcac attcccggcc acctcccagc caccatgccc 420

ctcatcccag tcccctgccc ctgccccccg ctgaccttca cccccacagc tcc atg 476

Met

1

gcc cag cgc tcg gac ctc ctg gag ctc gac tgt cag ctg aca cgg gac 524

Ala Gln Arg Ser Asp Leu Leu Glu Leu Asp Cys Gln Leu Thr Arg Asp

5 10 15

aga gtg gtg gtg gtg tca cat gat gag aac ctg tgc cgc cag tcg ggc 572

Arg Val Val Val Val Ser His Asp Glu Asn Leu Cys Arg Gln Ser Gly

20 25 30

cta aac agg gat gtg ggc agc ctg gac ttc gag gac ctg ccc ctc tac 620

Leu Asn Arg Asp Val Gly Ser Leu Asp Phe Glu Asp Leu Pro Leu Tyr

35 40 45

aag gag aag ctg gag gtt tac ttc tct cca ggc cac ttt gct cac ggg 668

Lys Glu Lys Leu Glu Val Tyr Phe Ser Pro Gly His Phe Ala His Gly

50 55 60 65

tca gac cgg cgc atg gtt cgt ctg gag gac ctg ttc cag agg ttt cca 716
Ser Asp Arg Arg Met Val Arg Leu Glu Asp Leu Phe Gln Arg Phe Pro
70 75 80

agg aca ccc atg agc gta gag atc aaa ggg aag aac gaa gag ctc atc 764
Arg Thr Pro Met Ser Val Glu Ile Lys Gly Lys Asn Glu Glu Leu Ile
85 90 95

cgt gag ata gca ggc ttg gtg aga cgc tat gac cgt aat gaa atc acc 812
Arg Glu Ile Ala Gly Leu Val Arg Arg Tyr Asp Arg Asn Glu Ile Thr
100 105 110

atc tgg gcc tcg gag aag agc tcg gtc atg aag aaa tgc aag gct gcc 860
Ile Trp Ala Ser Glu Lys Ser Ser Val Met Lys Lys Cys Lys Ala Ala
115 120 125

aac ccc gag atg ccc ctg tcc ttc aca ata agc cga gga ttc tgg gtg 908
Asn Pro Glu Met Pro Leu Ser Phe Thr Ile Ser Arg Gly Phe Trp Val
130 135 140 145

ctg ctt tcc tac tac ctg ggg ctg ctg ccc ttc atc cca atc cct gag 956

Leu Leu Ser Tyr Tyr Leu Gly Leu Leu Pro Phe Ile Pro Ile Pro Glu

150 155 160

aag ttc ttc ttc tgc ttc ctg ccc aac atc atc aac agg acc tat ttc 1004

Lys Phe Phe Phe Cys Phe Leu Pro Asn Ile Ile Asn Arg Thr Tyr Phe

165 170 175

cca ttt tcc tgc tct tgc ctg aac cag tta ttg gct gtg gtt tcg aaa 1052

Pro Phe Ser Cys Ser Cys Leu Asn Gln Leu Leu Ala Val Val Ser Lys

180 185 190

tgg ctg atc atg agg aag agt ctg atc cga cac ttg gag gag cga ggg 1100

Trp Leu Ile Met Arg Lys Ser Leu Ile Arg His Leu Glu Glu Arg Gly

195 200 205

gtg cag gtg gtc ttt tgg tgc ctt aat gaa gag tcg gat ttt gaa gca 1148

Val Gln Val Val Phe Trp Cys Leu Asn Glu Glu Ser Asp Phe Glu Ala

210 215 220 225

gcc ttc agc gtg gga gcc act ggc gtc ata acg gat tat ccc aca gcc 1196

Ala Phe Ser Val Gly Ala Thr Gly Val Ile Thr Asp Tyr Pro Thr Ala

230 235 240

ctg cgg cac tac ctg gac aac cat gga cca gct gcc cgg acc tcc 1241
Leu Arg His Tyr Leu Asp Asn His Gly Pro Ala Ala Arg Thr Ser

245 250 255

taagtccaga agcctcgagg tcttctgttt ctcttcctga aaaataaata tttgcctttc 1301

gatcaaaaaa aaaaaaaaaa aaaaaaaaaa aaaaa 1336

<210> 6
<211> 256
<212> PRT
<213> Homo sapiens

<400> 6

Met Ala Gln Arg Ser Asp Leu Leu Glu Leu Asp Cys Gln Leu Thr Arg

1 5 10 15

Asp Arg Val Val Val Val Ser His Asp Glu Asn Leu Cys Arg Gln Ser

20 25 30

Gly Leu Asn Arg Asp Val Gly Ser Leu Asp Phe Glu Asp Leu Pro Leu

35 40 45

Tyr Lys Glu Lys Leu Glu Val Tyr Phe Ser Pro Gly His Phe Ala His

50 55 60

Gly Ser Asp Arg Arg Met Val Arg Leu Glu Asp Leu Phe Gln Arg Phe

65 70 75 80

Pro Arg Thr Pro Met Ser Val Glu Ile Lys Gly Lys Asn Glu Glu Leu

85 90 95

Ile Arg Glu Ile Ala Gly Leu Val Arg Arg Tyr Asp Arg Asn Glu Ile

100 105 110

Thr Ile Trp Ala Ser Glu Lys Ser Ser Val Met Lys Lys Cys Lys Ala

115 120 125

Ala Asn Pro Glu Met Pro Leu Ser Phe Thr Ile Ser Arg Gly Phe Trp

130 135 140

Val Leu Leu Ser Tyr Tyr Leu Gly Leu Leu Pro Phe Ile Pro Ile Pro

145 150 155 160

Glu Lys Phe Phe Phe Cys Phe Leu Pro Asn Ile Ile Asn Arg Thr Tyr

165 170 175

Phe Pro Phe Ser Cys Ser Cys Leu Asn Gln Leu Leu Ala Val Val Ser

180 185 190

Lys Trp Leu Ile Met Arg Lys Ser Leu Ile Arg His Leu Glu Glu Arg

195 200 205

Gly Val Gln Val Val Phe Trp Cys Leu Asn Glu Glu Ser Asp Phe Glu

210 215 220

Ala Ala Phe Ser Val Gly Ala Thr Gly Val Ile Thr Asp Tyr Pro Thr

225 230 235 240

Ala Leu Arg His Tyr Leu Asp Asn His Gly Pro Ala Ala Arg Thr Ser

245 250 255

<210> 7
<211> 2631
<212> DNA
<213> Homo sapiens

<220>
<221> CDS
<222> (19) .. (2214)

<400> 7

gagagcactg cagcagca atg acg gag ggc acg tgt ctg cgg cgc cga ggg 51
Met Thr Glu Gly Thr Cys Leu Arg Arg Arg Gly
1 5 10

ggc ccc tac aag acc gag ccc gcc acc gac ctc ggc cgc tgg cga ctc 99
Gly Pro Tyr Lys Thr Glu Pro Ala Thr Asp Leu Gly Arg Trp Arg Leu
15 20 25

aac tgc gag agg ggc cgg cag acg tgg acc tac ctg cag gac gag cgc 147
Asn Cys Glu Arg Gly Arg Gln Thr Trp Thr Tyr Leu Gln Asp Glu Arg
30 35 40

gcc ggc cgc gag cag acc ggc ctg gaa gcc tac gcc ctg ggg ctg gac 195
Ala Gly Arg Glu Gln Thr Gly Leu Glu Ala Tyr Ala Leu Gly Leu Asp
45 50 55

acc aag aat tac ttt aag gac ttg ccc aaa gcc cac acc gcc ttt gag 243
Thr Lys Asn Tyr Phe Lys Asp Leu Pro Lys Ala His Thr Ala Phe Glu
60 65 70 75

ggg gct ctg aac ggg atg aca ttt tac gtg ggg ctg cag gct gag gat 291
Gly Ala Leu Asn Gly Met Thr Phe Tyr Val Gly Leu Gln Ala Glu Asp
80 85 90

ggg cac tgg acg ggt gat tat ggt ggc cca ctt ttc ctc ctg cca ggc 339
Gly His Trp Thr Gly Asp Tyr Gly Gly Pro Leu Phe Leu Leu Pro Gly
95 100 105

ctc ctg atc act tgc cac gtg gca cgc atc cct ctg cca gcc gga tac 387
Leu Leu Ile Thr Cys His Val Ala Arg Ile Pro Leu Pro Ala Gly Tyr
110 115 120

aga gaa gag att gtg cgg tac ctg cgg tca gtg cag ctc cct gac ggt 435

Arg Glu Glu Ile Val Arg Tyr Leu Arg Ser Val Gln Leu Pro Asp Gly
125 130 135

ggc tgg ggc ctg cac att gag gat aag tcc acc gtg ttt ggg act gcg 483
Gly Trp Gly Leu His Ile Glu Asp Lys Ser Thr Val Phe Gly Thr Ala
140 145 150 155

ctc aac tat gtg tct ctc aga att ctg ggt gtt ggg cct gac gat cct 531
Leu Asn Tyr Val Ser Leu Arg Ile Leu Gly Val Gly Pro Asp Asp Pro
160 165 170

gac ctg gta cga gcc cgg aac att ctt cac aag aaa ggt ggt gct gtg 579
Asp Leu Val Arg Ala Arg Asn Ile Leu His Lys Lys Gly Gly Ala Val
175 180 185

gcc atc ccc tcc tgg ggg aag ttc tgg ctg gct gtc ctg aat gtt tac 627
Ala Ile Pro Ser Trp Gly Lys Phe Trp Leu Ala Val Leu Asn Val Tyr
190 195 200

agc tgg gaa ggc ctc aat acc ctg ttc cca gag atg tgg ctg ttt cct 675
Ser Trp Glu Gly Leu Asn Thr Leu Phe Pro Glu Met Trp Leu Phe Pro

205 210 215

gac tgg gca ccg gca cac ccc tcc aca ctc tgg tgc cac tgc cgg cag 723

Asp Trp Ala Pro Ala His Pro Ser Thr Leu Trp Cys His Cys Arg Gln

220 225 230 235

gtg tac ctg ccc atg agc tac tgc tac gcc gtt cgg ctg agt gcc gcg 771

Val Tyr Leu Pro Met Ser Tyr Cys Tyr Ala Val Arg Leu Ser Ala Ala

240 245 250

gaa gac ccg ctg gtc cag agc ctc cgc cag gag ctc tat gtg gag gac 819

Glu Asp Pro Leu Val Gln Ser Leu Arg Gln Glu Leu Tyr Val Glu Asp

255 260 265

ttc gcc agc att gac tgg ctg gcg cag agg aac aac gtg gcc ccc gac 867

Phe Ala Ser Ile Asp Trp Leu Ala Gln Arg Asn Asn Val Ala Pro Asp

270 275 280

gag ctg tac acg ccg cac agc tgg ctg ctc cgc gtg gta tat gcg ctc 915

Glu Leu Tyr Thr Pro His Ser Trp Leu Leu Arg Val Val Tyr Ala Leu

285 290 295

ctc aac ctg tat gag cac cac cac agt gcc cac ctg cgg cag cgg gcc 963

Leu Asn Leu Tyr Glu His His His Ser Ala His Leu Arg Gln Arg Ala

300 305 310 315

gtg cag aag ctg tat gaa cac att gtg gcc gac gac cga ttc acc aag 1011

Val Gln Lys Leu Tyr Glu His Ile Val Ala Asp Asp Arg Phe Thr Lys

320 325 330

agc atc agc atc ggc ccg atc tcg aaa acc atc aac atg ctt gtg cgc 1059

Ser Ile Ser Ile Gly Pro Ile Ser Lys Thr Ile Asn Met Leu Val Arg

335 340 345

tgg tat gtg gac ggg ccc gcc tcc act gcc ttc cag gag cat gtc tcc 1107

Trp Tyr Val Asp Gly Pro Ala Ser Thr Ala Phe Gln Glu His Val Ser

350 355 360

aga atc ccg gac tat ctc tgg atg ggc ctt gac ggc atg aaa atg cag 1155

Arg Ile Pro Asp Tyr Leu Trp Met Gly Leu Asp Gly Met Lys Met Gln

365 370 375

ggc acc aac ggc tca cag atc tgg gac acc gca ttc gcc atc cag gct 1203
Gly Thr Asn Gly Ser Gln Ile Trp Asp Thr Ala Phe Ala Ile Gln Ala
380 385 390 395

ctg ctt gag gcg ggc ggg cac cac agg ccc gag ttt tcg tcc tgc ctg 1251
Leu Leu Glu Ala Gly Gly His His Arg Pro Glu Phe Ser Ser Cys Leu
400 405 410

cag aag gct cat gag ttc ctg agg ctc tca cag gtc cca gat aac cct 1299
Gln Lys Ala His Glu Phe Leu Arg Leu Ser Gln Val Pro Asp Asn Pro
415 420 425

ccc gac tac cag aag tac tac cgc cag atg cgc aag ggt ggc ttc tcc 1347
Pro Asp Tyr Gln Lys Tyr Tyr Arg Gln Met Arg Lys Gly Gly Phe Ser
430 435 440

ttc agt acg ctg gac tgc ggc tgg atc gtt tct gac tgc acg gct gag 1395
Phe Ser Thr Leu Asp Cys Gly Trp Ile Val Ser Asp Cys Thr Ala Glu
445 450 455

gcc ttg aag gct gtg ctg ctc ctg cag gag aag tgt ccc cat gtc acc 1443

Ala Leu Lys Ala Val Leu Leu Leu Gln Glu Lys Cys Pro His Val Thr

460 465 470 475

gag cac atc ccc aga gaa cgg ctc tgc gat gct gtg gct gtg ctg ctg 1491

Glu His Ile Pro Arg Glu Arg Leu Cys Asp Ala Val Ala Val Leu Leu

480 485 490

aac atg aga aat cca gat gga ggg ttc gcc acc tat gag acc aag cgt 1539

Asn Met Arg Asn Pro Asp Gly Gly Phe Ala Thr Tyr Glu Thr Lys Arg

495 500 505

ggg ggg cac ttg ctg gag ctg ctg aac ccc tcg gag gtc ttc ggg gac 1587

Gly Gly His Leu Leu Glu Leu Leu Asn Pro Ser Glu Val Phe Gly Asp

510 515 520

atc atg att gac tac acc tat gtg gag tgc acc tca gcc gtg atg cag 1635

Ile Met Ile Asp Tyr Thr Tyr Val Glu Cys Thr Ser Ala Val Met Gln

525 530 535

gcg ctt aag tat ttc cac aag cgt ttc ccg gag cac agg gca gcg gag 1683

Ala Leu Lys Tyr Phe His Lys Arg Phe Pro Glu His Arg Ala Ala Glu

540 545 550 555

atc cgg gag acc ctc acg cag ggc tta gag ttc tgt cgg cgg cag cag 1731
Ile Arg Glu Thr Leu Thr Gln Gly Leu Glu Phe Cys Arg Arg Gln Gln
560 565 570

agg gcc gat ggc tcc tgg gaa ggc tcc tgg gga gtt tgc ttc acc tac 1779
Arg Ala Asp Gly Ser Trp Glu Gly Ser Trp Gly Val Cys Phe Thr Tyr
575 580 585

ggc acc tgg ttt ggc ctg gag gcc ttc gcc tgt atg ggg cag acc tac 1827
Gly Thr Trp Phe Gly Leu Glu Ala Phe Ala Cys Met Gly Gln Thr Tyr
590 595 600

cga gat ggg act gcc tgt gca gag gtc tcc cgg gcc tgt gac ttc ctg 1875
Arg Asp Gly Thr Ala Cys Ala Glu Val Ser Arg Ala Cys Asp Phe Leu
605 610 615

ctg tcc cgg cag atg gca gac gga ggc tgg ggg gag gac ttt gag tcc 1923
Leu Ser Arg Gln Met Ala Asp Gly Gly Trp Gly Glu Asp Phe Glu Ser
620 625 630 635

tgc gag gag cgg cgt tat ttg cag agt gcc cag tcc cag atc cat aac 1971
Cys Glu Glu Arg Arg Tyr Leu Gln Ser Ala Gln Ser Gln Ile His Asn
640 645 650

aca tgc tgg gcc atg atg ggg ctg atg gcc gtt cgg cat cct gac atc 2019
Thr Cys Trp Ala Met Met Gly Leu Met Ala Val Arg His Pro Asp Ile
655 660 665

gag gcc cag gag aga gga gtc cgg tgt cta ctt gag aaa cag ctc ccc 2067
Glu Ala Gln Glu Arg Gly Val Arg Cys Leu Leu Glu Lys Gln Leu Pro
670 675 680

aat ggc gac tgg ccg cag gaa aac att gct ggg gtc ttc aac aag tcc 2115
Asn Gly Asp Trp Pro Gln Glu Asn Ile Ala Gly Val Phe Asn Lys Ser
685 690 695

tgt gcc atc tcc tac acg agc tac agg aac atc ttc ccc atc tgg gcc 2163
Cys Ala Ile Ser Tyr Thr Ser Tyr Arg Asn Ile Phe Pro Ile Trp Ala
700 705 710 715

ctc ggc cgc ttc tcc cag ctg tac cct gag aga gcc ctt gct ggc cac 2211

Leu Gly Arg Phe Ser Gln Leu Tyr Pro Glu Arg Ala Leu Ala Gly His

720 725 730

ccc tgagaacatg cctacctgct gggtgccgtc tgtgcgttcc atggccttca 2264

Pro

agtcacagga cgcagcgatt ccctgccctc ttcggtgtta ttacacaggc aggacttcag 2324

tgtcagtatc cctgccttca gtcttcttta gaaatcacat ctgtgttcaa tccattgttt 2384

agagggagtg tatttttcct gttccacgaa gaggactttt tgttcacaat tggatcacaa 2444

tgcagaggag tctgttcctc cccegtcggc ttctcggtgc tgggagggtg acctgtccca 2504

gatgactcat caccctgaca tgctcttgac aaaggacacc accaagagga gatggcagct 2564

gtaccggtgc agcctctgtc tgagggggat atttgcctca gtgtgattaa aaatcagtca 2624

tgaaaga 2631

<210> 8
<211> 732
<212> PRT
<213> Homo sapiens

<400> 8

Met Thr Glu Gly Thr Cys Leu Arg Arg Arg Gly Gly Pro Tyr Lys Thr
1 5 10 15

Glu Pro Ala Thr Asp Leu Gly Arg Trp Arg Leu Asn Cys Glu Arg Gly
20 25 30

Arg Gln Thr Trp Thr Tyr Leu Gln Asp Glu Arg Ala Gly Arg Glu Gln
35 40 45

Thr Gly Leu Glu Ala Tyr Ala Leu Gly Leu Asp Thr Lys Asn Tyr Phe

50 55 60

Lys Asp Leu Pro Lys Ala His Thr Ala Phe Glu Gly Ala Leu Asn Gly

65 70 75 80

Met Thr Phe Tyr Val Gly Leu Gln Ala Glu Asp Gly His Trp Thr Gly

85 90 95

Asp Tyr Gly Gly Pro Leu Phe Leu Leu Pro Gly Leu Leu Ile Thr Cys

100 105 110

His Val Ala Arg Ile Pro Leu Pro Ala Gly Tyr Arg Glu Glu Ile Val

115 120 125

Arg Tyr Leu Arg Ser Val Gln Leu Pro Asp Gly Gly Trp Gly Leu His

130 135 140

Ile Glu Asp Lys Ser Thr Val Phe Gly Thr Ala Leu Asn Tyr Val Ser
145 150 155 160

Leu Arg Ile Leu Gly Val Gly Pro Asp Asp Pro Asp Leu Val Arg Ala
165 170 175

Arg Asn Ile Leu His Lys Lys Gly Gly Ala Val Ala Ile Pro Ser Trp
180 185 190

Gly Lys Phe Trp Leu Ala Val Leu Asn Val Tyr Ser Trp Glu Gly Leu
195 200 205

Asn Thr Leu Phe Pro Glu Met Trp Leu Phe Pro Asp Trp Ala Pro Ala
210 215 220

His Pro Ser Thr Leu Trp Cys His Cys Arg Gln Val Tyr Leu Pro Met

225 230 235 240

Ser Tyr Cys Tyr Ala Val Arg Leu Ser Ala Ala Glu Asp Pro Leu Val

245 250 255

Gln Ser Leu Arg Gln Glu Leu Tyr Val Glu Asp Phe Ala Ser Ile Asp

260 265 270

Trp Leu Ala Gln Arg Asn Asn Val Ala Pro Asp Glu Leu Tyr Thr Pro

275 280 285

His Ser Trp Leu Leu Arg Val Val Tyr Ala Leu Leu Asn Leu Tyr Glu

290 295 300

His His His Ser Ala His Leu Arg Gln Arg Ala Val Gln Lys Leu Tyr

305 310 315 320

Glu His Ile Val Ala Asp Asp Arg Phe Thr Lys Ser Ile Ser Ile Gly

325 330 335

Pro Ile Ser Lys Thr Ile Asn Met Leu Val Arg Trp Tyr Val Asp Gly

340 345 350

Pro Ala Ser Thr Ala Phe Gln Glu His Val Ser Arg Ile Pro Asp Tyr

355 360 365

Leu Trp Met Gly Leu Asp Gly Met Lys Met Gln Gly Thr Asn Gly Ser

370 375 380

Gln Ile Trp Asp Thr Ala Phe Ala Ile Gln Ala Leu Leu Glu Ala Gly

385 390 395 400

Gly His His Arg Pro Glu Phe Ser Ser Cys Leu Gln Lys Ala His Glu

405 410 415

Phe Leu Arg Leu Ser Gln Val Pro Asp Asn Pro Pro Asp Tyr Gln Lys

420 425 430

Tyr Tyr Arg Gln Met Arg Lys Gly Gly Phe Ser Phe Ser Thr Leu Asp

435 440 445

Cys Gly Trp Ile Val Ser Asp Cys Thr Ala Glu Ala Leu Lys Ala Val

450 455 460

Leu Leu Leu Gln Glu Lys Cys Pro His Val Thr Glu His Ile Pro Arg

465 470 475 480

Glu Arg Leu Cys Asp Ala Val Ala Val Leu Leu Asn Met Arg Asn Pro

485 490 495

Asp Gly Gly Phe Ala Thr Tyr Glu Thr Lys Arg Gly Gly His Leu Leu

500 505 510

Glu Leu Leu Asn Pro Ser Glu Val Phe Gly Asp Ile Met Ile Asp Tyr

515 520 525

Thr Tyr Val Glu Cys Thr Ser Ala Val Met Gln Ala Leu Lys Tyr Phe

530 535 540

His Lys Arg Phe Pro Glu His Arg Ala Ala Glu Ile Arg Glu Thr Leu

545 550 555 560

Thr Gln Gly Leu Glu Phe Cys Arg Arg Gln Gln Arg Ala Asp Gly Ser
565 570 575

Trp Glu Gly Ser Trp Gly Val Cys Phe Thr Tyr Gly Thr Trp Phe Gly
580 585 590

Leu Glu Ala Phe Ala Cys Met Gly Gln Thr Tyr Arg Asp Gly Thr Ala
595 600 605

Cys Ala Glu Val Ser Arg Ala Cys Asp Phe Leu Leu Ser Arg Gln Met
610 615 620

Ala Asp Gly Gly Trp Gly Glu Asp Phe Glu Ser Cys Glu Glu Arg Arg
625 630 635 640

Tyr Leu Gln Ser Ala Gln Ser Gln Ile His Asn Thr Cys Trp Ala Met

645 650 655

Met Gly Leu Met Ala Val Arg His Pro Asp Ile Glu Ala Gln Glu Arg

660 665 670

Gly Val Arg Cys Leu Leu Glu Lys Gln Leu Pro Asn Gly Asp Trp Pro

675 680 685

Gln Glu Asn Ile Ala Gly Val Phe Asn Lys Ser Cys Ala Ile Ser Tyr

690 695 700

Thr Ser Tyr Arg Asn Ile Phe Pro Ile Trp Ala Leu Gly Arg Phe Ser

705 710 715 720

Gln Leu Tyr Pro Glu Arg Ala Leu Ala Gly His Pro

725 730

<210> 9
<211> 1168
<212> DNA
<213> Homo sapiens

<220>
<221> CDS
<222> (30).. (800)

<400> 9

gagctggaag tgagagcaga tccctaacc atg agc acc agc caa cca ggg gcc 53

Met Ser Thr Ser Gln Pro Gly Ala

1 5

tgc cca tgc cag gga gct gca agc cgc ccc gcc att ctc tac gca ctt 101

Cys Pro Cys Gln Gly Ala Ala Ser Arg Pro Ala Ile Leu Tyr Ala Leu

10 15 20

ctg agc tcc agc ctc aag gct gtc ccc cga ccc cgt agc cgc tgc cta 149
Leu Ser Ser Ser Leu Lys Ala Val Pro Arg Pro Arg Ser Arg Cys Leu
25 30 35 40

tgt agg cag cac cgg ccc gtc cag cta tgt gca cct cat cgc acc tgc 197
Cys Arg Gln His Arg Pro Val Gln Leu Cys Ala Pro His Arg Thr Cys
45 50 55

cgg gag gcc ttg gat gtt ctg gcc aag aca gtg gcc ttc ctc agg aac 245
Arg Glu Ala Leu Asp Val Leu Ala Lys Thr Val Ala Phe Leu Arg Asn
60 65 70

ctg cca tcc ttc tgg cag ctg cct ccc cag gac cag cgg cgg ctg ctg 293
Leu Pro Ser Phe Trp Gln Leu Pro Pro Gln Asp Gln Arg Arg Leu Leu
75 80 85

cag ggt tgc tgg ggc ccc ctc ttc ctg ctt ggg ttg gcc caa gat gct 341
Gln Gly Cys Trp Gly Pro Leu Phe Leu Leu Gly Leu Ala Gln Asp Ala
90 95 100

gtg acc ttt gag gtg gct gag gcc ccg gtg ccc agc ata ctc aag aag 389
Val Thr Phe Glu Val Ala Glu Ala Pro Val Pro Ser Ile Leu Lys Lys
105 110 115 120

att ctg ctg gag gag ccc agc agc agt gga ggc agt ggc caa ctg cca 437
Ile Leu Leu Glu Glu Pro Ser Ser Ser Gly Gly Ser Gly Gln Leu Pro
125 130 135

gac aga ccc cag ccc tcc ctg gct gcg gtg cag tgg ctt caa tgc tgt 485
Asp Arg Pro Gln Pro Ser Leu Ala Ala Val Gln Trp Leu Gln Cys Cys
140 145 150

ctg gag tcc ttc tgg agc ctg gag ctt agc ccc aag gaa tat gcc tgc 533
Leu Glu Ser Phe Trp Ser Leu Glu Leu Ser Pro Lys Glu Tyr Ala Cys
155 160 165

ctg aaa ggg acc atc ctc ttc aac ccc gat gtg cca ggc ctc caa gcc 581
Leu Lys Gly Thr Ile Leu Phe Asn Pro Asp Val Pro Gly Leu Gln Ala
170 175 180

gcc tcc cac att ggg cac ctg cag cag gag gct cac tgg gtg ctg tgt 629
Ala Ser His Ile Gly His Leu Gln Gln Glu Ala His Trp Val Leu Cys
185 190 195 200

gaa gtc ctg gaa ccc tgg tgc cca gca gcc caa ggc cgc ctg acc cgt 677
Glu Val Leu Glu Pro Trp Cys Pro Ala Ala Gln Gly Arg Leu Thr Arg
205 210 215

gtc ctc ctc acg gcc tcc acc ctc aag tcc att ccg acc agc ctg ctt 725
Val Leu Leu Thr Ala Ser Thr Leu Lys Ser Ile Pro Thr Ser Leu Leu
220 225 230

ggg gac ctc ttc ttt cgc cct atc att gga gat gtt gac atc gct ggc 773
Gly Asp Leu Phe Phe Arg Pro Ile Ile Gly Asp Val Asp Ile Ala Gly
235 240 245

ctt ctt ggg gac atg ctt ttg ctc agg tgacctgttc cagcccaggc 820
Leu Leu Gly Asp Met Leu Leu Leu Arg
250 255

agagatcagg tgggcagagg ctggcagtgc tgattcagcc tggccatccc cagaggtgac 880

ccaatgctcc tggaggggca agcctgtata gacagcactt ggctccttag gaacagctct 940

tcactcagcc acaccccaca ttggacttcc ttggtttgga cacagtgctc cagctgcctg 1000

ggaggctttt ggtggtcccc acagcctctg ggccaagact cctgtccctt cttgggatga 1060

gaatgaaagc ttaggctgct tattggacca gaagtcctat cgactttata cagaactgaa 1120

ttaagttatt gatttttgta ataaaaggta tgaaacacta aaaaaaa 1168

<210> 10
<211> 257
<212> PRT
<213> Homo sapiens

<400> 10

Met Ser Thr Ser Gln Pro Gly Ala Cys Pro Cys Gln Gly Ala Ala Ser
1 5 10 15

Arg Pro Ala Ile Leu Tyr Ala Leu Leu Ser Ser Ser Leu Lys Ala Val
20 25 30

Pro Arg Pro Arg Ser Arg Cys Leu Cys Arg Gln His Arg Pro Val Gln
35 40 45

Leu Cys Ala Pro His Arg Thr Cys Arg Glu Ala Leu Asp Val Leu Ala
50 55 60

Lys Thr Val Ala Phe Leu Arg Asn Leu Pro Ser Phe Trp Gln Leu Pro
65 70 75 80

Pro Gln Asp Gln Arg Arg Leu Leu Gln Gly Cys Trp Gly Pro Leu Phe
85 90 95

Leu Leu Gly Leu Ala Gln Asp Ala Val Thr Phe Glu Val Ala Glu Ala

100 105 110

Pro Val Pro Ser Ile Leu Lys Lys Ile Leu Leu Glu Glu Pro Ser Ser

115 120 125

Ser Gly Gly Ser Gly Gln Leu Pro Asp Arg Pro Gln Pro Ser Leu Ala

130 135 140

Ala Val Gln Trp Leu Gln Cys Cys Leu Glu Ser Phe Trp Ser Leu Glu

145 150 155 160

Leu Ser Pro Lys Glu Tyr Ala Cys Leu Lys Gly Thr Ile Leu Phe Asn

165 170 175

Pro Asp Val Pro Gly Leu Gln Ala Ala Ser His Ile Gly His Leu Gln
180 185 190

Gln Glu Ala His Trp Val Leu Cys Glu Val Leu Glu Pro Trp Cys Pro
195 200 205

Ala Ala Gln Gly Arg Leu Thr Arg Val Leu Leu Thr Ala Ser Thr Leu
210 215 220

Lys Ser Ile Pro Thr Ser Leu Leu Gly Asp Leu Phe Phe Arg Pro Ile
225 230 235 240

Ile Gly Asp Val Asp Ile Ala Gly Leu Leu Gly Asp Met Leu Leu Leu
245 250 255

Arg

<210> 11
<211> 489
<212> DNA
<213> Homo sapiens

<220>

<221> CDS
<222> (43).. (423)

<400> 11

agagccgcag gtcagtcgtg aagagggagc tctattgcca cc atg agt ttc tcc 54
Met Ser Phe Ser
1

ggc aag tac caa ctg cag agc cag gaa aac ttt gaa gcc ttc atg aag 102
Gly Lys Tyr Gln Leu Gln Ser Gln Glu Asn Phe Glu Ala Phe Met Lys
5 10 15 20

gca atc ggt ctg ccg gaa gag ctc atc cag aag ggg aag gat atc aag 150
Ala Ile Gly Leu Pro Glu Glu Leu Ile Gln Lys Gly Lys Asp Ile Lys
25 30 35

ggg gtg tcg gaa atc gtg cag aat ggg aag cac ttc aag ttc acc atc 198
Gly Val Ser Glu Ile Val Gln Asn Gly Lys His Phe Lys Phe Thr Ile
40 45 50

acc gct ggg tcc aaa gtg atc caa aac gaa ttc acg gtg ggg gag gaa 246
Thr Ala Gly Ser Lys Val Ile Gln Asn Glu Phe Thr Val Gly Glu Glu
55 60 65

tgt gag ctg gag aca atg aca ggg gag aaa gtc aag aca gtg gtt cag 294
Cys Glu Leu Glu Thr Met Thr Gly Glu Lys Val Lys Thr Val Val Gln
70 75 80

ttg gaa ggt gac aat aaa ctg gtg aca act ttc aaa aac atc aag tct 342
Leu Glu Gly Asp Asn Lys Leu Val Thr Thr Phe Lys Asn Ile Lys Ser
85 90 95 100

gtg acc gaa ctc aac ggc gac ata atc acc aat acc atg aca ttg ggt 390

Val Thr Glu Leu Asn Gly Asp Ile Ile Thr Asn Thr Met Thr Leu Gly

105 110 115

gac att gtc ttc aag aga atc agc aag aga att taaacaagtc tgcatttcat 443

Asp Ile Val Phe Lys Arg Ile Ser Lys Arg Ile

120 125

attattttag tgtgtaaaat taatgtaata aagtgaactt tgtttt 489

<210> 12
<211> 127
<212> PRT
<213> Homo sapiens

<400> 12

Met Ser Phe Ser Gly Lys Tyr Gln Leu Gln Ser Gln Glu Asn Phe Glu

1 5 10 15

Ala Phe Met Lys Ala Ile Gly Leu Pro Glu Glu Leu Ile Gln Lys Gly
20 25 30

Lys Asp Ile Lys Gly Val Ser Glu Ile Val Gln Asn Gly Lys His Phe
35 40 45

Lys Phe Thr Ile Thr Ala Gly Ser Lys Val Ile Gln Asn Glu Phe Thr
50 55 60

Val Gly Glu Glu Cys Glu Leu Glu Thr Met Thr Gly Glu Lys Val Lys
65 70 75 80

Thr Val Val Gln Leu Glu Gly Asp Asn Lys Leu Val Thr Thr Phe Lys
85 90 95

Asn Ile Lys Ser Val Thr Glu Leu Asn Gly Asp Ile Ile Thr Asn Thr

100 105 110

Met Thr Leu Gly Asp Ile Val Phe Lys Arg Ile Ser Lys Arg Ile

115 120 125

<210> 13
<211> 1783
<212> DNA
<213> Homo sapiens

<220>
<221> CDS
<222> (246).. (1496)

<220>
<221> sig_peptide
<222> (246).. (731)

<220>
<221> mat_peptide
<222> (732).. (1496)

<400> 13

tcgagcccgc tttccaggga ccctacctga gggcccacag gtgaggcagc ctggcctagc 60

aggccccacg ccaccgcctc tgcctccagg ccgcccgctg ctgcggggcc accatgctcc 120

tgcccaggcc tggagactga cccgaccccg gcactacctc gaggctccgc ccccacctgc 180

tggaccccag ggtcccaccc tggcccagga ggtcagccag ggaatcatta acaagaggca 240

gtgac atg gcg cag aag gag ggt ggc cgg act gtg cca tgc tgc tcc 287

Met Ala Gln Lys Glu Gly Gly Arg Thr Val Pro Cys Cys Ser

-160 -155 -150

aga ccc aag gtg gca gct ctc act gcg ggg acc ctg cta ctt ctg 332

Arg Pro Lys Val Ala Ala Leu Thr Ala Gly Thr Leu Leu Leu Leu

-145 -140 -135

aca gcc atc ggg gcg gca tcc tgg gcc att gtg gct gtt ctc ctc 377

Thr Ala Ile Gly Ala Ala Ser Trp Ala Ile Val Ala Val Leu Leu

-130 -125 -120

agg agt gac cag gag ccg ctg tac cca gtg cag gtc agc tct gcg 422

Arg Ser Asp Gln Glu Pro Leu Tyr Pro Val Gln Val Ser Ser Ala

-115 -110 -105

gac gct cgg ctc atg gtc ttt gac aag acg gaa ggg acg tgg cgg ctg 470

Asp Ala Arg Leu Met Val Phe Asp Lys Thr Glu Gly Thr Trp Arg Leu

-100 -95 -90

ctg tgc tcc tcg cgc tcc aac gcc agg gta gcc gga ctc agc tgc gag 518

Leu Cys Ser Ser Arg Ser Asn Ala Arg Val Ala Gly Leu Ser Cys Glu

-85 -80 -75

gag atg ggc ttc ctc agg gca ctg acc cac tcc gag ctg gac gtg cga 566

Glu Met Gly Phe Leu Arg Ala Leu Thr His Ser Glu Leu Asp Val Arg

-70 -65 -60

acg gcg ggc gcc aat ggc acg tcg ggc ttc ttc tgt gtg gac gag ggg 614
Thr Ala Gly Ala Asn Gly Thr Ser Gly Phe Phe Cys Val Asp Glu Gly
-55 -50 -45 -40

agg ctg ccc cac acc cag agg ctg ctg gag gtc atc tcc gtg tgt gat 662
Arg Leu Pro His Thr Gln Arg Leu Leu Glu Val Ile Ser Val Cys Asp
-35 -30 -25

tgc ccc aga ggc cgt ttc ttg gcc gcc atc tgc caa gac tgt ggc cgc 710
Cys Pro Arg Gly Arg Phe Leu Ala Ala Ile Cys Gln Asp Cys Gly Arg
-20 -15 -10

agg aag ctg ccc gtg gac cgc atc gtg gga ggc cgg gac acc agc ttg 758
Arg Lys Leu Pro Val Asp Arg Ile Val Gly Gly Arg Asp Thr Ser Leu
-5 -1 1 5

ggc cgg tgg ccg tgg caa gtc agc ctt cgc tat gat gga gca cac ctc 806
Gly Arg Trp Pro Trp Gln Val Ser Leu Arg Tyr Asp Gly Ala His Leu
10 15 20 25

tgt ggg gga tcc ctg ctc tcc ggg gac tgg gtg ctg aca gcc gcc cac 854

Cys Gly Gly Ser Leu Leu Ser Gly Asp Trp Val Leu Thr Ala Ala His
30 35 40

tgc ttc ccg gag cgg aac cgg gtc ctg tcc cga tgg cga gtg ttt gcc 902
Cys Phe Pro Glu Arg Asn Arg Val Leu Ser Arg Trp Arg Val Phe Ala
45 50 55

ggt gcc gtg gcc cag gcc tct ccc cac ggt ctg cag ctg ggg gtg cag 950
Gly Ala Val Ala Gln Ala Ser Pro His Gly Leu Gln Leu Gly Val Gln
60 65 70

gct gtg gtc tac cac ggg ggc tat ctt ccc ttt cgg gac ccc aac agc 998
Ala Val Val Tyr His Gly Gly Tyr Leu Pro Phe Arg Asp Pro Asn Ser
75 80 85

gag gag aac agc aac gat att gcc ctg gtc cac ctc tcc agt ccc ctg 1046
Glu Glu Asn Ser Asn Asp Ile Ala Leu Val His Leu Ser Ser Pro Leu
90 95 100 105

ccc ctc aca gaa tac atc cag cct gtg tgc ctc cca gct gcc ggc cag 1094
Pro Leu Thr Glu Tyr Ile Gln Pro Val Cys Leu Pro Ala Ala Gly Gln

110 115 120

gcc ctg gtg gat ggc aag atc tgt acc gtg acg ggc tgg ggc aac acg 1142
Ala Leu Val Asp Gly Lys Ile Cys Thr Val Thr Gly Trp Gly Asn Thr
125 130 135

cag tac tat ggc caa cag gcc ggg gta ctc cag gag gct cga gtc ccc 1190
Gln Tyr Tyr Gly Gln Gln Ala Gly Val Leu Gln Glu Ala Arg Val Pro
140 145 150

ata atc agc aat gat gtc tgc aat ggc gct gac ttc tat gga aac cag 1238
Ile Ile Ser Asn Asp Val Cys Asn Gly Ala Asp Phe Tyr Gly Asn Gln
155 160 165

atc aag ccc aag atg ttc tgt gct ggc tac ccc gag ggt ggc att gat 1286
Ile Lys Pro Lys Met Phe Cys Ala Gly Tyr Pro Glu Gly Gly Ile Asp
170 175 180 185

gcc tgc cag ggc gac agc ggt ggt ccc ttt gtg tgt gag gac agc atc 1334
Ala Cys Gln Gly Asp Ser Gly Gly Pro Phe Val Cys Glu Asp Ser Ile
190 195 200

tct cgg acg cca cgt tgg cgg ctg tgt ggc att gtg agt tgg ggc act 1382

Ser Arg Thr Pro Arg Trp Arg Leu Cys Gly Ile Val Ser Trp Gly Thr

205 210 215

ggc tgt gcc ctg gcc cag aag cca ggc gtc tac acc aaa gtc agt gac 1430

Gly Cys Ala Leu Ala Gln Lys Pro Gly Val Tyr Thr Lys Val Ser Asp

220 225 230

ttc cgg gag tgg atc ttc cag gcc ata aag act cac tcc gaa gcc agc 1478

Phe Arg Glu Trp Ile Phe Gln Ala Ile Lys Thr His Ser Glu Ala Ser

235 240 245

ggc atg gtg acc cag ctc tgaccggtgg cttctcgctg cgcagcctcc 1526

Gly Met Val Thr Gln Leu

250 255

agggcccgag gtgatcccgg tggtgggatc cacgctgggc cgaggatggg acgtttttct 1586

tcttgggccc ggtccacagg tccaaggaca ccctccctcc agggtcctct cttccacagt 1646

ggcgggccca ctcagccccg agaccaccca acctcaccct cctgaccccc atgtaaatat 1706

tgttctgctg tctgggactc ctgtctaggt gcccctgatg atgggatgct ctttaaataa 1766

taaagatggt tttgatt 1783

<210> 14
<211> 417
<212> PRT
<213> Homo sapiens

<400> 14

Met Ala Gln Lys Glu Gly Gly Arg Thr Val Pro Cys Cys Ser Arg
-160 -155 -150

Pro Lys Val Ala Ala Leu Thr Ala Gly Thr Leu Leu Leu Leu Thr
-145 -140 -135

Ala Ile Gly Ala Ala Ser Trp Ala Ile Val Ala Val Leu Leu Arg

-130 -125 -120

Ser Asp Gln Glu Pro Leu Tyr Pro Val Gln Val Ser Ser Ala Asp

-115 -110 -105

Ala Arg Leu Met Val Phe Asp Lys Thr Glu Gly Thr Trp Arg Leu Leu

-100 -95 -90

Cys Ser Ser Arg Ser Asn Ala Arg Val Ala Gly Leu Ser Cys Glu Glu

-85 -80 -75

Met Gly Phe Leu Arg Ala Leu Thr His Ser Glu Leu Asp Val Arg Thr

-70 -65 -60 -55

Ala Gly Ala Asn Gly Thr Ser Gly Phe Phe Cys Val Asp Glu Gly Arg

-50 -45 -40

Leu Pro His Thr Gln Arg Leu Leu Glu Val Ile Ser Val Cys Asp Cys

-35 -30 -25

Pro Arg Gly Arg Phe Leu Ala Ala Ile Cys Gln Asp Cys Gly Arg Arg

-20 -15 -10

Lys Leu Pro Val Asp Arg Ile Val Gly Gly Arg Asp Thr Ser Leu Gly

-5 -1 1 5 10

Arg Trp Pro Trp Gln Val Ser Leu Arg Tyr Asp Gly Ala His Leu Cys

15 20 25

Gly Gly Ser Leu Leu Ser Gly Asp Trp Val Leu Thr Ala Ala His Cys

30 35 40

Phe Pro Glu Arg Asn Arg Val Leu Ser Arg Trp Arg Val Phe Ala Gly

45 50 55

Ala Val Ala Gln Ala Ser Pro His Gly Leu Gln Leu Gly Val Gln Ala

60 65 70

Val Val Tyr His Gly Gly Tyr Leu Pro Phe Arg Asp Pro Asn Ser Glu

75 80 85 90

Glu Asn Ser Asn Asp Ile Ala Leu Val His Leu Ser Ser Pro Leu Pro

95 100 105

Leu Thr Glu Tyr Ile Gln Pro Val Cys Leu Pro Ala Ala Gly Gln Ala

110 115 120

Leu Val Asp Gly Lys Ile Cys Thr Val Thr Gly Trp Gly Asn Thr Gln
125 130 135

Tyr Tyr Gly Gln Gln Ala Gly Val Leu Gln Glu Ala Arg Val Pro Ile
140 145 150

Ile Ser Asn Asp Val Cys Asn Gly Ala Asp Phe Tyr Gly Asn Gln Ile
155 160 165 170

Lys Pro Lys Met Phe Cys Ala Gly Tyr Pro Glu Gly Gly Ile Asp Ala
175 180 185

Cys Gln Gly Asp Ser Gly Gly Pro Phe Val Cys Glu Asp Ser Ile Ser
190 195 200

Arg Thr Pro Arg Trp Arg Leu Cys Gly Ile Val Ser Trp Gly Thr Gly

205 210 215

Cys Ala Leu Ala Gln Lys Pro Gly Val Tyr Thr Lys Val Ser Asp Phe

220 225 230

Arg Glu Trp Ile Phe Gln Ala Ile Lys Thr His Ser Glu Ala Ser Gly

235 240 245 250

Met Val Thr Gln Leu

255

<210> 15
<211> 1534
<212> DNA
<213> Homo sapiens

<220>
<221> CDS
<222> (26) .. (1324)

<220>
<221> sig_peptide
<222> (26).. (100)

<220>
<221> mat_peptide
<222> (101) .. (1324)

<400> 15

ggaattccct ggagcagagt tgaga atg gag aga atg tta cct ctc ctg gct 52
Met Glu Arg Met Leu Pro Leu Leu Ala
-25 -20

ctg ggg ctc ttg gcg gct ggg ttc tgc cct gct gtc ctc tgc cac cct 100
Leu Gly Leu Leu Ala Ala Gly Phe Cys Pro Ala Val Leu Cys His Pro

−15 −10 −5 −1

aac agc cca ctt gac gag gag aat ctg acc cag gag aac caa gac cga 148
Asn Ser Pro Leu Asp Glu Glu Asn Leu Thr Gln Glu Asn Gln Asp Arg
1 5 10 15

ggg aca cac gtg gac ctc gga tta gcc tcc gcc aac gtg gac ttc gct 196
Gly Thr His Val Asp Leu Gly Leu Ala Ser Ala Asn Val Asp Phe Ala
20 25 30

ttc agc ctg tac aag cag tta gtc ctg aag gcc ctt gat aag aat gtc 244
Phe Ser Leu Tyr Lys Gln Leu Val Leu Lys Ala Leu Asp Lys Asn Val
35 40 45

atc ttc tcc cca ctg agc atc tcc acc gcc ttg gcc ttc ctg tct ctg 292
Ile Phe Ser Pro Leu Ser Ile Ser Thr Ala Leu Ala Phe Leu Ser Leu
50 55 60

ggg gcc cat aat acc acc ctg aca gag att ctc aag gcc tcg agt tca 340
Gly Ala His Asn Thr Thr Leu Thr Glu Ile Leu Lys Ala Ser Ser Ser
65 70 75 80

```
cct cac gga gac tta ctg agg cag aaa ttc act cag agc ttc cag cac        388
Pro His Gly Asp Leu Leu Arg Gln Lys Phe Thr Gln Ser Phe Gln His
                85                  90                  95

ctc cgc gca ccc tca atc agt tcc agc gat gag ctg cag ctg agt atg        436
Leu Arg Ala Pro Ser Ile Ser Ser Ser Asp Glu Leu Gln Leu Ser Met
            100                 105                 110

gga aat gcc atg ttt gtc aaa gag caa ctc agt ctg ctg gac agg ttc        484
Gly Asn Ala Met Phe Val Lys Glu Gln Leu Ser Leu Leu Asp Arg Phe
        115                 120                 125

acg gag gat gcc aag agg ctg tat ggc tcc gag gcc ttt gcc act gac        532
Thr Glu Asp Ala Lys Arg Leu Tyr Gly Ser Glu Ala Phe Ala Thr Asp
    130                 135                 140

ttt cag gac tca gct gca gct aag aag ctc atc aac gac tac gtg aag        580
Phe Gln Asp Ser Ala Ala Ala Lys Lys Leu Ile Asn Asp Tyr Val Lys
145                 150                 155                 160
```

aat gga act agg ggg aaa atc aca gat ctg atc aag gac ccc gac tcg 628

Asn Gly Thr Arg Gly Lys Ile Thr Asp Leu Ile Lys Asp Pro Asp Ser

165 170 175

cag aca atg atg gtc ctg gtg aat tac atc ttc ttt aaa gcc aaa tgg 676

Gln Thr Met Met Val Leu Val Asn Tyr Ile Phe Phe Lys Ala Lys Trp

180 185 190

gag atg ccc ttt gac ccc caa gat act cat cag tca agg ttc tac ttg 724

Glu Met Pro Phe Asp Pro Gln Asp Thr His Gln Ser Arg Phe Tyr Leu

195 200 205

agc aag aaa aag tgg gta atg gtg ccc atg atg agt ttg cat cac ctg 772

Ser Lys Lys Lys Trp Val Met Val Pro Met Met Ser Leu His His Leu

210 215 220

act ata cct tac ttc cgg gac gag gag ctg tcc tgc acc gtg gtg gag 820

Thr Ile Pro Tyr Phe Arg Asp Glu Glu Leu Ser Cys Thr Val Val Glu

225 230 235 240

ctg aag tac aca ggc aat gcc agc gca ctc ttc atc ctc cct gat caa 868

Leu Lys Tyr Thr Gly Asn Ala Ser Ala Leu Phe Ile Leu Pro Asp Gln
245 250 255

gac aag atg gag gaa gtg gaa gcc atg ctg ctc cca gag acc ctg aag 916
Asp Lys Met Glu Glu Val Glu Ala Met Leu Leu Pro Glu Thr Leu Lys
260 265 270

cgg tgg aga gac tct ctg gag ttc aga gag ata ggt gag ctc tac ctg 964
Arg Trp Arg Asp Ser Leu Glu Phe Arg Glu Ile Gly Glu Leu Tyr Leu
275 280 285

cca aag ttt tcc atc tcg agg gac tat aac ctg aac gac ata ctt ctc 1012
Pro Lys Phe Ser Ile Ser Arg Asp Tyr Asn Leu Asn Asp Ile Leu Leu
290 295 300

cag ctg ggc att gag gaa gcc ttc acc agc aag gct gac ctg tca ggg 1060
Gln Leu Gly Ile Glu Glu Ala Phe Thr Ser Lys Ala Asp Leu Ser Gly
305 310 315 320

atc aca ggg gcc agg aac cta gca gtc tcc cag gtg gtc cat aag gtc 1108
Ile Thr Gly Ala Arg Asn Leu Ala Val Ser Gln Val Val His Lys Val

325 330 335

gtg tct gat gta ttt gag gag ggc aca gaa gca tct gct gcc aca gca 1156

Val Ser Asp Val Phe Glu Glu Gly Thr Glu Ala Ser Ala Ala Thr Ala

340 345 350

gtc aaa atc acc ctc ctt tct gca tta gtg gag aca agg acc att gtg 1204

Val Lys Ile Thr Leu Leu Ser Ala Leu Val Glu Thr Arg Thr Ile Val

355 360 365

cgt ttc aac agg ccc ttc ctg atg atc att gtc cct aca gac acc cag 1252

Arg Phe Asn Arg Pro Phe Leu Met Ile Ile Val Pro Thr Asp Thr Gln

370 375 380

aac atc ttc ttc atg agc aaa gtc acc aat ccc agc aag cct aga gct 1300

Asn Ile Phe Phe Met Ser Lys Val Thr Asn Pro Ser Lys Pro Arg Ala

385 390 395 400

tgc atc aag cag tgg ggc tct cag taaggaactt ggaatgcaag ctggatgcct 1354

Cys Ile Lys Gln Trp Gly Ser Gln

405

gggtctctgg gcacagctgg cccctgtgca ccgtagtggc catggcatgt gtggccctgt 1414

ctgcttatcc ttggaaggtg acagcgattc cctgtgaagc tctcacacgc acaggggccc 1474

atggactctt cagtctggag ggtcctggcc tcctgacagc aataaataat ttcgttggcc 1534

<210> 16
<211> 433
<212> PRT
<213> Homo sapiens

<400> 16

Met Glu Arg Met Leu Pro Leu Leu Ala Leu Gly Leu Leu Ala Ala Gly
-25 -20 -15 -10

Phe Cys Pro Ala Val Leu Cys His Pro Asn Ser Pro Leu Asp Glu Glu
-5 -1 1 5

Asn Leu Thr Gln Glu Asn Gln Asp Arg Gly Thr His Val Asp Leu Gly

10 15 20

Leu Ala Ser Ala Asn Val Asp Phe Ala Phe Ser Leu Tyr Lys Gln Leu

25 30 35

Val Leu Lys Ala Leu Asp Lys Asn Val Ile Phe Ser Pro Leu Ser Ile

40 45 50 55

Ser Thr Ala Leu Ala Phe Leu Ser Leu Gly Ala His Asn Thr Thr Leu

60 65 70

Thr Glu Ile Leu Lys Ala Ser Ser Ser Pro His Gly Asp Leu Leu Arg

75 80 85

Gln Lys Phe Thr Gln Ser Phe Gln His Leu Arg Ala Pro Ser Ile Ser
90 95 100

Ser Ser Asp Glu Leu Gln Leu Ser Met Gly Asn Ala Met Phe Val Lys
105 110 115

Glu Gln Leu Ser Leu Leu Asp Arg Phe Thr Glu Asp Ala Lys Arg Leu
120 125 130 135

Tyr Gly Ser Glu Ala Phe Ala Thr Asp Phe Gln Asp Ser Ala Ala Ala
140 145 150

Lys Lys Leu Ile Asn Asp Tyr Val Lys Asn Gly Thr Arg Gly Lys Ile
155 160 165

Thr Asp Leu Ile Lys Asp Pro Asp Ser Gln Thr Met Met Val Leu Val

170 175 180

Asn Tyr Ile Phe Phe Lys Ala Lys Trp Glu Met Pro Phe Asp Pro Gln

185 190 195

Asp Thr His Gln Ser Arg Phe Tyr Leu Ser Lys Lys Lys Trp Val Met

200 205 210 215

Val Pro Met Met Ser Leu His His Leu Thr Ile Pro Tyr Phe Arg Asp

220 225 230

Glu Glu Leu Ser Cys Thr Val Val Glu Leu Lys Tyr Thr Gly Asn Ala

235 240 245

Ser Ala Leu Phe Ile Leu Pro Asp Gln Asp Lys Met Glu Glu Val Glu

250 255 260

Ala Met Leu Leu Pro Glu Thr Leu Lys Arg Trp Arg Asp Ser Leu Glu

265 270 275

Phe Arg Glu Ile Gly Glu Leu Tyr Leu Pro Lys Phe Ser Ile Ser Arg

280 285 290 295

Asp Tyr Asn Leu Asn Asp Ile Leu Leu Gln Leu Gly Ile Glu Glu Ala

300 305 310

Phe Thr Ser Lys Ala Asp Leu Ser Gly Ile Thr Gly Ala Arg Asn Leu

315 320 325

Ala Val Ser Gln Val Val His Lys Val Val Ser Asp Val Phe Glu Glu

330 335 340

Gly Thr Glu Ala Ser Ala Ala Thr Ala Val Lys Ile Thr Leu Leu Ser

345 350 355

Ala Leu Val Glu Thr Arg Thr Ile Val Arg Phe Asn Arg Pro Phe Leu

360 365 370 375

Met Ile Ile Val Pro Thr Asp Thr Gln Asn Ile Phe Phe Met Ser Lys

380 385 390

Val Thr Asn Pro Ser Lys Pro Arg Ala Cys Ile Lys Gln Trp Gly Ser

395 400 405

Gln

<210> 17
<211> 2008
<212> DNA
<213> Homo sapiens

<220>
<221> CDS
<222> (222).. (857)

<400> 17

tgggacactg ctcagggaag agcctgctac ggtggactgt gagactcagt gcactgtcct 60

cctcccagcg accccacgct ggaccccctg ccggaccctc caccccttcgg cccccaagct 120

tcccaggggc ttcctttgga ctggactgtc cctgctcatc cattctcctg ccaccccccag 180

acctcctcag ctccaggttg ccacctcctc tcgccagagt g atg agg tcc cgg ctt 236

Met Arg Ser Arg Leu

1 5

ctg ctc tcc gtg gcc cat ctg ccc aca att cgg gag acc acg gag gag 284

Leu Leu Ser Val Ala His Leu Pro Thr Ile Arg Glu Thr Thr Glu Glu

10 15 20

atg ctg ctt ggg ggt cct gga cag gag ccc cca ccc tct cct agc ctg 332

Met Leu Leu Gly Gly Pro Gly Gln Glu Pro Pro Pro Ser Pro Ser Leu

25 30 35

gat gac tac gtg agg tct ata tct cga ctg gca cag ccc acc tct gtg 380

Asp Asp Tyr Val Arg Ser Ile Ser Arg Leu Ala Gln Pro Thr Ser Val

40 45 50

ctg gac aag gcc acg gcc cag ggc caa ccc agg cca ccc cac agg cca 428

Leu Asp Lys Ala Thr Ala Gln Gly Gln Pro Arg Pro Pro His Arg Pro

55 60 65

gcc cag gcc tgc cgg aag ggc cgc cct gct gtg tcc ctg cga gac atc 476

Ala Gln Ala Cys Arg Lys Gly Arg Pro Ala Val Ser Leu Arg Asp Ile

70 75 80 85

acc gca cgt ttc agt ggc cag cag ccc aca ctg ccc atg gct gat act 524

Thr Ala Arg Phe Ser Gly Gln Gln Pro Thr Leu Pro Met Ala Asp Thr

90 95 100

gtg gac ccc ctg gac tgg ctt ttt ggg gag tcc cag gaa aag cag cca 572

Val Asp Pro Leu Asp Trp Leu Phe Gly Glu Ser Gln Glu Lys Gln Pro

105 110 115

agc cag agg gac ctg cca agg agg act ggc ccc tct gct ggc ctc tgg 620

Ser Gln Arg Asp Leu Pro Arg Arg Thr Gly Pro Ser Ala Gly Leu Trp

120 125 130

ggt cca cat aga cag atg gac agc agc aag ccc acg ggg gcc ccc aga 668

Gly Pro His Arg Gln Met Asp Ser Ser Lys Pro Thr Gly Ala Pro Arg

135 140 145

ggg agg ctc tgt gaa gcc agg atg cct ggg cat tcc ctg gca aga cca 716

Gly Arg Leu Cys Glu Ala Arg Met Pro Gly His Ser Leu Ala Arg Pro

150 155 160 165

ccg cag gat ggg cag cag agc tct gac cta aga agc tgg act ttt ggg 764

Pro Gln Asp Gly Gln Gln Ser Ser Asp Leu Arg Ser Trp Thr Phe Gly

170 175 180

cag tct gcc caa gcc atg gcc tcc cgc cac cgc ccc cgc ccc agc agt 812

Gln Ser Ala Gln Ala Met Ala Ser Arg His Arg Pro Arg Pro Ser Ser

185 190 195

gtc ctc aga aca ctc tac tcg cac ctc ccg gtg atc cat gaa ctc 857

Val Leu Arg Thr Leu Tyr Ser His Leu Pro Val Ile His Glu Leu

200 205 210

tgacccctcc ccagtaaagg cttctgtaga gagcatgctg ggtctgcatc tcctctcgtc 917

tcctccatgg tggtcactgc ccctggcagg tctctgaaag ggaaatgctt ttctgcggag 977

gcccctgctt gggcagttca cagtgagacc gaccccctct gaatatgata acagcctgtt 1037

tcacatgagg agatgttacc aatcccgttc gctctgaccc ttgctggctg atcaccttga 1097

gcaacttact taacatctgt gttcctcagt ttctcatggg taatataggg ataattactg 1157

gcacctgcct cccaggccat tctgacgtgt aaccgcatat aggagcccac tggctgagta 1217

gctaccatca tcgctggtgg ggaaactggt ggtaggggtg tgagggtagt gggggtgtca 1277

gcccccccagg tgtttcagaa caaggcctcg ggcactccca agtctgcctc ttggctccca 1337

ccctcaaagc ccatgttctg cgaggcccaa gagaacacat ggagtcttag caaatgcact 1397

aatgtattcc gggggactgt cacctggcac cactggggca ctctgctggc tacaactcat 1457

acgtcctgtg gtggcattgg gagagttccc ccatgatgag ggccaagata gaatctgtac 1517

cactcagtgc taccatcccc acccctacac cacttccaca caggggcctc atggcatggt 1577

cagggtccca gctgtaggtg agagcagggc actgtccagc tgtccactgg ggaagtcaag 1637

atgtcctaag gcccaggtca gggcatctgg agtctgaagg accctagttc ctagaggcat 1697

ctggcagcaa gaaggtgagg catcagggaa cgggaatcag gctgggactg atcagaggtg 1757

aagggacaga gagaggagag gaggaagatt gagctggggg caacagccaa gctcacctgg 1817

gcaggtctct gccacctcct tgctctgtga gctgtcagtc taggttattc tcttttttg 1877

tggctatttt taattgcttt ggatttgtta aatgttttct gtcttctgtt aagtgtgttt 1937

tctctggaga tagaatgtaa accatattaa aaggaaaaag tttcagacaa gcaaaaaaaa 1997

aaaaaaaaaa a 2008

<210> 18
<211> 212
<212> PRT
<213> Homo sapiens

<400> 18

Met Arg Ser Arg Leu Leu Leu Ser Val Ala His Leu Pro Thr Ile Arg
1 5 10 15

Glu Thr Thr Glu Glu Met Leu Leu Gly Gly Pro Gly Gln Glu Pro Pro

20 25 30

Pro Ser Pro Ser Leu Asp Asp Tyr Val Arg Ser Ile Ser Arg Leu Ala

35 40 45

Gln Pro Thr Ser Val Leu Asp Lys Ala Thr Ala Gln Gly Gln Pro Arg

50 55 60

Pro Pro His Arg Pro Ala Gln Ala Cys Arg Lys Gly Arg Pro Ala Val

65 70 75 80

Ser Leu Arg Asp Ile Thr Ala Arg Phe Ser Gly Gln Gln Pro Thr Leu

85 90 95

Pro Met Ala Asp Thr Val Asp Pro Leu Asp Trp Leu Phe Gly Glu Ser

100 105 110

Gln Glu Lys Gln Pro Ser Gln Arg Asp Leu Pro Arg Arg Thr Gly Pro

115 120 125

Ser Ala Gly Leu Trp Gly Pro His Arg Gln Met Asp Ser Ser Lys Pro

130 135 140

Thr Gly Ala Pro Arg Gly Arg Leu Cys Glu Ala Arg Met Pro Gly His

145 150 155 160

Ser Leu Ala Arg Pro Pro Gln Asp Gly Gln Gln Ser Ser Asp Leu Arg

165 170 175

Ser Trp Thr Phe Gly Gln Ser Ala Gln Ala Met Ala Ser Arg His Arg

180 185 190

Pro Arg Pro Ser Ser Val Leu Arg Thr Leu Tyr Ser His Leu Pro Val

195 200 205

Ile His Glu Leu

210

<210> 19
<211> 1649
<212> DNA
<213> Homo sapiens

<220>
<221> CDS
<222> (367).. (1068)

<400> 19

agcgagcggg gccagcgctg cagaaggcgg cggctggctc tccgggacgg tcacatcccg 60

ctgcaggggc gggcggaggc cgccgcactg cctcccgcac cggggaccca ggccagcgtc 120

cgggcaacgc cccctgctcc cggacagact ccgtggcccg ctcgagccct gggggctccg 180

cagacccgcg cccgctccgc ccgcagctcg gccccgcgct gcccgcgtcg ccgggcccgc 240

gccgggatgg ggtaggggca gcgccaccga gtcgggcgat gggccgccct ctgggcaccg 300

agcagccccc cgaggcctga ccaaccgcga ggaccggcgg aggagccccg cctggatgtc 360

aagcgg atg cca agc gga tgc cac agt tcc ccc ccc agc gga ctc cgt 408

Met Pro Ser Gly Cys His Ser Ser Pro Pro Ser Gly Leu Arg

1 5 10

ggg gac atg gct tcg ctg gtg ccc ctt tcc cca tat cta agc ccc acg 456

Gly Asp Met Ala Ser Leu Val Pro Leu Ser Pro Tyr Leu Ser Pro Thr

15 20 25 30

gtc ctc ctg ctg gtc agc tgt gac ctg ggc ttc gtg cga gca gac cgg 504

Val Leu Leu Leu Val Ser Cys Asp Leu Gly Phe Val Arg Ala Asp Arg

35 40 45

cct ccc tct cct gtg aat gtg acg gtc act cac ctc aga gcc aac tcg 552

Pro Pro Ser Pro Val Asn Val Thr Val Thr His Leu Arg Ala Asn Ser

50 55 60

gcc act gtg tcc tgg gac gtc cca gaa ggc aac atc gtc att ggc tac 600

Ala Thr Val Ser Trp Asp Val Pro Glu Gly Asn Ile Val Ile Gly Tyr

65 70 75

tcc att tcc cag caa cgg cag aat ggc ccc ggg cag cgt gtg att cgg 648

Ser Ile Ser Gln Gln Arg Gln Asn Gly Pro Gly Gln Arg Val Ile Arg

80 85 90

gag gtg aac acc acc acc cgg gcc tgt gcc ctc tgg ggc ctg gct gaa 696

Glu Val Asn Thr Thr Thr Arg Ala Cys Ala Leu Trp Gly Leu Ala Glu

95 100 105 110

gac agt gac tac aca gtg cag gtc agg agc atc ggc ctt cgg gga gag 744

Asp Ser Asp Tyr Thr Val Gln Val Arg Ser Ile Gly Leu Arg Gly Glu

115 120 125

agt ccc cca ggg ccc cgg gtg cac ttc cga act ctc aag ggt tct gac 792

Ser Pro Pro Gly Pro Arg Val His Phe Arg Thr Leu Lys Gly Ser Asp

130 135 140

cgg cta cct tca aac agt tca agc cca ggt gac atc aca gtg gaa ggt 840

Arg Leu Pro Ser Asn Ser Ser Ser Pro Gly Asp Ile Thr Val Glu Gly

145 150 155

ctg gat gga gag cgg cca ctg cag act ggg gaa gtg gtc atc att gtg 888

Leu Asp Gly Glu Arg Pro Leu Gln Thr Gly Glu Val Val Ile Ile Val

160 165 170

gtg gtg ttg ctc atg tgg gct gct gta att ggg ctg ttc tgc cgt cag 936

Val Val Leu Leu Met Trp Ala Ala Val Ile Gly Leu Phe Cys Arg Gln

175 180 185 190

tat gac atc atc aag gac aat gac tcc aac aac aat ccc aag gag aag 984

Tyr Asp Ile Ile Lys Asp Asn Asp Ser Asn Asn Asn Pro Lys Glu Lys

195 200 205

gga aag ggg ccg gaa cag agt cct cag gga agg cca gtg ggg aca aga 1032
Gly Lys Gly Pro Glu Gln Ser Pro Gln Gly Arg Pro Val Gly Thr Arg
210 215 220

cag aaa aag tca cca tct atc aac acc atc gac gtt tgagtgaaga 1078
Gln Lys Lys Ser Pro Ser Ile Asn Thr Ile Asp Val
225 230

aacacaccca gaagagagat gcactaacaa ctggggatag ggatggggtc agggggagcc 1138

caagatggtg atctgcccga gactcccaga gggtaatgcc actcccacaa tctcaggcct 1198

ggtacccatc ctctttccac tgtgagcaga gccagaaggt aggtctgttc agagtctgtg 1258

cccctggacc tggggagtgg atatcagatg ggatatctcc ttccattccc cggtccaggg 1318

gagagtcact agttgtaccc tactccatta ggtcccaaat gggggcccca tttcacctgt 1378

atcaggactc tgagcatccc cagctgcccc acatcttgcc tctggccctc agagaggggt 1438

gtttctgtgg gtactcctct taccccagca aataaaagga attgtctgac cctagaggca 1498

gatgctgcac tgcactactc caatgtcttc catggagcct caggtgctcc ccctctcacc 1558

tggcagcccc ttcagctgct agtgatatca cttgttggac atttttccaa taaaggttct 1618

tggacaaact ggaaaaaaaa aaaaaaaaaa a 1649

<210> 20
<211> 234
<212> PRT
<213> Homo sapiens

<400> 20

Met Pro Ser Gly Cys His Ser Ser Pro Pro Ser Gly Leu Arg Gly Asp
1 5 10 15

Met Ala Ser Leu Val Pro Leu Ser Pro Tyr Leu Ser Pro Thr Val Leu

20 25 30

Leu Leu Val Ser Cys Asp Leu Gly Phe Val Arg Ala Asp Arg Pro Pro

35 40 45

Ser Pro Val Asn Val Thr Val Thr His Leu Arg Ala Asn Ser Ala Thr

50 55 60

Val Ser Trp Asp Val Pro Glu Gly Asn Ile Val Ile Gly Tyr Ser Ile

65 70 75 80

Ser Gln Gln Arg Gln Asn Gly Pro Gly Gln Arg Val Ile Arg Glu Val

85 90 95

Asn Thr Thr Thr Arg Ala Cys Ala Leu Trp Gly Leu Ala Glu Asp Ser

100 105 110

Asp Tyr Thr Val Gln Val Arg Ser Ile Gly Leu Arg Gly Glu Ser Pro
115 120 125

Pro Gly Pro Arg Val His Phe Arg Thr Leu Lys Gly Ser Asp Arg Leu
130 135 140

Pro Ser Asn Ser Ser Ser Pro Gly Asp Ile Thr Val Glu Gly Leu Asp
145 150 155 160

Gly Glu Arg Pro Leu Gln Thr Gly Glu Val Val Ile Ile Val Val Val
165 170 175

Leu Leu Met Trp Ala Ala Val Ile Gly Leu Phe Cys Arg Gln Tyr Asp
180 185 190

Ile Ile Lys Asp Asn Asp Ser Asn Asn Asn Pro Lys Glu Lys Gly Lys

195 200 205

Gly Pro Glu Gln Ser Pro Gln Gly Arg Pro Val Gly Thr Arg Gln Lys

210 215 220

Lys Ser Pro Ser Ile Asn Thr Ile Asp Val

225 230

<210> 21
<211> 3915
<212> DNA
<213> Homo sapiens

<220>
<221> CDS
<222> (243) .. (1730)

<400> 21

gtggggtggg gtggggctgg gggcttgtcg ccctttcagg ctccaccctt tgcggagatt 60

ataaatagtc atgatcccag cgagacccag agatgcctgt aatggtgaga ctttggatcc 120

ttcctgagga cgtggagaaa actttctgct gagaaggaca ttttgaaggt tttgttggct 180

gaaaaagctg tttctggaat cacccctaga tctttcttga agacttgaat tagattacag 240

cg atg ggg aca cag aag gtc acc cca gct ctg ata ttt gcc atc aca 287
Met Gly Thr Gln Lys Val Thr Pro Ala Leu Ile Phe Ala Ile Thr
1 5 10 15

gtt gct aca atc ggc tct ttc caa ttt ggc tac aac act ggg gtc atc 335
Val Ala Thr Ile Gly Ser Phe Gln Phe Gly Tyr Asn Thr Gly Val Ile
20 25 30

aat gct cct gag aag atc ata aag gaa ttt atc aat aaa act ttg acg 383
Asn Ala Pro Glu Lys Ile Ile Lys Glu Phe Ile Asn Lys Thr Leu Thr

35 40 45

gac aag gga aat gcc cca ccc tct gag gtg ctg ctc acg tct ctc tgg 431
Asp Lys Gly Asn Ala Pro Pro Ser Glu Val Leu Leu Thr Ser Leu Trp
50 55 60

tcc ttg tct gtg gcc ata ttt tcc gtc ggg ggt atg atc ggc tcc ttt 479
Ser Leu Ser Val Ala Ile Phe Ser Val Gly Gly Met Ile Gly Ser Phe
65 70 75

tcc gtc gga ctc ttc gtc aac cgc ttt ggc agg cgc aat tca atg ctg 527
Ser Val Gly Leu Phe Val Asn Arg Phe Gly Arg Arg Asn Ser Met Leu
80 85 90 95

att gtc aac ctg ttg gct gtc act ggt ggc tgc ttt atg gga ctg tgt 575
Ile Val Asn Leu Leu Ala Val Thr Gly Gly Cys Phe Met Gly Leu Cys
100 105 110

aaa gta gct aag tcg gtt gaa atg ctg atc ctg ggt cgc ttg gtt att 623
Lys Val Ala Lys Ser Val Glu Met Leu Ile Leu Gly Arg Leu Val Ile
115 120 125

ggc ctc ttc tgc gga ctc tgc aca ggt ttt gtg ccc atg tac att gga 671

Gly Leu Phe Cys Gly Leu Cys Thr Gly Phe Val Pro Met Tyr Ile Gly

130 135 140

gag atc tcg cct act gcc ctg cgg ggt gcc ttt ggc act ctc aac cag 719

Glu Ile Ser Pro Thr Ala Leu Arg Gly Ala Phe Gly Thr Leu Asn Gln

145 150 155

ctg ggc atc gtt gtt gga att ctg gtg gcc cag atc ttt ggt ctg gaa 767

Leu Gly Ile Val Val Gly Ile Leu Val Ala Gln Ile Phe Gly Leu Glu

160 165 170 175

ttc atc ctt ggg tct gaa gag cta tgg ccg ctg cta ctg ggt ttt acc 815

Phe Ile Leu Gly Ser Glu Glu Leu Trp Pro Leu Leu Leu Gly Phe Thr

180 185 190

atc ctt cct gct atc cta caa agt gca gcc ctt cca ttt tgc cct gaa 863

Ile Leu Pro Ala Ile Leu Gln Ser Ala Ala Leu Pro Phe Cys Pro Glu

195 200 205

agt ccc aga ttt ttg ctc att aac aga aaa gaa gag gag aat gct aag 911
Ser Pro Arg Phe Leu Leu Ile Asn Arg Lys Glu Glu Glu Asn Ala Lys
210 215 220

cag atc ctc cag cgg ttg tgg ggc acc cag gat gta tcc caa gac atc 959
Gln Ile Leu Gln Arg Leu Trp Gly Thr Gln Asp Val Ser Gln Asp Ile
225 230 235

cag gag atg aaa gat gag agt gca agg atg tca caa gaa aag caa gtc 1007
Gln Glu Met Lys Asp Glu Ser Ala Arg Met Ser Gln Glu Lys Gln Val
240 245 250 255

acc gtg cta gag ctc ttt aga gtg tcc agc tac cga cag ccc atc atc 1055
Thr Val Leu Glu Leu Phe Arg Val Ser Ser Tyr Arg Gln Pro Ile Ile
260 265 270

att tcc att gtg ctc cag ctc tct cag cag ctc tct ggg atc aat gct 1103
Ile Ser Ile Val Leu Gln Leu Ser Gln Gln Leu Ser Gly Ile Asn Ala
275 280 285

gtg ttc tat tac tca aca gga atc ttc aag gat gca ggt gtt caa gag 1151

Val Phe Tyr Tyr Ser Thr Gly Ile Phe Lys Asp Ala Gly Val Gln Glu

290 295 300

ccc atc tat gcc acc atc ggc gcg ggt gtg gtt aat act atc ttc act 1199

Pro Ile Tyr Ala Thr Ile Gly Ala Gly Val Val Asn Thr Ile Phe Thr

305 310 315

gta gtt tct cta ttt ctg gtg gaa agg gca gga aga agg act ctg cat 1247

Val Val Ser Leu Phe Leu Val Glu Arg Ala Gly Arg Arg Thr Leu His

320 325 330 335

atg ata ggc ctt gga ggg atg gct ttt tgt tcc acg ctc atg act gtt 1295

Met Ile Gly Leu Gly Gly Met Ala Phe Cys Ser Thr Leu Met Thr Val

340 345 350

tct ttg tta tta aag gat aac tat aat ggg atg agc ttt gtc tgt att 1343

Ser Leu Leu Leu Lys Asp Asn Tyr Asn Gly Met Ser Phe Val Cys Ile

355 360 365

ggg gct atc ttg gtc ttt gta gcc ttc ttt gaa att gga cca ggc ccc 1391

Gly Ala Ile Leu Val Phe Val Ala Phe Phe Glu Ile Gly Pro Gly Pro

370 375 380

att ccc tgg ttt att gtg gcc gaa ctc ttc agc cag ggc ccc cgc cca 1439
Ile Pro Trp Phe Ile Val Ala Glu Leu Phe Ser Gln Gly Pro Arg Pro
385 390 395

gct gcg atg gca gtg gcc ggc tgc tcc aac tgg acc tcc aac ttc cta 1487
Ala Ala Met Ala Val Ala Gly Cys Ser Asn Trp Thr Ser Asn Phe Leu
400 405 410 415

gtc gga ttg ctc ttc ccc tcc gct gct cac tat tta gga gcc tac gtt 1535
Val Gly Leu Leu Phe Pro Ser Ala Ala His Tyr Leu Gly Ala Tyr Val
420 425 430

ttt att atc ttc acc ggc ttc ctc att acc ttc ttg gct ttt acc ttc 1583
Phe Ile Ile Phe Thr Gly Phe Leu Ile Thr Phe Leu Ala Phe Thr Phe
435 440 445

ttc aaa gtc cct gag acc cgt ggc agg act ttt gag gat atc aca cgg 1631
Phe Lys Val Pro Glu Thr Arg Gly Arg Thr Phe Glu Asp Ile Thr Arg
450 455 460

gcc ttt gaa ggg cag gca cac ggt gca gat aga tct gga aag gac ggc 1679

Ala Phe Glu Gly Gln Ala His Gly Ala Asp Arg Ser Gly Lys Asp Gly

465 470 475

gtc atg gag atg aac agc atc gag cct gct aag gag acc acc acc aat 1727

Val Met Glu Met Asn Ser Ile Glu Pro Ala Lys Glu Thr Thr Thr Asn

480 485 490 495

gtc taagtcgtgc ctccttccac ctccctcccg gcatgggaaa gccacctctc 1780

Val

cctcaacaag ggagagacct catcaggatg aacccaggac gcttctgaat gctgctactt 1840

aattcctttc tcatcccacg cactccatga gcaccccaag gctgcggttt gttggatctt 1900

caatggcttt ttaaatttta tttcctggac atcctcttct gcttaggaga gaccgagtga 1960

acctaccttc atttcaggag ggattggccg cttggcacat gacaactttg ccagcttttc 2020

ctcccttggg ttctgatatt gccgcactag gggatatagg agaggaaaag taaggtgcag 2080

ttcccccaac ctcagactta ccaggaagca gatacatatg agtgtggaag ccggagggtg 2140

tttatgtaag agcaccttcc tcacttccat acagctctac gtggcaaatt aacttgagtt 2200

ttatttattt tatcctctgg tttaattaca taattttttt tttttactt taagtttcag 2260

gatacatgtg ccgaatgtgc aggtttgtta cataggtata tatatgccat gatggaaata 2320

tttatttttt taagcgtaat tttgccaaat aataaaaaca gaaggaaatt gagattagag 2380

ggaggtgttt aaagagaggt tatagagtag aagatttgat gctggagagg ttaaggtgca 2440

ataagaattt agggagaaat gttgttcatt attggagggt aaatgatgtg gtgcctgagg 2500

tctgtacgtt acctcttaac aatttctgtc cttcagatgg aaactcttta acttctcgta 2560

aaagtcatat acctatataa taaagctact gatttccttg gagctttttt ctttaagata 2620

atagtttaca tgtagtagta cttgaaatct aggattatta actaatatgg gcattgtagt 2680

taatgatggt tgatgggttc taattttgga tggagtccag ggaagagaaa gtgatttcta 2740

gaaagcctgt tcccctcact ggatgaaata actccttctt gtagtagtct cattactttt 2800

gaagtaatcc cgccacctat ctcgtgggag agccatccaa ataagaaacc taaaataatt 2860

ggttcttggt agagattcat tatttttcca ctttgttctt taggagattt taggtgttga 2920

ttttctgttg tattttaact catacctttta aaggaattcc ccaaagaatg tttatagcaa 2980

acttggaatt tgtaacctca gctctgggag aggatttttt tctgagcgat tattatctaa 3040

agtgtgttgt tgctttaggc tcacggcacg cttgcgtatg tctgttacca tgtcactgtg 3100

gtcctatgcc gaatgccctc aggggacttg aatctttcca ataaaccagg tttagacagt 3160

atgagtcaat gtgcagtgta gcccacactt gagaggatga atgtatgtgc actgtcactt 3220

tgctctgggt ggaagtacgt tattgttgac ttatttctc tgtgtttgtt cctacagccc 3280

cttttcata tgttgctcag tctccctttc ccttcttggt gcttacacat ctcagaccct 3340

ttagccaaac ccttgtcagt gacagtattt tggttcttag ttctcactgt tccctctgct 3400

cctggagcct ttgaataaaa atgcacgtag ctgaggccgg atgcggtggc tcacgcctgt 3460

aatcccagca ctttgggagg cctaggcggg cggtcagggg ttcgagacca gtctggccaa 3520

catcgtgaaa ccctgtctct actaaaaatg caaaaattag ccgggcgtgg tggcgggcgc 3580

ctgtaatccc agctacttgg gaagctgagg cgggagaatc atgtgaaccc gggacgcagg 3640

ggttgcagtg agcggagatc gcatcattgc actctagcct gggccacagg gcgagactcc 3700

gtctcaaaaa aaaaaaaatg cacatagcta tcgagtgtgc tttagcttga aaaggtgacc 3760

ttgcaacttc atgtcaactt tctggctcct caaacagtag gttggcagta aggcagggtc 3820

ccatttctca ctgagaagat tgtgaatatt tccatatgga ttttctattg ttactctggt 3880

tctttgtttt aaaataaaaa ttctgaatgt acacg 3915

<210> 22
<211> 496
<212> PRT
<213> Homo sapiens

<400> 22

Met Gly Thr Gln Lys Val Thr Pro Ala Leu Ile Phe Ala Ile Thr Val
1 5 10 15

Ala Thr Ile Gly Ser Phe Gln Phe Gly Tyr Asn Thr Gly Val Ile Asn
20 25 30

Ala Pro Glu Lys Ile Ile Lys Glu Phe Ile Asn Lys Thr Leu Thr Asp
35 40 45

Lys Gly Asn Ala Pro Pro Ser Glu Val Leu Leu Thr Ser Leu Trp Ser

50 55 60

Leu Ser Val Ala Ile Phe Ser Val Gly Gly Met Ile Gly Ser Phe Ser

65 70 75 80

Val Gly Leu Phe Val Asn Arg Phe Gly Arg Arg Asn Ser Met Leu Ile

85 90 95

Val Asn Leu Leu Ala Val Thr Gly Gly Cys Phe Met Gly Leu Cys Lys

100 105 110

Val Ala Lys Ser Val Glu Met Leu Ile Leu Gly Arg Leu Val Ile Gly

115 120 125

Leu Phe Cys Gly Leu Cys Thr Gly Phe Val Pro Met Tyr Ile Gly Glu

130 135 140

Ile Ser Pro Thr Ala Leu Arg Gly Ala Phe Gly Thr Leu Asn Gln Leu
145 150 155 160

Gly Ile Val Val Gly Ile Leu Val Ala Gln Ile Phe Gly Leu Glu Phe
165 170 175

Ile Leu Gly Ser Glu Glu Leu Trp Pro Leu Leu Leu Gly Phe Thr Ile
180 185 190

Leu Pro Ala Ile Leu Gln Ser Ala Ala Leu Pro Phe Cys Pro Glu Ser
195 200 205

Pro Arg Phe Leu Leu Ile Asn Arg Lys Glu Glu Glu Asn Ala Lys Gln
210 215 220

Ile Leu Gln Arg Leu Trp Gly Thr Gln Asp Val Ser Gln Asp Ile Gln
225 230 235 240

Glu Met Lys Asp Glu Ser Ala Arg Met Ser Gln Glu Lys Gln Val Thr
245 250 255

Val Leu Glu Leu Phe Arg Val Ser Ser Tyr Arg Gln Pro Ile Ile Ile
260 265 270

Ser Ile Val Leu Gln Leu Ser Gln Gln Leu Ser Gly Ile Asn Ala Val
275 280 285

Phe Tyr Tyr Ser Thr Gly Ile Phe Lys Asp Ala Gly Val Gln Glu Pro
290 295 300

Ile Tyr Ala Thr Ile Gly Ala Gly Val Val Asn Thr Ile Phe Thr Val
305 310 315 320

Val Ser Leu Phe Leu Val Glu Arg Ala Gly Arg Arg Thr Leu His Met
325 330 335

Ile Gly Leu Gly Gly Met Ala Phe Cys Ser Thr Leu Met Thr Val Ser
340 345 350

Leu Leu Leu Lys Asp Asn Tyr Asn Gly Met Ser Phe Val Cys Ile Gly
355 360 365

Ala Ile Leu Val Phe Val Ala Phe Phe Glu Ile Gly Pro Gly Pro Ile
370 375 380

Pro Trp Phe Ile Val Ala Glu Leu Phe Ser Gln Gly Pro Arg Pro Ala
385 390 395 400

Ala Met Ala Val Ala Gly Cys Ser Asn Trp Thr Ser Asn Phe Leu Val
405 410 415

Gly Leu Leu Phe Pro Ser Ala Ala His Tyr Leu Gly Ala Tyr Val Phe
420 425 430

Ile Ile Phe Thr Gly Phe Leu Ile Thr Phe Leu Ala Phe Thr Phe Phe
435 440 445

Lys Val Pro Glu Thr Arg Gly Arg Thr Phe Glu Asp Ile Thr Arg Ala
450 455 460

Phe Glu Gly Gln Ala His Gly Ala Asp Arg Ser Gly Lys Asp Gly Val

465 470 475 480

Met Glu Met Asn Ser Ile Glu Pro Ala Lys Glu Thr Thr Thr Asn Val

485 490 495

<210> 23
<211> 1085
<212> DNA
<213> Homo sapiens

<220>
<221> CDS
<222> (76) .. (678)

<400> 23

atcctgtctg tccgaaccca gacacaagtc ttcactcctt cctgcgagcc ctgaggaagc 60

cttctttccc cagac atg gcc aac aag ggt cct tcc tat ggc atg agc cgc 111

Met Ala Asn Lys Gly Pro Ser Tyr Gly Met Ser Arg
1 5 10

gaa gtg cag tcc aaa atc gag aag aag tat gac gag gag ctg gag gag 159
Glu Val Gln Ser Lys Ile Glu Lys Lys Tyr Asp Glu Glu Leu Glu Glu
15 20 25

cgg ctg gtg gag tgg atc ata gtg cag tgt ggc cct gat gtg ggc cgc 207
Arg Leu Val Glu Trp Ile Ile Val Gln Cys Gly Pro Asp Val Gly Arg
30 35 40

cca gac cgt ggg ccc ttg ggc ttc cag gtg tgg ctg aag aat ggc gtg 255
Pro Asp Arg Gly Pro Leu Gly Phe Gln Val Trp Leu Lys Asn Gly Val
45 50 55 60

att ctg agc aag ctg gtg aac agc ctg tac cct gat ggc tcc aag ccg 303
Ile Leu Ser Lys Leu Val Asn Ser Leu Tyr Pro Asp Gly Ser Lys Pro
65 70 75

gtg aag gtg ccc gag aac cca ccc tcc atg gtc ttc aag cag atg gag 351
Val Lys Val Pro Glu Asn Pro Pro Ser Met Val Phe Lys Gln Met Glu

80 85 90

cag gtg gct cag ttc ctg aag gcg gct gag gac tct ggg gtc atc aag 399

Gln Val Ala Gln Phe Leu Lys Ala Ala Glu Asp Ser Gly Val Ile Lys

95 100 105

act gac atg ttc cag act gtt gac ctc ttt gaa ggc aaa gac atg gca 447

Thr Asp Met Phe Gln Thr Val Asp Leu Phe Glu Gly Lys Asp Met Ala

110 115 120

gca gtg cag agg acc ctg atg gct ttg ggc agc ttg gca gtg acc aag 495

Ala Val Gln Arg Thr Leu Met Ala Leu Gly Ser Leu Ala Val Thr Lys

125 130 135 140

aat gat ggg cac tac cgt gga gat ccc aac tgg ttt atg aag aaa gcg 543

Asn Asp Gly His Tyr Arg Gly Asp Pro Asn Trp Phe Met Lys Lys Ala

145 150 155

cag gag cat aag agg gaa ttc aca gag agc cag ctg cag gag gga aag 591

Gln Glu His Lys Arg Glu Phe Thr Glu Ser Gln Leu Gln Glu Gly Lys

160 165 170

cat gtc att ggc ctt cag atg ggc agc aac aga ggg gcc tcc cag gcc 639
His Val Ile Gly Leu Gln Met Gly Ser Asn Arg Gly Ala Ser Gln Ala
175 180 185

ggc atg aca ggc tac gga cga cct cgg cag atc atc agt tagagcggag 688
Gly Met Thr Gly Tyr Gly Arg Pro Arg Gln Ile Ile Ser
190 195 200

agggctagcc ctgagcccgg cgctccccca gctccttggc tgcagccatc ccgcttagcc 748

tgcctcaccc acacccgtgt ggtaccttca gccctggcca agctttgagg ctctgtcact 808

gagcaatggt aactgcacct gggcagctcc tccctgtgcc cccagcctca gcccaacttc 868

ttacccgaaa gcatcactgc cttggcccct ccctcccggc ggcccccatc acctctactg 928

tctcctccct gggctaagca ggggagaagc gggctggggg tagcctggat gtgggcgaag 988

tccactgtcc tccttggcgg caaaagccca ttgaagaaga accagcccag cctgcccccct 1048

atcttgtacc tggaatattt ttggggttgg aactctc 1085

<210> 24
<211> 201
<212> PRT
<213> Homo sapiens

<400> 24

Met Ala Asn Lys Gly Pro Ser Tyr Gly Met Ser Arg Glu Val Gln Ser

1 5 10 15

Lys Ile Glu Lys Lys Tyr Asp Glu Glu Leu Glu Glu Arg Leu Val Glu

20 25 30

Trp Ile Ile Val Gln Cys Gly Pro Asp Val Gly Arg Pro Asp Arg Gly

35 40 45

Pro Leu Gly Phe Gln Val Trp Leu Lys Asn Gly Val Ile Leu Ser Lys

50 55 60

Leu Val Asn Ser Leu Tyr Pro Asp Gly Ser Lys Pro Val Lys Val Pro

65 70 75 80

Glu Asn Pro Pro Ser Met Val Phe Lys Gln Met Glu Gln Val Ala Gln

85 90 95

Phe Leu Lys Ala Ala Glu Asp Ser Gly Val Ile Lys Thr Asp Met Phe

100 105 110

Gln Thr Val Asp Leu Phe Glu Gly Lys Asp Met Ala Ala Val Gln Arg

115 120 125

Thr Leu Met Ala Leu Gly Ser Leu Ala Val Thr Lys Asn Asp Gly His

130 135 140

Tyr Arg Gly Asp Pro Asn Trp Phe Met Lys Lys Ala Gln Glu His Lys

145 150 155 160

Arg Glu Phe Thr Glu Ser Gln Leu Gln Glu Gly Lys His Val Ile Gly

165 170 175

Leu Gln Met Gly Ser Asn Arg Gly Ala Ser Gln Ala Gly Met Thr Gly

180 185 190

Tyr Gly Arg Pro Arg Gln Ile Ile Ser

195 200

<210> 25
<211> 30
<212> DNA
<213> Artificial

<220>
<223> Oligonucleotide designed to act as primer for amplifying kiaa1001 gene transcript.

<400> 25
ggaacatctc tttgaattgt atttcttgta 30

<210> 26
<211> 22

<212> DNA
<213> Artificial

<220>
<223> Oligonucleotide designed to act as primer for amplifying kiaa1001 gene transcript.

<400> 26
agccacagcc aaaaaagact tt 22

<210> 27
<211> 32
<212> DNA
<213> Artificial

<220>
<223> Oligonucleotide designed to act as TaqMan probe for detecting amplification of kiaa1001 gene transcript.

<400> 27
ttacatactt agagagagac tcttttagcc ag 32

<210> 28
<211> 28
<212> DNA
<213> Artificial

<220>
<223> Oligonucleotide designed to act as primer for amplifying asah1 gene transcript.

<400> 28
accctaagga agttgctaac ttaaaaaa 28

<210> 29
<211> 29
<212> DNA
<213> Artificial

<220>
<223> Oligonucleotide designed to act as primer for amplifying asah1 gene transcript.

<400> 29
tccacaagtc tttgacttgt ttatttact 29

<210> 30
<211> 23
<212> DNA
<213> Artificial

<220>
<223> Oligonucleotide designed to act as TaqMan probe for detecting amplification of asah1 gene transcript.

<400> 30
ctgcatccca cgttctgtta att 23

<210> 31
<211> 22
<212> DNA
<213> Artificial

<220>
<223> Oligonucleotide designed to act as primer for amplifying mgc4171 gene transcript.

<400> 31
caggtggtct tttggtgcct ta 22

<210> 32
<211> 18
<212> DNA
<213> Artificial

<220>
<223> Oligonucleotide designed to act as primer for amplifying mgc4171 gene transcript.

<400> 32
agtggctccc acgctgaa 18

<210> 33
<211> 24
<212> DNA
<213> Artificial

<220>
<223> Oligonucleotide designed to act as TaqMan probe for detecting amplification of mgc4171 gene transcript.

<400> 33
tgaagagtcg gattttgaag cagc 24

<210> 34
<211> 24
<212> DNA
<213> Artificial

<220>
<223> Oligonucleotide designed to act as primer for amplifying 1ss gene transcript.

<400> 34
gtccggtgtc tacttgagaa acag 24

<210> 35
<211> 21
<212> DNA
<213> Artificial

<220>
<223> Oligonucleotide designed to act as primer for amplifying 1ss gene transcript.

<400> 35
agaccccagc aatgttttcc t 21

<210> 36
<211> 18
<212> DNA
<213> Artificial

<220>
<223> Oligonucleotide designed to act as TaqMan probe for detecting amplification of 1ss gene transcript.

<400> 36
cccaatggcg actggccg 18

<210> 37
<211> 21
<212> DNA
<213> Artificial

<220>
<223> Oligonucleotide designed to act as primer for amplifying nr0b2 gene transcript.

<400> 37
cagcacttgg ctccttagga a 21

<210> 38
<211> 23
<212> DNA
<213> Artificial

<220>
<223> Oligonucleotide designed to act as primer for amplifying nr0b2 gene transcript.

<400> 38
actgtgtcca aaccaaggaa gtc 23

<210> 39
<211> 23
<212> DNA
<213> Artificial

<220>
<223> Oligonucleotide designed to act as TaqMan probe for detecting amplification of nr0b2 gene transcript.

<400> 39
agctcttcac tcagccacac ccc 23

<210> 40
<211> 22
<212> DNA
<213> Artificial

<220>
<223> Oligonucleotide designed to act as primer for amplifying fabp1 gene transcript.

<400> 40
gagtttctcc ggcaagtacc aa 22

<210> 41
<211> 20
<212> DNA
<213> Artificial

<220>
<223> Oligonucleotide designed to act as primer for amplifying fabp1 gene transcript.

<400> 41
cagaccgatt gccttcatga 20

<210> 42
<211> 25
<212> DNA
<213> Artificial

<220>
<223> Oligonucleotide designed to act as TaqMan probe for detecting amplification of fabp1 gene transcript.

<400> 42
tgcagagcca ggaaaacttt gaagc 25

<210> 43
<211> 22
<212> DNA
<213> Artificial

<220>
<223> Oligonucleotide designed to act as primer for amplifying hpn gene transcript.

<400> 43
gaaaccagat caagcccaag at 22

<210> 44
<211> 18
<212> DNA
<213> Artificial

<220>
<223> Oligonucleotide designed to act as primer for amplifying hpn gene transcript.

<400> 44
ccctggcagg catcaatg 18

<210> 45
<211> 20
<212> DNA
<213> Artificial

<220>
<223> Oligonucleotide designed to act as TaqMan probe for detecting amplification of hpn gene transcript.

<400> 45
ttctgtgctg gctaccccga 20

<210> 46
<211> 21
<212> DNA
<213> Artificial

<220>
<223> Oligonucleotide designed to act as primer for amplifying serpina3 gene transcript.

<400> 46
gaggagggca cagaagcatc t 21

<210> 47
<211> 24
<212> DNA

<213> Artificial

<220>
<223> Oligonucleotide designed to act as primer for amplifying serpina3 gene transcript.

<400> 47
ccttgtctcc actaatgcag aaag 24

<210> 48
<211> 24
<212> DNA
<213> Artificial

<220>
<223> Oligonucleotide designed to act as TaqMan probe for detecting amplification of serpina3 gene transcript.

<400> 48
tgccacagca gtcaaaatca ccct 24

<210> 49
<211> 20
<212> DNA
<213> Artificial

<220>
<223> Oligonucleotide designed to act as primer for amplifying depp gene transcript.

<400> 49
tgtggtggca ttgggagagt 20

<210> 50
<211> 25
<212> DNA
<213> Artificial

<220>
<223> Oligonucleotide designed to act as primer for amplifying depp gene transcript.

<400> 50
tggtagcact gagtggtaca gattc 25

<210> 51
<211> 22
<212> DNA
<213> Artificial

<220>
<223> Oligonucleotide designed to act as TaqMan probe for detecting amplification of depp gene transcript.

<400> 51
cccccatgat gagggccaag at 22

<210> 52
<211> 22
<212> DNA
<213> Artificial

<220>

<223> Oligonucleotide designed to act as primer for amplifying flj22362 gene transcript.

<400> 52
ggtaatgcca ctcccacaat ct 22

<210> 53
<211> 21
<212> DNA
<213> Artificial

<220>
<223> Oligonucleotide designed to act as primer for amplifying flj22362 gene transcript.

<400> 53
ccttctggct ctgctcacag t 21

<210> 54
<211> 23
<212> DNA
<213> Artificial

<220>
<223> Oligonucleotide designed to act as TaqMan probe for detecting amplification of flj22362 gene transcript.

<400> 54
aggcctggta cccatcctct ttc 23

<210> 55
<211> 21
<212> DNA
<213> Artificial

<220>
<223> Oligonucleotide designed to act as primer for amplifying slc2a3 gene transcript.

<400> 55
gcttgaaaag gtgaccttgc a 21

<210> 56
<211> 23
<212> DNA
<213> Artificial

<220>
<223> Oligonucleotide designed to act as primer for amplifying slc2a3 gene transcript.

<400> 56
tgccttactg ccaacctact gtt 23

<210> 57
<211> 23
<212> DNA
<213> Artificial

<220>
<223> Oligonucleotide designed to act as TaqMan probe for detecting amplification of slc2a3 gene transcript.

<400> 57

tcatgtcaac tttctggctc ctc 23

<210> 58
<211> 25
<212> DNA
<213> Artificial

<220>
<223> Oligonucleotide designed to act as primer for amplifying tagln gene transcript.

<400> 58
gagcataaga gggaattcac agaga 25

<210> 59
<211> 20
<212> DNA
<213> Artificial

<220>
<223> Oligonucleotide designed to act as primer for amplifying tagln gene transcript.

<400> 59
ctgttgctgc ccatctgaag 20

<210> 60
<211> 27
<212> DNA
<213> Artificial

<220>
<223> Oligonucleotide designed to act as TaqMan probe for detecting amplification of tagln gene transcript.

<400> 60
agctgcagga gggaaagcat gtcattg 27